# EUROPEAN PATENT APPLICATION

(11) **EP 2 123 676 A1**
(43) Date of publication of application: **25.11.2009**
(21) Application number: 07860511.0
(22) Date of filing: 28.12.2007
(51) Int. Cl.: C07K 16/18, A61K 39/395, A61P 35/00, A61P 35/04, C12Q 1/68, G01N 33/15, G01N 33/50, G01N 33/574, C12N 15/09

(54) **DIAGNOSIS AND TREATMENT OF CANCER BY USING ANTI-PRG-3 ANTIBODY**

(30) Priority: 05.01.2007 JP 2007000804
(71) Applicant: The University of Tokyo, Bunkyo-Ku Tokyo 113-8654 (JP); Forerunner Pharma Research Co., Ltd., Meguro-ku Tokyo 153-0041 (JP)
(72) Inventor: ABURATANI, Hiroyuki, Bunkyo-ku Tokyo 113-8654 (JP); TORISU, Yuichi, Bunkyo-ku Tokyo 113-8654 (JP); FUNAHASHI, Shinichi, Meguro-ku Tokyo 153-0041 (JP)
(74) Representative: Woods, Geoffrey Corlett
(86) International application number: PCT/JP2007/075308
(87) International publication number: WO 2008/081942

(57) **Abstract**

The present invention discloses an antibody capable of binding to the PRG-3 protein and inhibiting the growth of cells expressing the PRG-3 protein. The growth inhibitory activity is cytotoxic activity, such as antibody-dependent cell-mediated cytotoxicity and complement-dependent cytotoxicity. The present invention also discloses a pharmaceutical composition, cell growth inhibitor, and an anticancer agent comprising the antibody of the present invention as an active ingredient. Examples of the type of cancer include hepatocellular carcinoma, lung cancer, colon cancer, and glioblastoma. In addition, the present invention discloses a method for diagnosing cancer by detecting expression of the PRG-3 protein or the gene encoding the PRG-3 protein, as well as a diagnostic agent and kit for use in the method.

## Description

### TECHNICAL FIELD

The present invention relates to a method for the diagnosis of cancer, a method for the treatment of cancer, and an anticancer agent.

### BACKGROUND ART

Primary hepatocellular carcinoma (HCC) is a type of cancer with a poor prognosis, with representing the third place in men (13%) and the fourth place in women (9.0%) among deaths caused by cancer, which was the number one cause of death in Japan in 2001 (excerpted from "Vital Statistics" issued by Statistics and Information Department, Minister's Secretariat, Ministry of Health, Labour and Welfare). Chronic cases due to viral infection are increasing year by year, and most progress from cirrhosis of the liver to HCC. Therefore, a method for early diagnosis at the transition stage from cirrhosis to HCC and a method for treatment of HCC are urgently needed. Unless a breakthrough occurs, it is believed that the increasing trend in mortality from HCC will continue for the next 10 to 15 years.

HCC is diagnosed from a comprehensive evaluation based on biochemical data such as serum levels of GOT/GPT, alkaline phosphatase, albumin, and the tumor marker a-fetoprotein (AFP), etc., and on diagnostic imaging. A small tissue sample is taken by needle biopsy when necessary, and a definitive diagnosis is made based on a histopathological evaluation. At present, tumor markers are mainly used for the diagnosis of HCC, and roughly 60% to 70% of patients with HCC test positive for AFP, which is the most frequently used marker. However, patients with chronic liver disease and pregnant women also test positive for AFP. The positive rate for the liver cancer tumor marker PIVKA-II is lower at less than 50%, but it is believed to be more specific for HCC than AFP. At present, tests are conducted mainly for these two markers. Because some patients test falsely positive and some test negative for both markers, a highly specific tumor marker has been sought.

The histopathological examination of a sample collected by needle biopsy is an important test for making a definitive diagnosis of liver disease. Due to the limited amount of collected tissue, a more reliable diagnostic technique is needed. It would be desirable to develop an antibody toward an antigen specifically expressed in liver cancer which can identify pathological characteristics and differentiate early HCC from non-cancerous tissue.

With respect to the diagnosis and monitoring of patients with liver disease, the transition from inflammation to fibrosis and to malignant transformation is diagnosed by testing for a plurality of markers and by biopsy examination. In most patients with liver disease, the condition progresses from viral infection to hepatitis, to chronic hepatitis, to cirrhosis, and then to liver cancer. Therefore, a method that enables simple diagnosis and monitoring of liver disease will be useful from the standpoint of medical economics, and such a method will alleviate the burden on the patient, and enable a clear and accurate therapeutic policy to be established.

In most medical facilities, HCC treatment mainly involves three therapeutic modes: surgical resection, trancecatheter arterial chemoembolization, and percutaneous ethanol injection therapy. Each mode of therapy has its strengths and weaknesses. While trancecatheter arterial chemoembolization has a relatively wide range of uses and is effective in extending survival, the complete cure rate is estimated to be only about 10% at present. Therefore, a novel mode of therapy is greatly desired.

Based on the recent technological revolutions in the life sciences, researches are underway to identify a molecule that is specifically expressed in cancer cells or is essential for the growth of cancer cells, to find substances that regulate such molecular functions, and to develop molecular targeted therapeutic drugs using those substances as cancer cell growth inhibitors. So far there have been no clinical applications of molecular targeted drugs for HCC. However, drugs have been developed which comprise as an active ingredient a monoclonal antibody capable of binding to a tumor antigen specifically expressed in breast cancer cells or lymphoma cells. These drugs have increased the response rate through mechanisms of action specific to antibodies, which is distinct from previous chemotherapy-based treatments. Such mechanisms include antibody-dependent cell-mediated cytotoxicity (ADCC) via effector cells, or complement-dependent cytotoxicity (CDC) via complement, antibody neutralization activity, or an agonistic effect of the antibody. At present the use of molecular targeted drugs to treat cancers other than HCC has started, and discovery of a molecular targeted drug aimed at HCC is eagerly anticipated.

The PRG-3 molecule is a protein encoded by GS91507 gene at locus 9q31-34 on the human chromosome, and is classified as a member of the plasticity-related gene family (hereinafter, PRG family) based on the characteristics of its amino acid sequence. Members of the PRG family are molecules that are known to catalyze the dephosphorylation of phospholipids in neurons and are involved in nerve plasticity. Unlike other members of the PRG family, the target molecule containing a phospholipid to be enzymatically degraded by PRG-3 has not been identified. It is known that this gene is highly expressed in the brain, and it exhibits dynamic expression profiles during brain differentiation and neuronal excitation (Eur. J. Neurosci. 19(1), 212-220 (2004)). With respect to the involvement of the PRG-3 molecule in human diseases, however, it has only been reported that PRG-3 may possibly be involved in diseases associated with the regulation of cholesterol flow, particularly Tangier disease, familial HDL deficiency disease, or diseases genetically linked to the locus 9q31-34 of Chromosome 9 (WO2000/071710). There have been no reports thus far indicating that this molecule may serve as a target for cancer therapy directed toward HCC. Furthermore, its role and association in other types of cancer remain unknown.

Furthermore, it has been reported that an antibody that binds to PRG family member molecules cannot be obtained because PRG family molecules assume a molecular structure with 6 transmembrane-spanning regions when expressed in a cell (Biochem. J. 387, 281-293 (2005)), and thus it has been extremely difficult to analyze their role in the body.

The reference documents cited in this description are listed below. The entire content disclosed in these documents is hereby incorporated by reference. None of these documents are admitted to represent prior art of the present invention.
[Patent document 1] WO2000071710
[Non-patent document 1] Eur. J. Neurosci. 19(1), 212-220 (2004)
[Non-patent document 2] Biochem. J. 387, 281-293 (2005)

### DISCLOSURE OF THE INVENTION

An object of the present invention is to provide a novel method for diagnosing cancer, a novel method for treating cancer, and a novel cell growth inhibitor and anticancer agent.

The inventors discovered that both the PRG-3 gene and the PRG-3 protein are expressed at high levels in HCC and other cancer cells. The present invention provides a monoclonal antibody capable of binding to the PRG-3 protein. Additionally, the inventors discovered that when the complement-dependent cytotoxicity (CDC) activity and the antibody-dependent cell-mediated cytotoxicity (ADCC) activity of the anti-PRG-3 antibody were measured, the anti-PRG-3 antibody of the invention was found to have both CDC and ADCC activity toward cells that express PRG-3. Based on the above findings, the inventors discovered that the anti-PRG-3 antibody is useful for the diagnosis, prevention, and treatment of various kinds of primary or metastatic cancers to achieve the present invention. More specifically, the inventors discovered that PRG-3 is useful as a tool for the diagnosis and treatment of HCC, lung cancer, colon cancer, glioblastoma, and other types of cancer wherein PRG-3 expression is elevated, to achieve the present invention.

The present invention provides an antibody capable of binding to the PRG-3 protein. Additionally, the present invention provides an antibody that not only binds to the PRG-3 protein, but also has cytotoxic activity toward a cell expressing the PRG-3 protein. The type of cytotoxic activity is preferably ADCC or CDC. Furthermore, the present invention provides an antibody conjugated with a cytotoxic substance. Examples of the cytotoxic substance to be conjugated with the antibody include a chemotherapeutic drug, a radioisotope, or a toxic peptide. Preferably, the antibody of the present invention can function as an antibody that itself has cytotoxic activity.

Additionally, the present invention provides a pharmaceutical composition comprising an antibody capable of binding to the PRG-3 protein as an active ingredient. The present invention also provides a cell growth inhibitor comprising an antibody capable of binding to the PRG-3 protein as an active ingredient. Furthermore, the present invention provides an anticancer agent comprising an antibody capable of binding to the PRG-3 protein as an active ingredient. Preferably, the antibody capable of binding to the PRG-3 protein has cytoxic activity. In a preferred embodiment of the present invention, the cancer to be treated is HCC, lung cancer, colon cancer, or glioblastoma. The anticancer agent comprising an anti-PRG-3 antibody according to the present invention is useful for the treatment of primary or metastatic cancers having an enhanced level of PRG-3 expression. HCC is a particularly preferred target of therapy according to the present invention.

The present invention also provides a pharmaceutical composition comprising an antibody capable of binding to the PRG-3 protein and a pharmaceutically acceptable carrier. The pharmaceutical composition of the present invention is useful for the prevention and/or treatment of a cancer having an enhanced level of PRG-3 expression. More specifically, the present invention relates to the use of an antibody capable of binding to the PRG-3 protein in the manufacture of a pharmaceutical composition for the prevention and/or treatment of cancer.

In another aspect, the present invention provides a method for causing cytotoxicity in cells expressing the PRG-3 protein by contacting the cells with an antibody capable of binding to the PRG-3 protein. Moreover, the present invention provides a method for inhibiting the growth of cells expressing the PRG-3 protein by contacting the cells with an antibody capable of binding to the PRG-3 protein. Preferably, the antibody capable of binding to the PRG-3 protein is an antibody having cytotoxic activity itself. Furthermore, the cell expressing the PRG-3 protein is preferably a cancer cell.

In another aspect, the present invention provides the use of the PRG-3 protein as a diagnostic marker for cancer.

In another aspect, the present invention provides a diagnostic method for cancer characterized in that the PRG-3 protein is detected using an antibody capable of binding to the PRG-3 protein. Preferably an extracellular domain of the PRG-3 protein is detected in the method of the present invention. Preferably, the method of the present invention is carried out using an antibody that recognizes the PRG-3 protein. Preferably, in the method of the present invention the PRG-3 protein is detected in an HCC tissue specimen that was collected from a patient afflicted with HCC, or in a test sample isolated from that HCC tissue specimen.

In another embodiment, the present invention provides a diagnostic method for cancer comprising the steps of:
(a) providing a test sample collected from a subject; and
(b) detecting the PRG-3 protein contained in the collected test sample using an antibody capable of binding to the PRG-3 protein.
In the present invention any type of test sample collected from a subject can be used. In one embodiment, a test sample is collected surgically or by biopsy from the patient. The cancer to be detected by this diagnostic method can be any type of cancer where the targeted cancer cells express the PRG-3 protein. Preferably, the type of cancer in the present invention is HCC, lung cancer, colon cancer, or glioblastoma. Both primary lesions and metastatic lesions of these types of cancer can be detected according to the present invention. The particularly preferred types of cancer to be detected are primary HCC and metastatic HCC.

In yet another aspect, the present invention provides a diagnostic method for cancer comprising the steps of:
(1) administering to a subject an antibody capable of binding to the PRG-3 protein and labeled with a radioisotope; and
(2) detecting the accumulation of the radioisotope.
In one embodiment, the radioisotope is a positron-emitting nuclide. A preferred positron-emitting nuclide in the present invention can be selected from the group consisting of 11C, 13N, 150, 18F, 45Ti, 55Co, 64Cu, 66Ga, 68Ga, 76Br, 89Zr and 124I.

In yet another aspect, the present invention provides a diagnostic method for cancer characterized in that the expression of a gene encoding the PRG-3 protein is detected.

In yet another aspect, the present invention also provides a diagnostic agent and a kit for use in the diagnostic method of the present invention.

Moreover, the present invention provides a method of screening for a candidate compound to be used in a therapeutic agent for cancer. In the present invention, a candidate compound to be used in a therapeutic agent for cancer can be selected based on the expression level of PRG-3 as an indicator.

More specifically, the present invention provides items [1] to [28] as follows:
[1] An antibody which binds to PRG-3 protein and has cell growth inhibitory activity toward a cell expressing the PRG-3 protein;
[2] The antibody according to [1], wherein the cell growth inhibitory activity is cytotoxic activity;
[3] The antibody according to [2], wherein the cytotoxic activity is antibody-dependent cell-mediated cytotoxicity (ADCC);
[4] The antibody according to [2], wherein the cytotoxic activity is complement-dependent cytotoxicity (CDC);
[5] A low molecular antibody prepared from the antibody according to [1];
[6] The antibody according to any of [1] to [5], conjugated with a chemotherapeutic agent or a toxic peptide;
[7] An antibody which binds to PRG-3 protein and is conjugated with a substance selected from the group consisting of a chemotherapeutic agent, a toxic peptide, and a radioisotope;
[8] An antibody according to any of (1) to (34) below:
   (1) an antibody comprising an H-chain having the amino acid sequence represented by SEQ ID NO: 2 as CDR1, the amino acid sequence represented by SEQ ID NO: 4 as CDR2, and the amino acid sequence represented by SEQ ID NO: 6 as CDR3;
   (2) an antibody comprising the H-chain according to (1) having the amino acid sequence represented by SEQ ID NO: 8 as a CH;
   (3) an antibody comprising the H-chain according to (1) having the amino acid sequence represented by SEQ ID NO: 10 as a CH;
   (4) an antibody comprising an L-chain having the amino acid sequence represented by SEQ ID NO: 12 as CDR1, the amino acid sequence represented by SEQ ID NO: 14 as CDR2, and the amino acid sequence represented by SEQ ID NO: 16 as CDR3;
   (5) an antibody comprising the L-chain according to (4) having the amino acid sequence represented by SEQ ID NO: 18 as a CL;
   (6) an antibody comprising the L-chain according to (4) having the amino acid sequence represented by SEQ ID NO: 20 as a CL;
   (7) an antibody comprising the H-chain according to (1) and the L-chain according to (4);
   (8) an antibody comprising the H-chain according to (2) and the L-chain according to (5);
   (9) an antibody comprising the H-chain according to (3) and the L-chain according to (6);
   (10) an antibody in which one or a plurality of amino acids is substituted, deleted from, added to, and/or inserted into the antibody according to any of (1) to (9), and which has activity equivalent to the antibody according to any of (1) to (9);
   (11) an antibody capable of binding to the same epitope as the epitope on the PRG-3 protein to which the antibody according to any of (1) to (10) is capable of binding;
   (12) an antibody comprising an H-chain having the amino acid sequence represented by SEQ ID NO: 22 as CDR1, the amino acid sequence represented by SEQ ID NO: 24 as CDR2, and the amino acid sequence represented by SEQ ID NO: 26 as CDR3;
   (13) an antibody comprising the H-chain according to (12) having the amino acid sequence represented by SEQ ID NO: 34 as a CH;
   (14) an antibody comprising the H-chain according to (12) having the amino acid sequence represented by SEQ ID NO: 10 as a CH;
   (15) an antibody comprising an L-chain having the amino acid sequence represented by SEQ ID NO: 28 as CDR1, the amino acid sequence represented by SEQ ID NO: 30 as CDR2, and the amino acid sequence represented by SEQ ID NO: 32 as CDR3;
   (16) an antibody comprising the L-chain according to (15) having the amino acid sequence represented by SEQ ID NO: 18 as a CL;
   (17) an antibody comprising the L-chain according to (15) having the amino acid sequence represented by SEQ ID NO: 20 as a CL;
   (18) an antibody comprising the H-chain according to (12) and the L-chain according to (15);
   (19) an antibody comprising the H-chain according to (13) and the L-chain according to (16);
   (20) an antibody comprising the H-chain according to (14) and the L-chain according to (17);
   (21) an antibody in which one or a plurality of amino acids is substituted, deleted from, added to, and/or inserted into the antibody according to any of (12) to (20), and which has activity equivalent to the antibody according to any of (12) to (20);
   (22) an antibody capable of binding to the same epitope as the epitope on the PRG-3 protein to which the antibody according to any of (12) to (21) capable of binding;
   (23) an antibody comprising an H-chain having the amino acid sequence represented by SEQ ID NO: 36 as CDR1, the amino acid sequence represented by SEQ ID NO: 38 as CDR2, and the amino acid sequence represented by SEQ ID NO: 40 as CDR3;
   (24) an antibody comprising the H-chain according to (23) having the amino acid sequence represented by SEQ ID NO: 34 as a CH;
   (25) an antibody comprising the H-chain according to (23) having the amino acid sequence represented by SEQ ID NO: 10 as a CH;
   (26) an antibody comprising an L-chain having the amino acid sequence represented by SEQ ID NO: 42 as CDR1, the amino acid sequence represented by SEQ ID NO: 44 as CDR2, and the amino acid sequence represented by SEQ ID NO: 46 as CDR3;
   (27) an antibody comprising the L-chain according to (26) having the amino acid sequence represented by SEQ ID NO: 18 as a CL;
   (28) an antibody comprising the L-chain according to (26) having the amino acid sequence represented by SEQ ID NO: 20 as a CL;
   (29) an antibody comprising the H-chain according to (23) and the L-chain according to (26);
   (30) an antibody comprising the H-chain according to (24) and the L-chain according to (27);
   (31) an antibody comprising the H-chain according to (25) and the L-chain according to (28);
   (32) an antibody in which one or a plurality of amino acids is substituted, deleted from, added to, and/or inserted into the antibody according to any of (23) to (31), and which has activity equivalent to the antibody according to any of (23) to (31);
   (33) an antibody which binds to the same epitope as the epitope on the PRG-3 protein to which the antibody according to any of (23) to (32) binds; and
   (34) a low molecular antibody of the antibody according to any of (1) to (33);
[9] A pharmaceutical composition comprising the antibody according to any of [1] to [8] as an active ingredient;
[10] A cell growth inhibitor comprising the antibody according to any of [1] to [8] as an active ingredient;
[11] An anticancer agent comprising the antibody according to any of [1] to [8] as an active ingredient;
[12] The anticancer agent according to [11], wherein the cancer is selected from the group consisting of hepatocellular carcinoma, lung cancer, colon cancer, and glioblastoma;
[13] The anticancer agent according to [12], wherein the cancer is a primary cancer;
[14] The anticancer agent according to [12], wherein the cancer is a metastatic cancer;
[15] A diagnostic method for cancer comprising detecting the expression of a gene encoding PRG-3 protein;
[16] A diagnostic method for cancer comprising detecting PRG-3 protein;
[17] The diagnostic method according to [16], wherein the PRG-3 protein is detected using an antibody which binds to the PRG-3 protein;
[18] A diagnostic method for cancer, the method comprising the steps of:
   (a) providing a test sample collected from a subject; and
   (b) detecting PRG-3 protein contained in the test sample of

   (a) using an antibody which binds to the PRG-3 protein;
[19] A diagnostic method for cancer, the method comprising the steps of:
   (1) administering to a subject an antibody that has binding activity toward PRG-3 protein and labeled with a radioisotope; and
   (2) detecting the accumulation of the aforementioned radioisotope;
[20] The diagnostic method according to any of [17] to [19], wherein the antibody according to [8] is used;
[21] The diagnostic method according to any of [15] to [20], wherein the cancer is selected from the group consisting of hepatocellular carcinoma, lung cancer, colon cancer, and glioblastoma;
[22] The diagnostic method according to [21], wherein the cancer is a primary cancer;
[23] The diagnostic method according to [21] wherein the cancer is a metastatic cancer;
[24] A diagnostic agent for use in the diagnosis of cancer comprising an antibody which binds to PRG-3 protein;
[25] A kit for use in the diagnosis of cancer comprising an antibody which binds to PRG-3 protein and a reagent for detecting the binding between the antibody and the PRG-3 protein;
[26] Use of PRG-3 protein and/or PRG-3 mRNA as a marker for the diagnosis of cancer;
[27] Use of an antibody which binds to PRG-3 for the diagnosis of cancer;
[28] A method of screening for a candidate compound to be used in an anticancer agent, the method comprising the steps of:
   (1) contacting the test compound with cells that express PRG-3;
   (2) measuring the level of PRG-3 expression in the cells that express PRG-3; and
   (3) selecting a compound that decreases the level of PRG-3 expression compared with a control, as a candidate compound to be used in an anticancer agent.

An analysis of gene expression in various types of cancer tissue and cancer cell lines has revealed a marked increase in the expression of the PRG-3 gene in cancer cells. In contrast, the expression of PRG-3 in normal cells was extremely low in organs other than the brain. Therefore, PRG-3 is useful as a marker for specifically detecting cancer.

In the present invention the cytotoxic effect of the anti-PRG-3 antibody on cells that express PRG-3 was observed. Although the level of PRG-3 expression was extremely low in normal cells other than the brain, it was enhanced in cancer calls. This finding indicates that a cancer cell-specific cytotoxic effect will be obtained in the body by the administration of the anti-PRG-3 antibody, for example. Therefore, an antibody that recognizes the PRG-3 protein will be useful as an anticancer agent, etc., and as a medicinal composition.

### BRIEF DESCRIPTION OF THE DRAWINGS

Figure 1 is a graph showing the amount of PRG-3 mRNA in normal tissues;
Figure 2 is a graph showing the amount of PRG-3 mRNA in clinical cancer tissues;
Figure 3 is a graph showing the amount of PRG-3 mRNA in cancer cell lines;
Figure 4 is a graph showing the reactivity of a PRG-3 hybridoma culture supernatant to PRG-3/293T-REX cells. The reactivity of the antibody contained in the hybridoma culture supernatant toward PRG3+/293 cells wherein the expression of PRG-3 was induced and PRG3-/293 cells wherein the expression of PRG-3 was not induced was compared by GeoMean values measured by flow cytometry;
Figure 5 is a graph showing the binding of the anti-PRG-3 monoclonal antibody to HepG2 cells. The binding of the antibody to HepG2 cells at antibody concentrations of 10 µg/mL, 1 µg/mL, and 0.1 µg/mL was compared by GeoMean values measured by flow cytometry;
Figure 6 is a graph showing selection of the PRG-3/BaF3 cell line using flow cytometry;
Figure 7 is a photograph of the detection of PRG-3 mRNA by RT-PCR in various cells that express PRG-3 and in HCC cell lines;
Figure 8 is a graph showing CDC activity of the anti-PRG-3 antibody in the OF5 cell line;
Figure 9 is a graph showing ADCC activity of the anti-PRG-3 antibody in the OF5-G8 cell line; and
Figure 10 is a diagram showing the interspecies conservation of the PRG-3 protein.

### PREFERRED EMBODIMENT OF THE INVENTION

PRG-3 is a protein that is a member of the PRG-3 family that has 6 transmembrane-spanning regions. The amino acid sequence of the PRG-3 protein and the base sequence of the gene encoding the same are disclosed in NCBI accession numbers AK000307 (SEQ ID NO: 59) and BAA91072 (SEQ ID NO: 60), respectively. In the present invention, the term "PRG-3 protein" refers to both the entire protein and a fragment thereof. Herein, the term "fragment" refers to a polypeptide containing any regions of the PRG-3 protein, and it does not necessarily have the function of the naturally occurring PRG-3 protein. An example of a fragment is one containing an extracellular domain of the PRG-3 protein. Extracellular domains of the PRG-3 protein correspond to amino acid residues 36 to 65, 150 to 200, and 248 to 256 in the amino acid sequence represented by SEQ ID NO: 60. Transmembrane regions correspond to amino acid residues 13 to 35, 66 to 88, 127 to 149, 201 to 217, 228 to 247, and 257 to 279 in the amino acid sequence represented by SEQ ID NO: 60.

In the present invention, analysis of clinical samples and cancer cell lines shows that PRG-3 is expressed at an extremely high frequency at the gene level in HCC, lung cancer, colon cancer, and glioblastoma. Furthermore, a high level of expression at the protein level is also shown in HCC cell lines. In other words, the PRG-3 protein is useful as a diagnostic marker for cancer.

### Detection of PRG-3

In one embodiment of the method of the present invention, the expression of the PRG-3 protein is detected. In the present invention the concept of detection includes both quantitative and qualitative detection. The following assays are examples of qualitative detection:
an assay simply to determine whether or not the PRG-3 protein is present;
an assay to determine whether or not a predetermined amount of the PRG-3 protein or more is present; and
an assay to compare the amount of PRG-3 protein with another test sample (for example, a control sample, etc.).
Quantitative detection includes an assay for the concentration of PRG-3 protein, an assay for the amount of PRG-3 protein, etc.

The test sample in the present invention is not particularly limited provided it is a test sample that possibly contains the PRG-3 protein. More specifically, a test sample collected from the body of an organism such as a mammal, etc., is preferred. A test sample collected from a human is more preferred. Specific examples of the test sample include blood, interstitial fluid, plasma, extravascular fluid, cerebrospinal fluid, synovial fluid, pleural fluid, serum, lymph, saliva, urine, tissue, etc. A preferred sample is one obtained from test sample material such as a specimen of fixed tissue or cells collected from the body of an organism or the liquid culture medium of cells.

The type of cancer to be diagnosed by the present invention is not particularly limited and can be any kind of cancer. Specific examples include HCC, lung cancer, and glioblastoma. In the present invention both primary lesions and metastatic lesions of these cancers can be diagnosed. In the present invention, primary HCC and metastatic HCC are the particularly preferred types of cancer.

In the present invention, if the PRG-3 protein is detected in a test sample, the level of the protein is indicative of cancer. More specifically, if the amount of the PRG-3 protein detected in a test sample is greater than the level in a negative control or in healthy individuals, it indicates that the patient has cancer or has a high likelihood of developing cancer in the future. In other words, the present invention relates to a diagnostic method for cancer comprising the following steps:
(1) the level of PRG-3 expression in a test sample collected from a subject is detected; and
(2) if the level of PRG-3 expression detected in (1) is higher than the level in a control, it is indicative that the subject has cancer.

In the present invention the term "control" refers to a sample serving as a standard for comparison, and it includes both negative controls and biological samples from healthy individuals. A negative control can be obtained by collecting biological samples from healthy individuals and mixing a plurality of samples as needed. The PRG-3 expression level in the control can be detected in parallel with the PRG-3 expression level in a biological sample from a subject. Alternatively, the PRG-3 expression level in biological samples from a plurality of healthy individuals can be detected beforehand, and the standard expression level in healthy individuals can be determined statistically. More specifically, a reference value of mean ±2 standard deviations (S.D.) or mean ±3 standard deviations can be used as a reference value. Statistically speaking, the mean ±2 standard deviations includes the values of 80% of healthy individuals, and the mean ±3 standard deviations includes the values of 90% of healthy individuals.

Alternatively, the PRG-3 expression level in the control can be established using a receiver operating characteristic curve (ROC curve). An ROC curve is a graph displaying the detection sensitivity on the vertical axis and the false positive rate (i.e., "1 - specificity") on the horizontal axis. In the present invention, an ROC curve can be obtained by plotting the change in sensitivity and the false positive rate when the reference value for determining the PRG-3 expression level in a biological sample is varied continuously.

The "reference value" for obtaining the ROC curve is a numerical value used temporarily for statistical analysis. Generally speaking, the "reference value" for obtaining the ROC curve is varied continuously within a range that covers all possible reference values that can be selected. For example, the reference value can be varied between the maximum and minimum of the measured values for PRG-3 in the population to be analyzed.

Based on the obtained ROC curve, the desired detection sensitivity and a reference value expected to be accurate can be selected. A reference value established statistically by an ROC curve and the like is also called a cut-off value. In the detection method for cancer based on a cut-off value, the PRG-3 expression level detected in (1) is compared in step (2) above. Moreover, cancer in the subject is detected when the PRG-3 expression level detected in (1) is higher than the cut-off value.

In the present invention the PRG-3 expression level can be determined by any methods. More specifically, it is possible to estimate the PRG-3 expression level by measuring the amount of PRG-3 mRNA, amount of the PRG-3 protein, or biological activity of the PRG-3 protein. The amounts of the PRG-3 mRNA and PRG-3 protein can be determined by methods disclosed in this description.

In the present invention, any animal species expressing the PRG-3 protein can be tested as the subject. For example, it is known that many nonhuman mammals such as chimpanzee (Pan *troglodytes*) (XP_00135494), mouse (*Mus musculus*) (NP_848871), rat (Rattus *norvegicus*) (NP_958428), domestic dog (*Canis familiaris*) (XP_538755), chicken (*Gallus gallus*) (XP_424888) and the like express the PRG-3 protein. Thus, these animals are included as subjects in the present invention. A human being is especially preferred as the subject. Needless to say, when a nonhuman animal is used as a subject, the PRG-3 protein inherent in that animal species will be detected.

The method for detecting the PRG-3 protein contained in a test sample is not particularly limited herein, but detection by an immunological method listed below using the anti-PRG-3 antibody is preferred.
Radioimmunoassay (RIA)
Enzyme immunoassay (EIA)
fluoroimmunoassay (FIA)
luminescence immunoassay (LIA)
Immunoprecipitation (IP)
Turbidimetric immunoassay (TIA)
Western blotting (WB)
Immunohistochemistry (IHC)
Single radial immunodiffusion (SRID)

Among these procedures, immunohistochemistry includes the step of detecting the PRG-3 protein on a section of fixed tissue or cells collected from a patient afflicted with cancer, and it is the preferred immunological assay in the diagnostic method for cancer. The above immunological methods such as immunohistochemistry and the like are publicly known methods to persons skilled in the art.

In other words, because PRG-3 is a membrane protein with specifically increased expression in cancer cells, the cancer cells or cancerous tissue can be detected by the anti-PRG-3 antibody. Thus, cancer calls contained in cells and tissue collected from the body can be detected by the above immunohistochemical analysis.

In another preferred embodiment, it is possible to detect cancer tissue in the body with the anti-PRG-3 antibody. More specifically, the present invention involves a method for detecting cancer in a subject comprising the steps of:
(1) administering to the subject an antibody capable of binding to the PRG-3 protein and labeled with a radioisotope; and
(2) detecting the accumulation of the labeled substance.
To trace the antibody administered to the body, the antibody can be labeled so that it can be detected. For example, it is possible to monitor the behavior in the body of an antibody labeled with a fluorescent or luminescent substance, or labeled with a radioisotope. An antibody labeled with a fluorescent substance or luminescent substance can be observed using an endoscopy or laparoscopy. With a radioisotope, the location of the antibody can be imaged by tracing the radioactivity. In the present invention, localization of the anti-PRG-3 antibody in the body represents the presence of cancer cells.

A positron-emitting nuclide can be used in the radioisotope labeling of the antibody for detection of cancer in the body. For example, the antibody can be labeled with a positron-emitting nuclide such as 18F, 55Co, 64Cu, 66Ga, 68Ga, 76Br, 89Zr and 124I. A publicly known method (Acta Oncol. 32, 825-830, 1993) can be used for labeling the anti-PRG-3 antibody with such a positron-emitting nuclide.

After the anti-PRG-3 antibody labeled with a positron-emitting nuclide is administered to a human or animal, the radiation emitted by the radionuclide can be measured from outside the body using a positron emission tomography (PET) device and converted to an image using a computer tomography procedure. PET enables data concerning behavior, etc., in the body of a drug to be obtained non-invasively. With PET the intensity of radiation can be imaged quantitatively as signal strength. By using such a PET procedure it is possible to detect an antigen molecule that is highly expressed in a specific cancer without collecting a sample from the patient. In addition to the above nuclides, the anti-PRG-3 antibody can also be labeled with a short-lived nuclide using a positron-emitting nuclide such as 11C, 13N, 150, 18F, 45Ti, and the like.

Research and development concerning the production of short-lived nuclides with a medical cyclotron using the above nuclides, manufacturing techniques of compounds labeled with short-lived nuclides, and the like is progressing. An anti-PRG-3 antibody can be labeled by a variety of radioisotopes using these techniques. An anti-PRG-3 antibody administered to a patient accumulates in primary and metastatic lesions in accordance with the specificity of the anti-PRG-3 antibody for pathological tissue at each location. If the anti-PRG-3 antibody is labeled with a positron-emitting nuclide, the presence of primary and metastatic lesions can be detected by detecting the radioactivity according to the localization of the radioactivity. For diagnostic applications, a γ-particle or positron emission energy of 25 to 4000 keV can be suitably used. In addition, if a suitable nuclide is selected and administered in a large amount, even a therapeutic effect may be expected. To obtain an anticancer effect from the radiation, a nuclide providing a γ-particle or positron emission energy of 70 to 700 keV can be used.

In another embodiment of the method of the present invention, expression of the PRG-3 gene is detected. The gene detected in the present invention is not particularly limited herein, but the detection of the amount of mRNA is preferred. In the present invention the term "detection" includes both quantitative and qualitative detection. Examples of qualitative detection include the following assay procedures:
an assay simply to determine whether or not PRG-3 mRNA is present;
an assay to determine whether or not a predetermined amount of PRG-3 mRNA or more is present; and
an assay comparing the amount of PRG-3 mRNA with another test sample (for example, a control sample, etc.)

Quantitative detection includes an assay for the concentration of PRG-3 mRNA, an assay for the amount of PRG-3 mRNA, etc.

Any samples that possibly contains PRG-3 mRNA can be used as the test sample in the present invention. A test sample collected from the body of an organism such as a mammal, etc., is preferred. A more preferred test sample is one collected from a human. Specific examples of the test sample include blood, interstitial fluid, plasma, extravascular fluid, cerebrospinal fluid, synovial fluid, pleural fluid, serum, lymph, saliva, urine, tissue, etc. A preferred sample is one obtained from test sample material such as a specimen of fixed tissue or cells collected from the body of an organism or the liquid culture medium of cells.

When using test sample material such as a specimen of fixed tissue or cells collected from the body of an organism or the liquid culture medium of cells, the *in situ* hybridization method can be most suitably used. The *in situ* hybridization method has been developed as a means for verifying the presence or distribution of specific DNA and RNA molecules in cells and tissues, and the expression level. The method relies on the property that a probe nucleic acid having a base sequence complementary to a specified nucleic acid sequence in a cell will hybridize to the DNA or RNA with a high specificity. *in situ* hybridization is used for the detection of DNA, RNA, and the like in cells. The probe is previously labeled with a radioisotope or an antigenic substance (hapten), and then the label is detected so that the site of hybridization can be distinguished. A label involving a radioisotope (RI) can be quite suitably used as the label of the probe. A fluorescent label utilizing the non-radioactive substance biotin, a hapten such as digoxigenin, and the like can also be used as a very suitable example. As a particularly suitable example, a detection method involving fluorescence *in situ* hybridization (FISH) can be used.

The type of cancer to be diagnosed is not particularly limited herein. More specifically, examples include HCC, lung cancer, glioblastoma and the like. In the present invention both primary lesions and metastatic lesions of these cancers can be diagnosed.

Any animal species expressing the PRG-3 protein can be used as the subject in the present invention. For example, it is known that many mammals other than humans such as mice and rats express PRG-3. However, the preferred subject is a human being. When using an animal species other than humans as the subject, the PRG-3 mRNA of that animal species will be detected.

A specific embodiment of the detection method is described below. First, a sample from a subject is prepared. Then the PRG-3 mRNA contained in the sample is detected. In the present invention cDNA synthesized from mRNA can also be detected. In the present invention, when PRG-3 mRNA or cDNA encoding PRG-3 is detected in the test sample, it can be concluded that cancer is most likely present. For example, when the amount of PRG-3 mRNA and cDNA encoding PRG-3 detected in the test sample is greater than in a negative control or in healthy individuals, this finding indicates that the subject has cancer, or will quite likely have cancer in the future.

Methods for detecting mRNA are publicly known. More specifically, a method such as northern blotting, RT-PCR, DNA array, and the like can be used in the present invention.

The above detection method of the present invention can be automated using various automatic detection devices. With automation it is possible to check a large number of samples in a short time.

The present invention provides a diagnostic agent or kit for diagnosing cancer comprising a reagent for detecting the PRG-3 protein in a test sample. The diagnostic agent of the present invention contains at least the anti-PRG-3 antibody.

A kit for the diagnosis of cancer can be prepared by combining the cancer diagnostic agent of the present invention with another component used to detect PRG-3. More specifically, the present invention relates to a kit for the detection of cancer, and this kit may contain an antibody capable of binding to PRG-3 and a reagent that detects the binding between that antibody and PRG-3. It may also contain a control sample comprising a biological sample containing PRG-3. The kit of the present invention may also be accompanied by instructions to explain the assay procedure.

The present invention also provides a diagnostic agent or kit for the diagnosis of cancer comprising a reagent for detecting PRG-3 mRNA or cDNA encoding PRG-3 in a test sample. The diagnostic agent of the present invention contains at least DNA encoding PRG-3 (SEQ ID NO: 59, DNA comprising the base sequence identified as AK000307) or an oligonucleotide of at least 15 nucleotides that is complementary to its complementary strand.

In this case, the term "complementary strand" refers to the opposite strand of one strand of a double-stranded nucleic acid comprising A:T (or U in the case of RNA) and G:C base pairs. Furthermore, the term "complementary" is not limited to a case wherein a sequence is completely complementary in a region of at least 15 continuous nucleotides, provided it has at least 70%, preferably at least 80%, more preferably 90%, and even more preferably 95% or more base sequence homology. The algorithm for determining homology as described herein may be used.

Such an oligonucleotide can be used as a probe and primer for detection and amplification of DNA encoding PRG-3, and as a probe and primer for detection of expression of that DNA. The probe can be used in the form of a substrate in a DNA array.

When that oligonucleotide is used as a primer, the length normally will be 15 bp to 100 bp. Preferably, the length of the primer will be 17 bp to 30 bp. Any oligonucleotides that can amplify at least a part of the DNA encoding PRG-3 or its complementary strand can be used as the primer. When using the oligonucleotide as a primer, the region at the 3' end can be made complementary, and a restriction enzyme recognition sequence, tag sequence, etc., can be added to the 5' end.

Furthermore, any oligonucleotides that specifically hybridizes with at least a part of the DNA encoding PRG-3 or its complementary strand can be used as a probe. The base sequence of the probe used to detect mRNA is selected from base sequences that are complementary to the sense strand of PRG-3. Normally, that probe will have a length of at least 15 bp, preferably 17 bp or more, and more preferably 20 bp or more.

In the present invention the oligonucleotide can suitably labeled and used as a probe. Methods for labeling an oligonucleotide are publicly known. For example, the 5' end of the oligonucleotide may be labeled by phosphorylation using T4 polynucleotide kinase and 32P-labeled ATP as a substrate. Alternatively, a method can be noted wherein DNA polymerase is used to incorporate a labeled substrate base using random hexamer oligonucleotides as primers (the random prime labeling method, etc.) In the random prime labeling method, a Klenow enzyme is utilized as the DNA polymerase. The base can be labeled by a radioisotope such as 32P, a fluorescent dye, biotin, or digoxin and the like.

The oligonucleotide in the present invention, can be prepared, for example, by a commercially available oligonucleotide synthesizer. The probe can be prepared as a double-stranded DNA fragment obtained by restriction enzyme treatment, etc.

In addition to the antibody and oligonucleotide active ingredients, other ingredients such as sterile water, physiological saline, vegetable oil, surfactant, lipid, solubilizing aid, buffer, protein stabilizer (BSA, gelatin, etc.), preservative, blocking solution, reaction solution, reaction stopping solution, reagent for treating the test sample, and the like can be mixed into the above diagnostic agent and kit as needed.

### Preparation of the anti-PRG-3 antibody

The origin, type, and form of the anti-PRG-3 antibody used in the present invention are not limited provided the antibody can bind to the PRG-3 protein. More specifically, a publicly known antibody such as a nonhuman animal antibody (for example, a mouse antibody, rat antibody, camel antibody), human antibody, chimeric antibody, humanized antibody, and the like can be used. In the present invention the anti-PRG-3 antibody can be used as a monoclonal or polyclonal antibody, preferably a monoclonal antibody. Specific binding of the antibody to the PRG-3 protein is preferred.

The anti-PRG-3 antibody used in the present invention can be obtained as either a polyclonal or a monoclonal antibody using publicly known means. A monoclonal antibody of mammalian origin is particularly preferred as the anti-PRG-3 antibody used in the present invention. The term "monoclonal antibody of mammalian origin" includes one produced by a hybridoma and one produced by a host transformed by an expression vector containing the antibody gene using genetic engineering procedures.

Basically, a hybridoma producing a monoclonal antibody can be prepared as described below using publicly known technology. First, immunization is carried out according to conventional immunological methods using the PRG-3 protein as the sensitizing antigen. Hybridomas are obtained by fusing immune cells obtained from an immunized animal with a publicly known parent cell using convention cell fusion procedures. Next, a hybridoma producing an anti-PRG-3 antibody is selected from those hybridomas by screening for a cell producing a target antibody using a conventional screening method.

More specifically, a monoclonal antibody can be prepared as shown below, for example. First, the PRG-3 protein to be used as the sensitizing antigen to obtain the antibody can be obtained by causing expression of the PRG-3 gene. The nucleotide sequence of the PRG-3 gene has been disclosed as NCBI accession number AK000307 (SEQ ID NO: 59), etc. In other words, a suitable host cell is transformed with a publicly known expression vector carrying a sequence encoding PRG-3, and the target human PRG-3 protein is purified from the host cells or culture supernatant using a publicly known method. Purified natural PRG-3 protein can also be used in the same manner. Furthermore, as employed in the present invention, a fusion protein obtained by fusing a desired partial polypeptide of the PRG-3 protein with a different polypeptide as the immunogen can also be used. For example, an antibody Fc fragment, a peptide tag, etc., can be used to produce the fusion protein serving as the immunogen. A vector expressing the fusion protein can be prepared by fusing genes encoding two or more desired polypeptide fragments in-frame and inserting the fusion gene into an expression vector. The method for preparing a fusion protein is described in Molecular Cloning, 2nd ed. (Sambrook J et al., Molecular Cloning 2nd ed., 9.47-9.58, Cold Spring Harbor Lab. Press, 1989).

A PRG-3 protein purified in that manner can be used as the sensitizing antigen for immunization of a mammal. A PRG-3 partial peptide can also be used as the sensitizing antigen. For example, the following kinds of peptides can be used as a sensitizing antigen:
a peptide obtained by chemical synthesis based on the human PRG-3 amino acid sequence;
a peptide obtained by incorporating part of the PRG-3 gene into an expression vector and expressing the same; and
a peptide obtained by degradation of the PRG-3 protein by enzymatic degradation.

The region and the size of a partial peptide of PRG-3 are not particularly limited in the invention. A preferred region can be selected from amino acid sequences constituting the extracellular domains of PRG-3 (in the amino acid sequence of SEQ ID NO: 60, positions 36 to 65, 150 to 200, and 248 to 256). The number of amino acids constituting the peptide serving as the immunogen is at least 3 or more, with 5 or more, or 6 or more being preferred. More specifically, a peptide of 8 to 50, and preferably 10 to 30 residues can be used as the sensitizing antigen.

The mammalian animal to be immunized by the sensitizing antigen is not particularly limited herein. To obtain a monoclonal antibody by cell fusion, the animal to be immunized is preferably selected while considering compatibility with the parent cell to be used for cell fusion. In general, use of a rodent as the immunized animal is preferred. More specifically, a mouse, rat, hamster, or rabbit can be used as the immunized animal. Alternatively, a monkey, etc., can be used as the immunized animal.

The above animal can be immunized by the sensitizing antigen following a publicly known method. As a general method, for example, a mammal can be immunized by intraperitoneal or subcutaneous injection of a sensitizing antigen. More specifically, the sensitizing antigen is administered to the mammal several times every 4 to 21 days. The sensitizing antigen can be diluted to a suitable dilution in phosphate-buffered saline (PBS), physiological saline, etc., and used for immunization. Additionally, the sensitizing antigen can be administered together with an adjuvant. For example, the antigen can be mixed with Freund's complete adjuvant and emulsified to make a sensitizing antigen preparation. Furthermore, a suitable carrier can be used when carrying out immunization with the sensitizing antigen. Especially when a partial peptide with a low molecular weight is used as the sensitizing antigen, it is desirable to attach the sensitizing antigen to a carrier peptide such as albumin, keyhole limpet hemocyanin, and the like and used for immunization.

On the one hand, a monoclonal antibody can be obtained by DNA immunization. DNA immunization is a method for imparting immunostimulation by administering to the immunized animal a vector DNA constructed in a form such that a gene encoding an antigen protein can be expressed in that immunized animal, and then expressing the immunizing antigen in the body of the immunized animal. In comparison with general immunological methods wherein a protein antigen is administered, the following advantages can be expected from DNA immunization:
it is possible to impart immunostimulation while retaining the structure of a membrane protein such as PRG-3; and purification of the immunizing antigen is not necessary.

On the other hand, however, with DNA immunization it is difficult to combine the antigen with an immunostimulatory means such as an adjuvant, etc. Because PRG-3 has the structural characteristic of having a tertiary structure with 6 membrane-spanning regions, it has been predicted that obtaining an immune response in the body with PRG-3 while retaining its natural structure will be very difficult. In fact, with respect to PRG-3 and the PRG family molecules having this common structural feature, it was already known that an antibody that binds to PRG-family molecules cannot be obtained by conventional immunological methods (Biochem. J. 387, 281-293, 1995). Because of such a structural feature, it has been totally unexpected that a monoclonal antibody capable of binding to PRG-3, which is a protein belonging to the PRG family could in fact obtained by DNA immunization.

To obtain the monoclonal antibody of the present invention by DNA immunization, first DNA expressing the PRG-3 protein is administered to the animal to be immunized. DNA encoding PRG-3 can be synthesized by PCR or another publicly known method. The DNA is incorporated into a suitable expression vector and administered to the animal to be immunized. A commercially available expression vector, for example pcDNA3.1, etc., can be used as the expression vector. A generally used method can also be employed as the method for administering the vector to the body. For example, DNA immunization can be carried out by injecting gold particles having the expression vector adhered thereto into a cell with a gene gun.

Based on the findings by the inventors, hybridomas producing antibodies capable of binding to PRG-3 can be obtained efficiently from mice immunized using DNA immunization. More specifically, after DNA immunization, the target hybridomas could be easily obtained in mice that were also administered cells having forced PRG-3 expression. In other words, the preferred method of obtaining the monoclonal antibody of the present invention is carrying out an additional immunization (booster) by cells expressing PRG-3 subsequent to DNA immunization.

After a mammal has been immunized in this manner, and an increase in the desired antibody titer in serum has been observed, immune cells are collected from the mammal and subjected to cell fusion. Spleen cells in particular can be used as the preferred immune cells.

Mammalian myeloma cells can be used as cells to be fused with the above immune cells. It is preferable to impart the myeloma cells with a suitable selection marker for screening. The term "selection marker" refers to a trait such that the cells can survive (or not survive) under a specified culturing condition. Publicly known selection markers include, for example, hypoxanthine guanine phosphoribosyltransferase deficiency (hereinafter, abbreviated as HGPRT deficiency) and thymidine kinase deficiency (hereinafter, abbreviated as TK deficiency). HGPRT- and TK-deficient cells are sensitive to hypoxanthine-aminopterin-thymidine (hereinafter, abbreviated as HAT sensitivity). HAT-sensitive cells cannot undergo DNA synthesis in HAT selection medium and die, but fusing them with normal cells enables them to grow even in HAT selection medium because they can utilize the salvage pathway of the normal cell and continue to synthesize DNA.

HGPRT-deficient and TK-deficient cells can be selected with medium containing 6-thioguanine, 8-azaguanine (hereinafter, abbreviated as 8AG), or 5'-bromodeoxyuridine. Normal cells incorporate these pyrimidine analogues into their DNA and die, but cells deficient in these enzymes can survive in selection medium because they cannot incorporate these pyrimidine analogues. Additionally, a selection marker called G418 resistance imparts resistance to 2-deoxystreptamine-containing antibiotics (gentamycin analogues) via a neomycin resistance gene. Various myeloma cells suitable for cell fusion are publicly known. For example, the following myeloma cells can be used for the production of the monoclonal antibody of the present invention:
P3 (P3x63Ag8.653)(J. Immunol. (1979) 123,1548-1550);
P3×63Ag8U.1 (Current Topics in Microbiology and Immunology (1978) 81, 1-7);
NS-1 (Kohler, G. and Milstein, C. Eur. J. Immunol. (1976) 6, 511-519);
MPC-11 (Margulies. D.H. et al., Cell (1976) 8, 405-415);
SP2/0 (Shulman, M. et al., Nature (1978) 276, 269-270);
FO (de St.Groth, S.F. et al., J. Immunol. Methods (1980) 35, 1-21);
S194 (Trowbridge, I.S. J. Exp. Med. (1978) 148, 313-323); and
R210 (Galfre, G. et al., Nature (1979) 277, 131-133), etc.

Basically, cell fusion between the immune cells and the myeloma cells can be carried out by a publicly known method such as the method of Kohler and Milstein (Kohler, G., and Milstein, C., Methods Enzymol. (1981), 73, 3-46).

Even more specifically, cell fusion can be conducted in a normal nutrient liquid culture medium in the presence of a cell fusion promoter, for example. Polyethylene glycol (PEG), Sendai virus (HVJ), and the like can be used as a fusion promoter. Additionally, an auxiliary substance such as dimethyl sulfoxide can be added if desired to increase fusion efficiency.

The ratio of immune cells and myeloma cells to be used can be arbitrarily established. For example, setting the ratio of immune cells at 1 to 10 times that of myeloma cells is preferred. A liquid culture medium conventionally used for this type of cell culturing such as a RPMI-1640 medium, MEM medium, etc., that is suitable for proliferation of the myeloma cell line can be utilized as the liquid culture medium for cell fusion. A serum-based supplement such as fetal calf serum (FCS) can also be added to the liquid culture medium.

For cell fusion the predetermined amounts of immune cells and myeloma cells are mixed well in the liquid culture medium, and mixed with the PEG solution that has been heated to about 37°C beforehand to form the target fused cells (hybridomas). In the cell fusion method, PEG having an average molecular weight ranging from about 1000 to 6000, for example, can normally be added at a concentration ranging from 30% to 60% (w/v). Next, cell fusion agents, etc., undesirable for hybridoma growth are removed by repeating a procedure of gradually adding the suitable liquid culture medium noted above and removing the supernatant by centrifugation.

Hybridomas obtained as above can be selected by utilizing a selection liquid culture medium suitable for the selection marker of the myeloma cells used for cell fusion. For example, cells having an HGPRT- and TK- deficiency can be selected by culturing in HAT liquid culture medium (a medium containing hypoxanthine-aminopterin-thymidine). In other words, when an HAT sensitive myeloma cell is used for cell fusion, normal cells and cells that have successfully formed fused cells can be selectively grown in HAT medium. Culturing in HAT medium is continued for a long enough time that the cells other than the target hybridomas (i.e., non-fusion cells) die off. More specifically, in general the target hybridomas can be selected by culturing over a range from several days to several weeks. Thereafter, a hybrid producing the target antibody is screened and monocloned by a conventional limiting dilution procedure. Alternatively, an antibody recognizing PRG-3 can be prepared by the method described in International Publication WO03/104453.

Screening for the target antibody and monocloning can be suitably carried out by a screening method based on a publicly known antigen-antibody reaction. For example, an antigen can be attached to a carrier such as beads made of polystyrene, a commercially available 96-well microplate, etc., and reacted with the culture supernatant of the hybridoma. Next, after the carrier is rinsed, a secondary antibody labeled with an enzyme can be reacted. If a target antibody that reacts with the sensitizing antibody is present in the culture supernatant, the secondary antibody will bind to that antibody via the carrier. Finally, by detecting the secondary antibody attached to the carrier, it can be determined whether or not the target antibody is present in the culture supernatant. A hybridoma producing a desired antibody capable of binding to the antigen can be cloned using the limiting dilution procedure, etc. In that process, the immunogen used for immunization or that is one essentially identical to the PRG-3 protein can be suitably used as the antigen. For example, a cell line expressing PRG-3, an extracellular domain of PRG-3, or an oligopeptide comprising a partial amino acid sequence constituting that domain can be used as the antigen.

Alternatively, human lymphocytes sensitized with the antigen can be used to obtain the target antibody instead of the method of obtaining the above hybridoma by immunizing a non-human animal with the antigen. More specifically, first human lymphocytes are sensitized by the PRG-3 protein in *vitro.* Then the immunosensitized lymphocytes are fused with a suitable fusion partner. For example, myeloma cells of human origin that are capable of dividing indefinitely (see Japanese Patent Publication No. H1-59878) can be used for the fusion partner. An anti-PRG-3 antibody thus obtained is a human antibody capable of binding to the PRG-3 protein.

Additionally, an anti-PRG-3 human antibody can be obtained by administering the PRG-3 protein as an antigen to a transgenic animal having a complete repertoire of human antibody genes, or by immunizing such an animal with DNA constructed so that PRG-3 is expressed in that animal. Antibody-producing cells from the immunized animal can be immortalized by a cell fusion treatment with a suitable fusion partner or by a treatment such as infection with the Epstein-Barr virus. A human antibody to the PRG-3 protein can be isolated from the immortalized cells thus obtained (see International Publications WO94/25585, WO93/12227, WO92/03918, and WO94/02602). By further cloning the immortalized cells, it is possible to clone cells that produce an antibody having target reaction specificity. When using a transgenic animal as the immunized animal, the immune system of that animal recognizes human PRG-3 as a foreign substance. As such, a human antibody toward human PRG-3 can easily be obtained.

A hybridoma producing a monoclonal antibody prepared in this manner can be subcultured in a conventional liquid culture medium. Moreover, the hybridoma can be preserved for a very long time in liquid nitrogen.

The hybridoma can be cultured following conventional methods, and the target monoclonal antibody can be obtained from the culture supernatant. Alternatively, it is possible to administer the hybridoma to a biocompatible mammalian animal, grow the hybridoma in the animal, and then obtain the monoclonal antibody from the ascitic fluid. The former method is suitable for obtaining a very pure antibody.

In the present invention an antibody encoded by an antibody gene cloned from an antibody-producing cell can be used. The cloned antibody gene can be expressed as an antibody by incorporating the same into a suitable vector and transfecting a host with the vector. A method for isolating the antibody gene, incorporating it into the vector, and transforming a host cell has already been established (for example, see Vandamme, A.M., et al., Eur. J. Biochem. (1990) 192, 767-775).

For example, cDNA encoding a variable region (V-region) of an anti-PRG-3 antibody can be obtained from a hybridoma cell that produces that anti-PRG-3 antibody. To accomplish that, normally total RNA is first extracted from the hybridoma. The following methods, for example, can be used to extract mRNA from a cell:
Guanidine ultracentrifugation method (Chirgwin, J.M. et al., Biochemistry (1979) 18, 5294-5299); and
AGPC method (Chomczynski, P. et al., Anal. Biochem. (1987) 162, 156-159).

The extracted mRNA can be purified using an mRNA Purification Kit (GE Healthcare Biosciences). Alternatively, kits such as the QuickPrep mRNA Purification Kit (GE Healthcare Biosciences) are commercially available for directly extracting total mRNA from a cell. The total mRNA can be obtained from the hybridoma using such a kit. The cDNA encoding the antibody V-region can be synthesized from the obtained mRNA using a reverse transcriptase. A primer publicly known to persons skilled in the art can be used as the primer. For example, a sequence of 15 to 30 bases selected from a sequence shared with a mouse antibody gene can be used (e.g., cDNA encoding the antibody V-region can be obtained by using primers having the DNA sequences represented by SEQ ID NOs: 61 to 63). The cDNA can be synthesized by an AMV Reverse Transcriptase First-strand cDNA Synthesis Kit (Seikagaku Corporation), etc. Moreover, a 5'-Ampli FINDER RACE Kit (Clontech) and the 5'-RACE method (Frohman, M.A. et al., Proc. Natl. Acad. Sci. USA (1988) 85, 8998-9002; Belyavsky, A. et al., Nucleic Acids Res. (1989) 17, 2919-2932) utilizing PCR can be used for synthesizing and amplifying the cDNA. Additionally, during the process of synthesizing the cDNA a suitable restriction enzyme site described below can be inserted at both ends of the cDNA.

The target cDNA fragment from the obtained PCR product is purified and then ligated to the vector DNA. After such a recombinant vector is prepared, introduced into *E. coli* and the like, and colonies are selected, a desired recombinant vector can be prepared from the colony-forming *E. coli.* Then the nucleotide sequences of the cDNA can be verified by a publicly known method such as dideoxynucleotide chain termination method, etc.

Furthermore, a cDNA library can be used to obtain a gene encoding an antibody variable region. First cDNA is synthesized using the mRNA extracted from an antibody-producing cell as a template to obtain a cDNA library. A commercially available kit can be used for synthesis of the cDNA library. In practice, only a very small amount of mRNA is obtained from a few cells, so the yield is low if that mRNA is purified directly. Therefore, usually mRNA is purified after adding carrier RNA that clearly does not contain the antibody gene. Alternatively, when a set amount of RNA can be extracted, it is possible to extract only the RNA of the antibody-producing cells. For example, the addition of RNA is not necessary in those cases where RNA is extracted from 10 or more, or 30 or more, preferably 50 or more antibody-producing cells.

The antibody gene can be amplified by PCR using the cDNA library as a template. Primers for amplifying an antibody gene by PCR are publicly known. For example, it is possible to design primers for amplification of a human antibody gene based on the disclosures in academic articles (J. Mol. Biol. (1991), 222, 581-597), and the like. These primers comprise nucleotide sequences that differ with each subclass of immunoglobulin. Therefore, all possibilities should be taken into consideration when PCR is carried out using a cDNA library of an unknown subclass as a template.

More specifically, to obtain a gene encoding a human IgG, primers capable of amplifying a gene encoding γ-1 to γ-5 as the heavy chain, and a κ-chain and λ-chain as a light chain can be used. To amplify an IgG variable region gene, generally a primer annealed to a part corresponding to the hinge region is used for the 3' end primer. Primers corresponding to each subclass can be used as the 5' end primer.

The PCR product produced by gene amplification primers of each subclass of heavy chain and light chain can each be used as an independent library. Using libraries synthesized in such a manner, immunoglobulins comprising combinations of heavy and light chains can be reconstructed. The target antibody can be screened based on the binding activity of the reconstructed immunoglobulins toward PRG-3.

For example, when intending to obtain an antibody to PRG-3, it is even more preferable if the binding of the antibody to PRG-3 is specific. An antibody capable of binding to PRG-3 may be screened by the steps of:
(1) contacting an antibody comprising a V-region encoded by the cDNA with PRG-3;
(2) detecting binding between PRG-3 and the antibody; and
(3) selecting an antibody capable of binding to PRG3.
A method for detecting binding between an antibody and PRG-3 is publicly known. More specifically, a test antibody to PRG-3 immobilized on a support is reacted, and then a labeled antibody that recognizes that antibody is reacted. If the labeled antibody on the support is detected after rinsing, binding of that test antibody to PRG-3 can be verified. An enzymatically active protein such as peroxidase, β-galactosidase, and the like, or a fluorescent substance such as FITC, etc., can be used for the label. A fixed specimen of cells expressing PRG-3 can be used to evaluate binding activity of the antibody.

The panning method using a phage vector can be used as the method of screening for an antibody used to indicate binding activity. A screening method utilizing a phage vector is advantageous when the above kind of antibody gene is obtained as a library of heavy-chain and light-chain subclasses. The genes encoding the heavy-chain and light-chain variable regions can be prepared as a single chain Fv (scFv) by linking them with a suitable linker. If a gene encoding the scFv is inserted into a phage vector, a phage expressing the scFv on the surface can be obtained. The phage is brought into contact with the target antigen and the phage bound to that antigen can be recovered. In this way, DNA encoding an scFv with the target binding activity may be recovered. The scFv having the target binding activity can be concentrated by repeating this operation as needed.

The polynucleotide encoding the antibody in the present invention may encode the whole antibody, or it may encode only a part of the antibody. The term "part of the antibody" refers to an arbitrary part of the antibody molecule. Hereinafter, the term "antibody fragment" is also used to refer to part of the antibody. A preferred antibody fragment in the present invention contains the complementarity determination region (CDR) of the antibody. Even more preferably, the antibody fragment of the present invention contains all three CDRs constituting its variable region.

After cDNA encoding the V-region of the target anti-PRG-3 antibody is obtained, the cDNA is digested by restriction enzymes that recognize the restriction enzyme sites inserted at each end of the cDNA. A preferred restriction enzyme recognizes and digests a nucleotide sequence that has little possibility of appearing in the nucleotide sequence constituting the antibody gene. Additionally, a restriction enzyme that produces a sticky end is preferred for inserting one copy of a digested fragment into a vector in the proper direction. An antibody expression vector can be obtained by inserting cDNA encoding the V-region of the anti-PRG-3 antibody digested in such a manner into a suitable expression vector. At that time, a chimeric antibody can be obtained by linking a gene encoding an antibody constant region (C-region) and the gene encoding the above V-region in-frame. Herein, the term "chimeric antibody" refers to one wherein the constant region and the variable region have different origins. Therefore, in addition to a heterogeneic chimeric antibody such as a mouse-human antibody, an allogeneic human-human chimeric antibody is also included in the definition of chimeric antibody in the present invention. The chimeric antibody expression vector can be constructed by inserting a V-region gene into an expression vector wherein DNA encoding the C-region has been incorporated beforehand.

More specifically, for example, an expression vector that carries DNA encoding the desired antibody constant region (C-region) may be constructed to contain at the 5' side of the C-region a restriction enzyme recognition sequence for the restriction enzyme used for digesting the V-region gene. The chimeric antibody expression vector is constructed by digesting both with the same combination of restriction enzymes and linking them in-frame.

To manufacture the anti-PRG-3 antibody used in the present invention, the antibody gene can be incorporated into an expression vector so that it is expressed under the control of an expression regulatory region. The expression regulatory region for expressing the antibody will include, for example, an enhancer and a promoter. Subsequently, a recombinant cell expressing DNA encoding the anti-PRG-3 antibody can be obtained by transforming a suitable host cell with that expression vector.

When expressing the antibody gene, it is possible to incorporate DNA encoding the antibody heavy chain (H-chain) and light chain (L-chain) in different expression vectors. An antibody molecule having both the H-chain and L-chain can be expressed by co-transfecting the same host cells with vectors wherein the H-chain and L-chain have been incorporated. Alternatively, the host cell can be transformed by incorporating the DNA encoding the H-chain and the L-chain into the same expression vector (see International Publication WO94/11523).

Most combinations of host and expression vector for inserting the isolated antibody gene into a suitable host and preparing the antibody are publicly known. Any of these expression systems can be applied in the present invention. If a eucaryotic cell is used as the host, an animal cell, plant cell, or fungus cell can be used. More specifically, the following cells can be listed as animal cells that can be utilized in the present invention:
(1) Mammalian cells: CHO, COS, myeloma, BHK (baby hamster kidney), HeLa, Vero, etc.;
(2) Amphibian cells: *Xenopus laevis* oocytes, etc.; and
(3) Insect cells: sf9, sf21, Tn5, etc.

Alternatively, in the case of plant cells an antibody gene expression system using cells originating in the genus *Nicotiana* such as *Nicotiana tabacum* and the like is publicly known. Callus cultured cells can also be used as transformed plant cells.

Additionally, the following fungus cells can be used:
Yeasts belonging to the genus *Saccharomyces* such as *Saccharomyces cerevisiae* and yeasts belonging to the genus *Pichia* such as methylotrophic yeast (*Pichia pastoris*), etc.; and
Molds belonging to the genus *Aspergillus* such as *Aspergillus niger,* etc.

Alternatively, an antibody gene expression system utilizing procaryotic cells is publicly known. For example, if bacterial cells are to be used, bacteria such as *Escherichia coli, Bacillus subtilis,* and the like can be utilized in the present invention.

When mammalian cells are utilized, a commonly used, useful promoter, the antibody gene to be expressed, and a poly-A signal sequence downstream in the 3' direction can be functionally ligated and expressed. The human cytomegalovirus immediate early promoter and enhancer can be noted as an example of a promoter and enhancer.

Furthermore, a virus promoter and enhancer or a promoter and enhancer originating in mammalian cells such as human elongation factor 1a (HEF1a) can also be used for antibody expression. Specific viruses having a promoter and enhancer that can be utilized are retroviruses, polyoma viruses, adenoviruses, simian virus 40 (SV40) and the like.

When the SV40 promoter and enhancer is used, the method of Mulligan et al. (Nature (1979) 277, 108) can be employed. The HEF1a promoter and enhancer can easily be utilized for expression of the target gene by following the method of Mizushima et al. (Nucleic Acids Res. (1990), 18, 5322).

In the case of *E. coli,* the gene can be expressed by functionally linking a commonly used, useful promoter, a signal sequence for antibody secretion, and the antibody gene to be expressed. The *lacZ* promoter and *araB* promoter can be noted as examples of promoters. When the *lacZ* promoter is used, the method of Ward et al. (Nature (1989) 341, 544-546 and FASEBJ (1992) 6, 2422-2427) can be utilized. Alternatively, the *araB* promoter can be utilized for expression of the target gene by following the method of Better et al. (Science (1988) 240, 1041-1043).

For production in the periplasm of *E*. *coli,* the *pelB* signal sequence (Lei, S.P. et al., J. Bacteriol. (1987) 169, 4379) can be used as the signal sequence for antibody secretion. After isolation of an antibody produced in the periplasm, the antibody structure is refolded by using a protein denaturing agent such as urea and guanidine hydrochloride so that it has the desired binding activity.

When the antibody is produced using animal cells, it is desirable to use an antibody heavy-chain gene or light-chain gene signal sequence as the signal sequence necessary for secretion outside the cell. Alternatively, the signal sequence of a secretory protein such as IL-3, IL-6, and the like can be used.

A replication origin from SV40, polyomavirus, adenovirus, bovine papilloma virus (BPV), and the like can be used as the replication origin to be inserted into the expression vector. Additionally, a selection marker can be inserted into the expression vector for amplification of multiple gene copies in the host cell system. More specifically, the following selection markers can be used:
Aminoglycoside transferase (APH) gene;
thymidine kinase (TK) gene;
*Escherichia coli* xanthine guanine phosphoribosyl transferase (Ecogpt) gene; and
dihydrofolate reductase (dhfr) gene.

The target antibody can be produced by inserting these expression vectors into host cells, and then culturing the transformed host cells either *in vitro* or *in vivo.* The host cells may be cultured according to publicly known methods. For example, DMEM, MEM, RPMI-1640, or IMDM can be used as the liquid culture medium, and a serum-based supplement such as fetal calf serum (FCS) and the like can be used in combination.

The antibody expressed and produced as described above can be purified by using publicly known methods normally used in protein purification either alone or in a suitable combination thereof. For example, the antibody can be isolated and purified by suitably selecting and combining an affinity column such as a protein A column, a chromatography column, and a filter as well as procedures such as ultrafiltration, salting out, and dialysis (Antibodies: A Laboratory Manual. Ed Harlow, David Lane: Cold Spring Harbor Laboratory, 1988).

In addition to the above host cells, a transgenic animal can be used for production of a recombinant antibody. In other words, the target antibody can be obtained from an animal transfected with the gene encoding that antibody. For example, the antibody gene can be constructed as a fusion gene by inserting the antibody gene in-frame within a gene encoding a protein that is specifically expressed in milk. Goat β-casein and the like can be used as the protein that is secreted in milk. A DNA fragment having the fusion gene containing the antibody gene can be injected into a goat embryo and the injected embryo can be implanted into a female goat. The desired antibody can be obtained as a fusion protein with the milk protein from the milk produced by the transgenic goat (or progeny thereof) that is born from the goat implanted with the embryo. Additionally, a hormone can be suitably used in the transgenic goat to increase the amount of milk containing the desired antibody that is produced by the transgenic goat (Ebert, K.M., et al., Bio/Technology (1994) 12, 699-702).

The C-region originating in an antibody of a nonhuman animal can be used as the C-region of the recombinant antibody of the present invention. As the heavy-chain C-region of a mouse antibody, *for example*, Cγ1, Cγ2a, Cγ2b, Cγ3, Cµ, Cd, Ca1, Ca2, and Ce can be used, and Cκ and Cλ can be used as the light-chain C-region. An antibody from an animal other than a mouse such as a rat, rabbit, goat, sheep, camel, monkey, and the like can be also be used. Their sequences are publicly known. The C-region can also be modified to improve the stability of the antibody or the production thereof.

In the present invention, if the antibody is administered to a human it is possible to make a recombinant type of antibody that has been artificially modified to decrease the xenoantigenicity to humans. The term "recombinant type of antibody" includes, for example, a chimeric antibody, a humanized antibody, etc. These modified antibodies can be produced using a publicly known method.

The term "chimeric antibody" refers to an antibody wherein a variable region and a constant region of mutually different origin are linked together. For example, an antibody comprising heavy-chain and light-chain variable regions of a mouse antibody and heavy-chain and light-chain constant regions of a human antibody is a mouse-human chimeric antibody. A recombinant vector expressing a chimeric antibody can be produced by linking DNA encoding the variable regions of a mouse antibody with DNA encoding the constant regions of a human antibody, and incorporating that into an expression vector. A chimeric antibody that is produced in culture can be obtained by culturing recombinant cells transformed by that vector and expressing the incorporated DNA. A human antibody C-region can be used for the C-region of a chimeric antibody and humanized antibody.

For example, in the H-chain Cγ1, Cγ2, Cγ3, Cγ4, Cµ, Cd, Ca1, Ca2, and Ce can be used as the C-region. Additionally, Cκ and Cλ can be used as the C-region of the L-chain. The amino acid sequences of these C-regions and the base sequences encoding each are publicly known. The human antibody C-region can be modified to improve the stability of the antibody itself or the production thereof.

Generally speaking, a chimeric antibody is made up of a C-region originating in a human antibody and a V-region of an antibody of nonhuman animal origin. In contrast, a humanized antibody is made up of a C-region and framework regions (FR) originating in a human antibody, a complementarity determining region (CDR) originating in a nonhuman animal antibody. A humanized antibody is useful as an active ingredient in the therapeutic agent of the present invention because its antigenicity in the human body is decreased.

For example, a mouse-human chimeric antibody obtained by linking the 4F1, 4F8, and 4F12 variable regions of an anti-PRG-3 monoclonal antibody prepared according to the present invention with an amino acid sequence constituting a human constant region is preferred as the monoclonal antibody in the present invention. In other words, the present invention provides a mouse-human chimeric monoclonal antibody consisting of an H-chain and an L-chain comprising the following amino acid sequences:
H-chain: the amino acid sequences represented by SEQ ID NO: 48, SEQ ID NO: 52, and SEQ ID NO: 56 (in the case of a sequence excluding the signal sequence, SEQ ID NOs: 78, 82, and 86, respectively); and
L-chain: the amino acid sequences represented by SEQ ID NO: 50, SEQ ID NO: 54, and SEQ ID NO: 58 (in the case of a sequence excluding the signal sequence, SEQ ID NOs: 80, 84, and 88, respectively).

The variable region of an antibody normally constitutes three complementarity-determining regions (CDR) sandwiched between four framework regions (FR). The CDR is the region that essentially determines the binding specificity of the antibody. The amino acid sequences of CDRs are abundant in variety. In comparison, the amino acid sequences constituting the FRs often exhibit a high degree of homology even among antibodies with different binding specificity. As a result, it is generally said that the binding specificity of a certain antibody can be transplanted to another antibody by transplanting the CDRs of the former.

A humanized antibody is also called a reshaped human antibody. More specifically, a humanized antibody wherein the CDRs of antibody from a nonhuman animal, e.g., a mouse, are transplanted into a human antibody, etc., is publicly known. General gene recombination procedures for obtaining a humanized antibody are also known.

More specifically, overlap extension PCR, for example, is publicly known as a method for transplanting mouse antibody CDRs into human FRs. In overlap extension PCR a nucleotide sequence encoding a mouse CDR to be transplanted is added to a primer for synthesizing a human antibody FR. Primers for each of the four FRs are prepared. In general, in the transplantation of a mouse CR onto a human FR, it is said that selection of a human FR having a high degree of homology with the mouse FR is advantageous for preserving CDR function. In other words, it is preferable to use a human FR comprising an amino acid sequence having a high degree of homology with an FR adjacent to the mouse CDR to be transplanted.

The base sequences to be linked are designed so that they are mutually connected in-frame. The human FRs are synthesized independently by each of the primers. As a result, a product is obtained wherein DNA encoding a mouse CDR is added to each FR. The nucleotide sequences encoding the mouse CDRs of each of the products are designed to mutually overlap. Next, using the human antibody gene as a template, the overlapping CDR members of the synthesized products are annealed, and a complementary strand synthesis reaction is carried out. The human FRs are connected via the mouse CDR sequences by this reaction.

The 5' end and 3' end of the V-region gene wherein three CDRs and four FRs are ultimately linked is annealed, and the whole length is amplified by a primer to which a suitable restriction enzyme recognition sequence has been added. A human antibody expression vector can be prepared by inserting the DNA obtained as described above and DNA encoding the human C-region into an expression vector such that they are linked in-frame. After this vector is inserted into a host and recombinant cells are established, the humanized antibody is produced in the culture product of the cultured cells by culturing the recombinant cells and expressing the DNA encoding the humanized antibody (see European Patent Application Laid-open No. EP 239400 and World Patent Application Laid-open No. WO 96/02576).

By qualitatively and quantitatively measuring and evaluating the binding activity of a humanized antibody prepared in the above matter to an antigen, it is possible to suitably select human antibody FRs that enable the CDRs to form a satisfactory antigen binding site when connected by the CDRs. As needed, amino acid residues of the FRs can be substituted so that the CDRs of the reshaped human antibody will form a suitable antigen binding site. For example, it is possible to insert a mutation in the amino acid sequence of an FR by applying PCR using transplantation of the mouse CDR onto a human FR. More specifically, a partial nucleotide sequence mutation can be inserted into a primer annealing the FR. The base sequence mutation is inserted into the FR synthesized by such a primer. The mutant FR sequences having desirable properties can be selected by measuring and evaluating binding activity of the mutant antibody with the substituted amino acid to the antigen using the methods described above (Sato, K. et al., Cancer Res. 1993, 53, 851-856).

Methods for acquiring a human antibody are also known. For example, human lymphocytes are sensitized *in vitro* by a desired antigen or cells expressing a desired antigen. Next, the desired human antibody capable of binding to the antigen can be acquired by fusing a sensitized lymphocyte with a human myeloma cell (see Japanese Patent Publication H1-59878). For example, U266 cells and the like can be utilized as the human myeloma cell line serving as the fusion partner.

In addition, a desired human antibody can be obtained by immunizing a transgenic animal having a complete repertoire of human antibody genes with a desired antigen (see World Patent Application Laid-open Nos. WO 93/12227, WO 92/03918, WO 94/02602, WO 94/25585, WO 96/34096, and WO 96/33735). Additionally, technology for obtaining a human antibody by panning using a human antibody library is known. For example, a human antibody V-region can be expressed on the surface of a phage as a single stranded antibody (scFv) by the phage display method, and a phage that binds to the antigen can be selected. The DNA sequence encoding the V-region of the human antibody that binds to the antigen can be determined by analyzing the genes of the selected phage. After the DNA sequence of the scFv that binds to the antigen has been determined, an expression vector can be prepared by fusing that V-region sequence with the sequence of a desired human antibody C-region in-frame, and then incorporating the same into a suitable expression vector. That expression vector is inserted into suitable expression cells such as those noted above, and the human antibody can be obtained by expressing the gene encoding that human antibody. These methods are already publicly known (World Patent Application Laid-open Nos. WO 92/01047, WO 92/20791, WO 93/06213, WO 93/11236, WO 93/19172, WO 95/01438, and WO 95/15388).

The antibody of the present invention includes not only a divalent antibody as typified by IgG, but also a monovalent antibody, or a polyvalent antibody as typified by IgM, provided it is capable of binding to the PRG-3 protein. The polyvalent antibody of the present invention includes a polyvalent antibody having antigen binding sites wherein are all the same, and a polyvalent antibody having antigen binding sites wherein part or all are different. The antibody of the present invention is not limited to whole antibody molecules, and a low molecular antibody or its modified form can also be used provided it is capable of binding to the PRG-3 protein.

The term "low molecular antibody" includes an antibody fragment wherein part of a whole antibody (e.g., whole IgG, etc.) is missing. It is known that a partially deficient antibody molecule is still capable of binding to a PRG-3 antigen. The antibody fragment of the present invention preferably includes either a heavy-chain variable region (VH) and a light-chain variable region(VL), or both. The amino acid sequence of the VH or VL can also involve a substitution, deletion, addition, and/or insertion. Additionally, either the VH or VL, or part of both can be missing provided the antibody is capable of binding to a PRG-3 antigen. Moreover, the variable region can be chimerized or humanized. Fab, Fab', F(ab')2, Fv, and the like can be noted as concrete examples of an antibody fragment. Fab, Fab', F(ab')2, Fv, scFv (single chain Fv), diabody, sc(Fv)2 (single chain (Fv)2), and the like can be noted as concrete examples of a low molecular antibody. Multimers (e.g., a dimer, trimer, tetramer, or polymer) of these antibodies are also included in the low molecular antibody of the present invention.

A fragment of an antibody can be obtained by treating the antibody with an enzyme to produce antibody fragments. Papain, pepsin, or plasmin, etc., are publicly known as enzymes that produce antibody fragments. Alternatively, it is possible to construct a gene encoding the antibody fragment, insert the gene into an expression vector, and express the same in a suitable host cell (see, for example, Co, M.S. et al., J. Immunol. (1994) 152, 2968-2976; Better, M. & Horwitz, A.H. Methods in Enzymology (1989) 178, 476-496; Plueckthun, A. & Skerra, A. Methods in Enzymology (1989) 178, 476-496; Lamoyi, E., Methods in Enzymology (1989) 121, 652-663; Rousseaux, J. et al., Methods in Enzymology (1989) 121, 663-669; Bird, R.E. et al., TIBTECH (1991) 9, 132-137).

Digestive enzymes cleave specific sites of the enzyme fragments and provide antibody fragments with the specific structures shown below. Any parts of the antibody can be deleted from these enzymatically obtained antibody fragments by genetic engineering methods:
Papain digestion: F(ab)2 or Fab
Pepsin digestion: F(ab')2 or Fab'
Plasmin digestion: Facb

Therefore, the low molecular antibody in the present invention can be an antibody fragment wherein an arbitrary region is deleted, provided it has binding affinity with PRG-3. Additionally, it is particularly desirable for the antibody to maintain effector activity in the treatment of cancer and other cell growth-related diseases in accordance with the present invention. In other words, the preferred low molecular antibody in the present invention has both binding affinity to PRG-3 and an effector function. The effector function of the antibody includes ADCC activity and CDC activity. It is particularly preferred if the antibody for therapeutic use in the present invention provides ADCC activity and/or CDC activity as an effector function.

The term "diabody" refers to a bivalent antibody fragment constructed by gene fusion (see Holliger, P. et al., Proc. Natl. Acad. Sci. USA 90:6444-6448 (1993); European Patent No. EP 404097; World Patent Application Laid-open No. WO 93/11161, etc.) A diabody is a dimer formed from two polypeptide chains. Normally, each of the polypeptide chains forming a dimer is one wherein a VL and VH are joined by a linker in the same chain. The linker in a diabody is generally so short that the VL and VH cannot bind to each other. More specifically, the amino acid residues that form the linker are about 5 residues long, for example. Therefore, the VL and VH encoded on the same polypeptide chain cannot form a single-chain variable fragment, and they form a dimer with a different single-chain variable fragment. As a result, a diabody has two antigen binding sites.

An scFv is obtained by linking together the H-chain and the L-chain V-regions of an antibody. In an scFv, the H-chain and L-chain V-regions are joined by a linker, preferably a peptide linker (Huston, J.S. et al., Proc. Natl. Acad. Sci. USA, 1988, 85, 5879-5883). The H-chain and L-chain V-regions in an scFv can originate in any of the antibodies described in this description. The linker joining the V-regions is not particularly limited herein. For example, any single chain peptides comprising about 3 to 25 amino acid residues can be used as a linker. More specifically, a peptide linker exemplified below, etc., can be used.

The V-regions of both chains can be linked, for example by the PCR method described above. To link the V-regions by PCR, from among the DNA sequences listed below DNA encoding all or a desired part of an amino acid sequence is first used as a template:
a DNA sequence encoding an H-chain or H-chain V-region of an antibody; and
a DNA sequence encoding an L-chain or L-chain V-region of an antibody.

The DNA sequences encoding the H-chain and L-chain V-regions are each amplified by PCR using a primer pair having sequences corresponding to the sequences at both ends of the DNA to be amplified. Next DNA encoding part of the peptide linker is readied. The DNA encoding the peptide linker can also be synthesized using PCR. A base sequence that can bind to each of the V-region amplification products synthesized separately is added to the 5' end of the primer to be used at that time. Next, a PCR reaction is carried out using primers for assembly PCR for the various DNA molecules to form an [H-chain V-region DNA]-[peptide linker DNA]-[L-chain V-region DNA] molecule.

The primers for assembly PCR consist of a combination of a primer that anneals to the 5' end of the [H-chain V-region DNA] and a primer that anneals to the 3' end of the [L-chain V-region DNA]. In other words, the primers for assembly PCR are a primer set that can amplify DNA encoding the whole scFv sequence to be synthesized. On the other hand, the a sequence that can bind to each V-region DNA is added to the [peptide linker DNA]. As a result, these DNA molecules are linked, and ultimately the whole scFv is also produced as an amplification product by the primers for assembly PCR. Once the DNA encoding the scFv is prepared, an expression vector containing the DNA, and recombinant cells transformed by that expression vector can be obtained in accordance with conventional methods. Additionally, the resulting recombinant cells can be cultured, and DNA encoding the scFv can be expressed to obtain the scFv.

An sc(Fv)2 is a low molecular antibody that is made into a single chain by joining two VH and two VL together via a linker, etc. (Hudson, et al., J. Immunol. Methods 1999, 231: 177-189). An sc(Fv)2 can be prepared by joining two scFv molecules via a linker.

In addition, a preferred antibody is one wherein the two VH and two VL are aligned in the order VH, VL, VH, VL ([VH]-linker-[VL]-linker-[VH]-linker-[VL]) using the N-terminus of a single chain polypeptide as an origin.

The order of the two VH and two VL is not limited to the above configuration, and they can be aligned in any order. For example, the following alignments can be noted:
[VL]-linker-[VH]-linker-[VH]-linker-[VL];
[VH]-linker-[VL]-linker-[VL]-linker-[VH];
[VH]-linker-[VH]-linker-[VL]-linker-[VL];
[VL]-linker-[VL]-linker-[VH]-linker-[VH]; and
[VL]-linker-[VH]-linker-[VL]-linker-[VH].

Any peptide linkers that can be inserted by genetic engineering methods or a synthetic linker (for example, the linker disclosed in Protein Engineering, 9(3), 299-305, 1996) can be used as a linker joining the variable regions of the antibody. A peptide linker is preferred in the present invention. The length of the peptide linker is not particularly limited herein, and it can be suitably selected by a person skilled in the art in accordance with the object. Normally, the number of amino acid residues constituting the peptide linker is 1 to 100 amino acids, preferably 3 to 50 amino acids, more preferably 5 to 30 amino acids, and a length of 12 to 18 amino acids (for example 15 amino acids) is especially preferred.

The amino acid sequence constituting the peptide linker can be of an sequences provided it does not inhibit the binding activity of the scFv. For example, in the case of a peptide linker the following kinds of amino acid sequences can be used:
Ser;
Gly·Ser;
Gly·Gly·Ser;
Ser·Gly·Gly;
Gly·Gly·Gly·Ser (SEQ ID NO: 64);
Ser·Gly·Gly·Gly (SEQ ID NO: 65);
Gly·Gly·Gly·Gly·Ser (SEQ ID NO: 66);
Ser·Gly·Gly·Gly·Gly (SEQ ID NO: 67);
Gly·Gly·Gly·Gly·Gly·Ser (SEQ ID NO: 68);
Ser·Gly·Gly·Gly·Gly·Gly (SEQ ID NO: 69);
Gly·Gly·Gly·Gly·Gly·Gly·Ser (SEQ ID NO: 70);
Ser·Gly·Gly·Gly·Gly·Gly·Gly (SEQ ID NO: 71);
(Gly·Gly·Gly·Gly·Ser (SEQ ID NO: 66))n; and
(Ser·Gly·Gly·Gly·Gly (SEQ ID NO: 67))n.
[wherein n is an integer of 1 or more]

The amino acid sequence of the peptide linker can be suitably selected by a person skilled in the art in accordance with the object. For example the integer n, which determines the length of the above peptide linker, is normally 1 to 5, preferably 1 to 3, and more preferably 1 or 2.

Therefore, the following example of an sc(Fv)2 can be noted as an embodiment of sc(Fv)2 that is particularly preferred in the present invention.
[VH]-peptide linker (15 amino acids)-[VL]-peptide linker (15 amino acids)-[VH]-peptide linker (15 amino acids)-[VL]

Alternatively, the V-regions can be linked using a synthetic chemical linker (crosslinking agent). A crosslinking agent that is normally used to crosslink peptide compounds, etc., can be used in the present invention. Examples of chemical crosslinking agents that are publicly known and commercially available are as follows:
N-hydroxysuccinimide (NHS);
disuccinimidyl suberate (DSS);
bis (sulfosuccinimidyl) suberate (BS3);
dithiobis (succinimidyl propionate) (DSP);
dithiobis (sulfosuccinimidyl propionate) (DTSSP);
ethylene glycol bis (succinimidyl succinate) (EGS);
ethylene glycol bis (sulfosuccinimidyl succinate) (sulfo-EGS);
disuccinimidyl tartrate (DST);
disulfosuccinimidyl tartarate (sulfo-DST);
bis [2-(succinimidooxycarbonyloxy) ethyl] sulfone [BSOCOES]; and
bis [2-sulfosuccinimidooxycarbonyloxy) ethyl] sulfone (sulfo-BSOCOES).

Normally three linkers are required when four antibody variable regions are joined together. The multiple linkers can be the same or can be different linkers. The preferred low molecular antibody in the present invention is a diabody or sc(Fv)2. To obtain such a low molecular antibody, an antibody can be treated with an enzyme such as papain, pepsin, etc., to produce antibody fragments, or DNA encoding these antibody fragments can be constructed and after it is inserted into an expression vector, it can be expressed in a suitable host cell (see, for example, Co, M.S. et al., J. Immunol. (1994) 152, 2968-2976; Better, M. & Horwitz, A.H. Methods Enzymol. (1989) 178, 476-496, Plueckthun, A. & Skerra, A. Methods Enzymol. (1989) 178, 497-515; Lamoyi, E., Methods Enzymol. (1989) 121, 652-663; Rousseaux, J. et al., Methods Enzymol (1986) 121, 663-669; Bird, R.E. and Walker, B.W., Trends Biotechnol. (1991) 9, 132-137).

Any antibodies recognizing PRG-3 can be used as the antibody of the present invention. For example, the antibodies described in (1) to (34) below can be listed as preferred antibodies. These antibodies can be, for example, whole antibodies, low molecular antibodies, animal antibodies, chimeric antibodies, humanized antibodies, or human antibodies.
(1) An antibody comprising an H-chain having the amino acid sequence represented by SEQ ID NO: 2 (H-chain CDR1 sequence of the 4F1 antibody) as CDR1, the amino acid sequence represented by SEQ ID NO: 4 (H-chain CDR2 sequence of the 4F1 antibody) as CDR2, and the amino acid sequence represented by SEQ ID NO: 6 (H-chain CDR3 sequence of the 4F1 antibody) as CDR3;
(2) An antibody comprising the H-chain according to (1) having the amino acid sequence represented by SEQ ID NO: 8 (CH sequence of the 4F1 antibody) as a CH (H-chain constant region);
(3) An antibody comprising the H-chain according to (1) having the amino acid sequence represented by SEQ ID NO: 10 (CH sequence of the 4F1 mouse-human chimeric antibody) as a CH;
(4) An antibody comprising an L-chain having the amino acid sequence represented by SEQ ID NO: 12 (L-chain CDR1 sequence of the 4F1 antibody) as CDR1, the amino acid sequence represented by SEQ ID NO: 14 (L-chain CDR2 sequence of the 4F1 antibody) as CDR2, and the amino acid sequence represented by SEQ ID NO: 16 (L-chain CDR3 sequence of the 4F1 antibody) as CDR3;
(5) An antibody comprising the L-chain according to (4) having the amino acid sequence represented by SEQ ID NO: 18 (CL sequence of the 4F1 antibody) as a CL;
(6) An antibody comprising the L-chain according to (4) having the amino acid sequence represented by SEQ ID NO: 20 (CL sequence of the 4F1 mouse-human chimeric antibody) as a CL;
(7) An antibody comprising the H-chain according to (1) and the L-chain according to (4);
(8) An antibody comprising the H-chain according to (2) and the L-chain according to (5);
(9) An antibody comprising the H-chain according to (3) and the L-chain according to (6);
(10) An antibody wherein one or more amino acids is substituted, deleted from, added to, and/or inserted into the antibody according to any of (1) to (9), and that has activity equivalent to the antibody according to any of (1) to (9);
(11) An antibody capable of binding to the same epitope as the epitope on the PRG-3 protein to which the antibody according to any of (1) to (10) is capable of binding;
(12) An antibody comprising an H-chain having the amino acid sequence represented by SEQ ID NO: 22 (H-chain CDR1 sequence of the 4F8 antibody) as CDR1, the amino acid sequence represented by SEQ ID NO: 24 (H-chain CDR2 sequence of the 4F8 antibody) as CDR2, and the amino acid sequence represented by SEQ ID NO: 26 (H-chain CDR3 sequence of the 4F8 antibody) as CDR3;
(13) An antibody comprising the H-chain according to (12) having the amino acid sequence represented by SEQ ID NO: 34 (CH sequence of the 4F8 antibody) as a CH;
(14) An antibody comprising the H-chain according to (12) having the amino acid sequence represented by SEQ ID NO: 10 as a CH;
(15) An antibody comprising an L-chain having the amino acid sequence represented by SEQ ID NO: 28 (L-chain CDR1 sequence of the 4F8 antibody) as CDR1, the amino acid sequence represented by SEQ ID NO: 30 (L-chain CDR2 sequence of the 4F8 antibody) as CDR2, and the amino acid sequence represented by SEQ ID NO: 32 (L-chain CDR3 sequence of the 4F8 antibody) as CDR3;
(16) An antibody comprising the L-chain according to (15) having the amino acid sequence represented by SEQ ID NO: 18 as a CL;
(17) An antibody comprising the L-chain according to (15) having the amino acid sequence represented by SEQ ID NO: 20 as a CL;
(18) An antibody comprising the H-chain according to (12) and the L-chain according to (15);
(19) An antibody comprising the H-chain according to (13) and the L-chain according to (16);
(20) An antibody comprising the H-chain according to (14) and the L-chain according to (17);
(21) An antibody wherein one or more amino acids is substituted, deleted from, added to, and/or inserted into the antibody according to any of (12) to (20), and that has activity equivalent to the antibody according to any of (12) to (20);
(22) An antibody capable of binding to the same epitope as the epitope on the PRG-3 protein to which the antibody according to any of (12) to (21) is capable of binding;
(23) An antibody comprising an H-chain having the amino acid sequence represented by SEQ ID NO: 36 (H-chain CDR1 sequence of the 4F12 antibody) as CDR1, the amino acid sequence represented by SEQ ID NO: 38 (H-chain CDR2 sequence of the 4F12 antibody) as CDR2, and the amino acid sequence represented by SEQ ID NO: 40 (H-chain CDR3 sequence of the 4F12 antibody)as CDR3;
(24) An antibody comprising the H-chain according to (23) having the amino acid sequence represented by SEQ ID NO: 34 as a CH;
(25) An antibody comprising the H-chain according to (23) having the amino acid sequence represented by SEQ ID NO: 10 as a CH;
(26) An antibody comprising an L-chain having the amino acid sequence represented by SEQ ID NO: 42 (L-chain CDR1 sequence of the 4F12 antibody) as CDR1, the amino acid sequence represented by SEQ ID NO: 44 (L-chain CDR2 sequence of the 4F12 antibody) as CDR2, and the amino acid sequence represented by SEQ ID NO: 46 (L-chain CDR3 sequence of the 4F12 antibody) as CDR3;
(27) An antibody comprising the L-chain according to (26) having the amino acid sequence represented by SEQ ID NO: 18 as a CL;
(28) An antibody comprising the L-chain according to (26) having the amino acid sequence represented by SEQ ID NO: 20 as a CL;
(29) An antibody comprising the H-chain according to (23) and the L-chain according to (26);
(30) An antibody comprising the H-chain according to (24) and the L-chain according to (27);
(31) An antibody comprising the H-chain according to (25) and the L-chain according to (28);
(32) An antibody wherein one or more amino acids is substituted, deleted from, added to, and/or inserted into the antibody according to any of (23) to (31), and that has activity equivalent to the antibody according to any of (23) to (31);
(33) An antibody capable of binding to the same epitope as the epitope on the PRG-3 protein to which the antibody according to any of (23) to (32) is capable of binding;
(34) A low molecular antibody of the antibody according to any of (1) to (33).

A VH having an amino acid sequence represented by SEQ ID NO: 48 (VH sequence of the 4F1 antibody) (SEQ ID NO: 78 without the signal sequence) can be listed as the VH in the H-chain having the following CDRs described in (1) above:
CDR1: the amino acid sequence represented by SEQ ID NO: 2 (H-chain CDR1 sequence of the 4F1 antibody);
CDR2: the amino acid sequence represented by SEQ ID NO: 4 (H-chain CDR2 sequence of the 4F1 antibody); and
CDR3: the amino acid sequence represented by SEQ ID NO: 6 (H-chain CDR3 sequence of the 4F1 antibody).

A VL having an amino acid sequence represented by SEQ ID NO: 50 (VL sequence of the 4F1 antibody) (SEQ ID NO: 80 without the signal sequence) can be listed as the VL in the L-chain having the following CDRs described in (4) above:
CDR1: the amino acid sequence represented by SEQ ID NO: 12 (L-chain CDR1 sequence of the 4F1 antibody);
CDR2: the amino acid sequence represented by SEQ ID NO: 14 (L-chain CDR2 sequence of the 4F1 antibody); and
CDR3: the amino acid sequence represented by SEQ ID NO: 16 (L-chain CDR3 sequence of the 4F1 antibody).

A VH having an amino acid sequence represented by SEQ ID NO: 52 (VH sequence of the 4F8 antibody) (SEQ ID NO: 82 without the signal sequence) can be listed as the VH in the H-chain having the following CDRs described in (12) above:
CDR1: the amino acid sequence represented by SEQ ID NO: 22 (H-chain CDR1 sequence of the 4F8 antibody);
CDR2: the amino acid sequence represented by SEQ ID NO: 24 (H-chain CDR2 sequence of the 4F8 antibody); and
CDR3: the amino acid sequence represented by SEQ ID NO: 26 (H-chain CDR3 sequence of the 4F8 antibody).

A VL having an amino acid sequence represented by SEQ ID NO: 54 (VL sequence of the 4F8 antibody) (SEQ ID NO: 84 without the signal sequence) can be listed as the VL in the L-chain having the following CDRs described in (15) above:
CDR1: the amino acid sequence represented by SEQ ID NO: 28 (L-chain CDR1 sequence of the 4F8 antibody);
CDR2: the amino acid sequence represented by SEQ ID NO: 30 (L-chain CDR2 sequence of the 4F8 antibody); and
CDR3: the amino acid sequence represented by SEQ ID NO: 32 (L-chain CDR3 sequence of the 4F8 antibody).

A VH having an amino acid sequence represented by SEQ ID NO: 56 (VH sequence of the 4F12 antibody) (SEQ ID NO: 86 without the signal sequence) can be listed as the VH in the H-chain having the following CDRs described in (23) above:
CDR1: the amino acid sequence represented by SEQ ID NO: 36 (H-chain CDR1 sequence of the 4F12 antibody);
CDR2: the amino acid sequence represented by SEQ ID NO: 38 (H-chain CDR2 sequence of the 4F12 antibody); and
CDR3: the amino acid sequence represented by SEQ ID NO: 40 (H-chain CDR3 sequence of the 4F12 antibody).

A VL having an amino acid sequence represented by SEQ ID NO: 58 (VL sequence of the 4F12 antibody) (SEQ ID NO: 88 without the signal sequence) can be listed as the VL in the L-chain having the following CDRs described in (26) above:
CDR1: the amino acid sequence represented by SEQ ID NO: 42 (L-chain CDR1 sequence of the 4F12 antibody);
CDR2: the amino acid sequence represented by SEQ ID NO: 44 (L-chain CDR2 sequence of the 4F12 antibody); and
CDR3: the amino acid sequence represented by SEQ ID NO: 46 (L-chain CDR3 sequence of the 4F12 antibody).

A preferred embodiment of the antibody according to (10), (21), or (32) above is an antibody wherein no modification has occurred in the CDR. As an example, among the antibodies according to (10) above, the preferred embodiment of "an antibody wherein one or more amino acids is substituted, deleted from, added to, and/or inserted into the antibody according to (1), and that has activity equivalent to the antibody according to (1)" is "an antibody wherein one or more amino acids is substituted, deleted from, added to, and/or inserted into the antibody according to (1), that has activity equivalent to the antibody according to (1), and that comprises an H-chain having the amino acid sequence represented by SEQ ID NO: 2 as CDR1, the amino acid sequence represented by SEQ ID NO: 4 as CDR2, and the amino acid sequence represented by SEQ ID NO: 6 as CDR3." Among the antibodies according to (10) above, preferred modes of the other antibodies can be similarly expressed. In the present invention, the concept of having activity equivalent to an antibody of the present invention means that the binding activity toward PRG-3 and/or cytotoxic activity toward a cell expressing PRG-3 will be equivalent to the activity of a specified antibody.

The method for introducing a mutation into a polypeptide is one well known to persons skilled in the art for preparing a polypeptide that is functionally equivalent to a certain peptide. For example, a person skilled in the art can prepare an antibody that is functionally equivalent to the antibody of the present invention by introducing a suitable mutation into the antibody using a site-specific mutation induction method (Hashimoto-Gotoh, T. et al. (1995) Gene 152, 271-275; Zoller, MJ, and Smith, M. (1983) Methods Enzymol. 100, 468-500; Kramer, W. et al. (1984) Nucleic Acids Res. 12, 9441-9456; Kramer W, and Fritz HJ (1987) Methods. Enzymol. 154, 350-367; Kunkel, TA (1985) Proc Natl Acad Sci USA. 82, 488-492; Kunkel (1988) Methods Enzymol. 85, 2763-2766), etc. Furthermore, mutations in amino acids can occur in the natural world. Thus, the antibody of the present invention also includes an antibody having an amino acid sequence wherein a mutation is present in one or a plurality of amino acids in the amino acid sequence of the antibody of the present invention, and is functionally equivalent.

The number of mutated amino acids in such a mutant form is normally 50 amino acids or fewer, preferably 30 amino acids or fewer, and even more preferably 10 amino acids or fewer (for example, 5 amino acids or fewer).

It is desirable that the mutated amino acid residue be substituted with a different amino acid that conserves the properties of the amino acid side chain. For example, the following classification has been established based on amino acid side-chain properties.
Hydrophobic amino acids (A, I, L, M, F, P, W, Y, V)
Hydrophilic amino acid (R, D, N, C, E, Q, G, H, K, S, T)
Amino acids with an aliphatic side chain (G, A, V, L, I, P)
Amino acids with a hydroxyl-containing side chain (S, T, Y)
Amino acids with a side chain containing a sulfur atom (C, M)
Amino acids with a side chain containing a carboxyl and an amide group (D, N, E, Q)
Amino acids with a basic side chain (R, K, H)
Amino acids with a side chain containing an aromatic compound (H, F, Y, W)
(Letters in parentheses are the one-letter abbreviations of amino acids.)

A polypeptide having an amino acid sequence wherein one or more amino acids is substituted, deleted from, added to, and/or inserted into a certain amino acid sequence that retains the original biological activity is already known (Mark, D.F. et al., Proc. Natl. Acad. Sci. USA (1984) 81, 5662-5666; Zoller, M.J. and Smith, M., Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413). In other words, generally speaking, in an amino acid constituting a certain polypeptide, when amino acids classified into each group are mutually substituted, it will be very likely that the activity of that polypeptide will be retained. Substitution of amino acids within the same group in the aforementioned amino acid groups is referred to as conservative substitution in the present invention.

Moreover, the present invention also provides an antibody capable of binding to the same epitope as the epitope to which the anti-PRG-3 antibody disclosed in the present invention binds. More specifically, the present invention relates to an antibody that recognizes the same epitope as the epitope recognized by the 4F1, 4F8, or 4F12 antibody, and to the utility of such an antibody. Such an antibody can be obtained by the methods described below, for example.

Whether a test antibody shares an epitope with certain antibody can be determined by competition for the same epitope between both. The competition between antibodies is detected by a cross-blocking assay and the like. A competitive ELISA assay, for example, is the preferred cross-blocking assay.

More specifically, in a cross-blocking assay, after preincubation of microtiter plate wells coated with the PRG-3 protein either in the presence or absence of a candidate competing antibody, the anti-PRG-3 antibody of the present invention is added. The amount of anti-PRG-3 antibody of the present invention that binds to the PRG-3 protein in a well correlates indirectly with the binding capacity of the candidate competing antibody (test antibody) that competes in binding to the same epitope. In other words, as the affinity of a test antibody to the same epitope increases, the amount of binding of the anti-PRG-3 antibody of the present invention decreases in a well coated with the PRG-3 protein, and the amount of binding of the test antibody increases in a well coated with the PRG-3 protein.

The amount of antibody bound in the well can be easily measured by labeling the antibody beforehand. For example, a biotin-labeled antibody can be measured by using an avidin peroxidase conjugate and a proper substrate. A cross-blocking assay using an enzyme label such as peroxidase is particularly referred to as a competitive ELISA assay. The antibody can be labeled by other labels that can be detected or measured. More specifically, radiolabels, fluorescent labels and the like are publicly known.

In addition, when a test antibody has a constant region originating in a different species from the anti-PRG-3 antibody of the present invention, any antibody bound in the well can be measured by a labeled antibody that recognizes the constant region. Alternatively, even when the antibody originates in the same species, the bound antibody in the well can be measured with an antibody that can distinguish between classes if the class is different.

If a candidate antibody can block binding of the anti-PRG-3 antibody at least 20%, preferably at least 20 to 50%, and even more preferably at least 50% compared to the binding activity obtained in a control test run in the absence of the candidate competing antibody, then the candidate competing antibody is either one which can bind to essentially the same epitope as the anti-PRG-3 antibody of the present invention or one which can competes with the antibody of the invention in binding to the same epitope. Examples of an antibody capable of binding to the same epitope as the anti-PRG-3 antibody include, but not limited to, the antibodies listed in (11), (22), and (33) above.

Moreover, as noted previously, the antibody described in (1) to (34) above includes not only a univalent antibody but also a polyvalent antibody. The term "polyvalent antibody" of the present invention includes a polyvalent antibody having antigen binding sites that are all the same and a polyvalent antibody having antigen binding sites that are partly or all different.

An antibody that binds to human PRG-3 but not to mouse PRG-3 can be noted as one preferred embodiment of anti-PRG-3 antibody used in the present invention. In the present invention, "an antibody that binds to human PRG-3 but not to mouse PRG-3" refers to an antibody wherein normally the binding activity to mouse PRG-3 is 50% less, preferably 30% or less, and even more preferably 10% or less than its binding activity to human PRG-3.

Various molecules such as polyethylene glycol (PEG) and bound antibodies can be used as a modifier of the antibody. Moreover, a chemotherapeutic agent, a toxic peptide or a radiochemical and the like can also be conjugated to the antibody. Such an antibody modifier (hereinafter, called an antibody conjugate) can be obtained by a chemical modification on the obtained antibody. Antibody modification methods have already been established in this field. As described below, an antibody can be obtained as a designed molecular form such as a bispecific antibody using gene recombination technology such that the antibody will recognize not only the PRG-3 protein but also a chemotherapeutic agent, a toxic peptide, a radiochemical and the like. These antibodies are included in the term "antibody" in the present invention.

The following chemotherapeutic agents can be listed as examples of a chemotherapeutic agent that can be combined with the anti-PRG-3 antibody and exhibiting a cytotoxic activity: azaribine, anastrozole, azacytidine, bleomycin, bortezomib, bryostatin-1, busulfan, camptothecin, 10-hydroxycamptothecin, carmustine, celebrex, chlorambucil, cisplatin, irinotecan, carboplatin, cladribine, cyclophosphamide, cytarabine, dacarbazine, docetaxel, dactinomycin, daunomycin glucuronide, daunorubicin, dexamethasone, diethylstilbestrol, doxorubicin, doxorubicin glucuronide, epirubicin, ethinyl estradiol, estramustine, etoposide, etoposide glucuronide, floxuridine, fludarabine, flutamide, fluorouracil, fluoxymesterone, gemcitabine, hydroxyprogesterone caproate, hydroxyurea, idarubicin, ifosfamide, leucovorin, lomustine, mechlorethamine, medroxyprogesterone acetate, megestrol acetate, melphalan, mercaptopurine, methotrexate, mitoxantrone, mithramycin, mitomycin, mitotane, phenylbutyrate, prednisone, procarbazine, paclitaxel, pentostatin, semustine, streptozocin, tamoxifen, taxanes, taxol, testosterone propionate, thalidomide, thioguanine, thiotepa, teniposide, topotecan, uracil mustard, vinblastine, vinorelbine, and vincristine.

A preferred chemotherapeutic agent in the present invention is one with a low molecular weight. A chemotherapeutic with a low molecular weight has little possibility of interfering with antibody function when conjugated with the antibody. A chemotherapeutic agent with a low molecular weight in the present invention normally has a molecular weight of 100 to 2000, and preferably 200 to 1000. All of the chemotherapeutic agents listed herein are chemotherapeutic agents with a low molecular weight. These chemotherapeutic agents in the present invention also include prodrugs that are converted to active chemotherapeutic agents in the body. The activation of the prodrug can be achieved by enzymatic conversion or by non-enzymatic conversion.

Moreover, the antibody can be modified by a toxic peptide such as ricin, abrin, ribonuclease, onconase, DNase I, *Staphylococcus* enterotoxin A, pokeweed antivirus protein, gelonin, diphtheria toxin, *Pseudomonas aeruginosa* exotoxin, *Pseudomonas aeruginosa* endotoxin, L-Asparaginase, and PEG L-Asparaginase. Moreover, in another embodiment it is possible combine and use one or more low molecular weight chemotherapeutic agents and toxic peptides for the modification of the antibody. Covalent bonding or noncovalent bonding can be used to attach the aforementioned low molecular weight chemotherapeutic agent to the anti-PRG-3 antibody. Methods for producing an antibody conjugated with these chemotherapeutic agents are publicly known.

In addition, the protein-based medicine and toxin can be attached to the antibody by a genetic engineering method. More specifically, a recombinant vector can be constructed by fusing DNA encoding the aforementioned toxic peptide and DNA encoding the anti-PRG-3 antibody in-frame and incorporating the same into an expression vector. An anti-PRG-3 antibody conjugated with a toxic peptide can be obtained by culturing transformed cells obtained by inserting that vector into suitable host cells and expressing the DNA incorporated therein. In general, when obtaining a fusion protein containing an antibody, the protein drug and toxin are arranged on the C-terminal side of the antibody. A peptide linker can also be placed between the antibody and the protein drug and toxin.

Additionally, the antibody of the present invention can also be a bispecific antibody. The term "bispecific antibody" refers to an antibody having variable regions within the same antibody molecule that recognize different epitopes. In the present invention the bispecific antibody can have antigen binding sites that recognize different epitopes on the PRG-3 molecule. With such a bispecific antibody, two antibody molecules can bind to one PRG-3 molecule, whereby a more potent cytotoxic effect can be expected.

Alternatively, a bispecific antibody can be constructed wherein one antigen binding site recognizes PRG-3 and another antigen binding site recognizes a cytotoxic substance. More specifically, the term "cytotoxic substance" includes a chemotherapeutic agent, a toxic peptide, or a radiochemical, etc. Such a bispecific antibody is capable of binding to a cell expressing PRG-3 on the one hand and captures a cytotoxic substance on the other. Thus, the cytotoxic substance can directly act on a cell expressing PRG-3. In other words, it is possible to specifically damage tumor cells and inhibit tumor cell growth with a bispecific antibody that recognizes a cytotoxic substance.

A bispecific antibody that recognizes an antigen other than PRG-3 can also be constructed. For example, a bispecific antibody can be constructed that recognizes an antigen other than PRG-3 that is specifically expressed on the surface of a targeted cancer cell in the same manner as PRG-3.

Methods for manufacturing a bispecific antibody are publicly known. For example, a bispecific antibody can be prepared by combining two types of antibodies that recognize different antigens. The combined antibody can be one-half molecules each having an H-chain and an L-chain, or can be one-fourth molecules comprising only H-chains. Alternatively, hybridomas producing different monoclonal antibodies can be fused, and a bispecific antibody-producing fusion cell can be prepared. In addition, the bispecific antibody can be prepared by genetic engineering methods.

A publicly known means can be used for the measurement of antigen binding activity of the antibody (Antibodies: A Laboratory Manual., Ed Harlow and David Lane, Cold Spring Harbor Laboratory, 1988). For example, ELISA (enzyme-liked-immunosorbent assay), EIA (enzyme immunoassay), RIA (radioimmunoassay) or a fluorescent immunoassay and the like can be used.

The antibody of the present invention can be an antibody wherein a sugar chain has been modified. It is known that the cytotoxic activity of an antibody can be enhanced by modifying its sugar chain. For example, the following antibodies are publicly known as an antibody with a modified sugar chain:
an antibody modified by glycosylation (International Publication WO99/54342 and the like);
an antibody wherein a fucose attached to the sugar chain is missing (International Publications WO00/61739, WO02/31140, and the like); and
an antibody with a sugar chain that has bisecting GlcNAc (International Publication WO02/79255 and the like).

The antibody of the present invention is preferably an antibody that has cytotoxic activity.

Antibody-dependent cell-mediated cytotoxicity (ADCC), complement-dependent cytotoxicity (CDC) and the like can be noted as examples of cytotoxic activity in the present invention. In the present invention, CDC activity means cytotoxic activity via the complement system. On the other hand, ADCC activity refers to a damage to the target cells where an antibody specific to a cell surface antigen attaches to a target cell, and a cell with an Fcγ receptor (immunocyte etc.) binds to the Fc member of the antibody through the Fcγ receptor.

Whether or not an anti-PRG-3 antibody has ADCC activity or CDC activity can be determined by a publicly known method (For example, Current Protocols in Immunology, Chapter 7. Immunologic studies in humans, Editor, John E. Coligan, et al., John Wiley & Sons, Inc. (1993), etc.)

More specifically, first effector cells, complement solution, and target cells are prepared.

### (1) Preparation of effector cells

The spleen is removed from a CBA/N mouse and the like, and spleen cells are isolated in RPMI-1640 medium (Invitrogen). The effector cells are prepared by rinsing the cells in the same medium containing 10% fetal bovine serum (FBS, HyClone) and adjusting the concentration to 5 × 10⁶ cells/mL.

### (2) Preparation of complement solution

Baby rabbit complement (Cedarlane) can be diluted tenfold with medium containing 10% FBS (Invitrogen) to prepare the complement solution.

### (3) Preparation of target cells

Target cells can be radiolabeled by culturing cells that express the PRG-3 protein in DMEM medium containing 10% FBS for 1 hour at 37°C together with 0.2 mCi of sodium chromate-51Cr (GE Healthcare Bio Science). Cells transformed by a gene encoding the PRG-3 protein, HCC cells, metastatic liver cancer cells, lung cancer cells, colon cancer cells, and glioblastoma cells, etc., can be used as the cells that express the PRG-3 protein. After the radioactive labeling, the target cells can be prepared by rinsing the cells three times with RPMI-1640 medium containing 10% FBS and adjusting the cell concentration to 2 × 10⁵ cells/mL.

The ADCC activity or CDC activity can be measured by the following methods. For the measurement of the ADCC activity, 50 µL each of the target cells and the anti-PRG-3 antibody are added to a 96-well U-bottomed plate (Becton Dickinson) and reacted for 15 minutes on ice. Thereafter 100 µL of effector cells are added, and the cells are cultured for 4 hours in a carbon dioxide incubator. The final antibody concentration is set at 0 or 10 µg/mL. After culturing, 100 µL of supernatant is collected and radioactivity is measured with a gamma counter (COBRAII AUTO-GAMMA, MODEL D5005, Packard Instrument Company). Cytotoxic activity (%) can be calculated by using the obtained values based on the formula (A-C)/(B-C) × 100. In the formula, A represents the radioactivity (cpm) in each test sample, B represents the radioactivity (cpm) in a test sample to which 1% NP-40 (Nacalai Tesque) has been added, and C represents the radioactivity (cpm) of a test sample including only the target cells.

For the measurement of the CDC activity, on the other hand, 50 µL each of the target cells and the anti-PRG-3 antibody are added to a 96-well flat-bottomed plate (Becton Dickinson) and reacted for 15 minutes on ice. Thereafter 100 µL of complement solution are added, and the cells are cultured for 4 hours in a carbon dioxide incubator. The final antibody concentration is set at 0 or 3 µg/mL. After culturing, 100 µL of supernatant is collected and radioactivity is measured with a gamma counter. Cytotoxic activity can be calculated in the same way as in the measurement of ADCC activity.

For the measurement of cytotoxic activity by an antibody conjugate, 50 µL each of the target cells and the anti-PRG-3 antibody conjugate are added to a 96-well flat-bottomed plate (Becton Dickinson) and reacted for 15 minutes on ice. The cells are cultured for 1 to 4 hours in a carbon dioxide incubator. The final antibody concentration is set at 0 or 3 µg/mL. After culturing, 100 µL of supernatant is collected and radioactivity is measured with a gamma counter. Cytotoxic activity can be calculated in the same way as in the measurement of ADCC activity.

Moreover, the present invention provides a polynucleotide that encodes the antibody of the present invention or a polynucleotide that encodes an antibody that hybridizes under stringent conditions with that polynucleotide and has activity equivalent to the antibody of the present invention. Moreover, the present invention provides a vector incorporating these polynucleotides and a transformant (including a transformed cell) containing that vector.

The polynucleotide of the present invention is a polymer comprising a plurality of deoxyribonucleic acid (DNA) or ribonucleic acid (RNA) bases or base pairs, and is not particularly limited herein provided it encodes the antibody of the present invention. The polynucleotide of the present invention may contain non-naturally occurring bases.

The polynucleotide of the present invention can be used when expressing the antibody by a genetic engineering method. Moreover, it can be used as a probe when screening for an antibody having equivalent function to the antibody of the present invention. That is, with techniques such as hybridization and gene amplification procedures (for example PCR), etc., that use the polynucleotide that encodes the antibody of the present invention or a part thereof as a probe, DNA encoding an antibody that hybridizes under stringent conditions with that polynucleotide and has activity equivalent to the antibody of the present invention can be obtained. Such DNA is included in the polynucleotide of the present invention. Hybridization techniques are well known to persons skilled in the art (Sambrook, J et al., Molecular Cloning 2nd ed., 9.47-9.58, Cold Spring Harbor Lab. Press, 1989).

Low stringency conditions, for example, can be noted as the conditions for hybridization. Low stringency conditions are, for example, 42°C, 0.1 × SSC, and 0.1% SDS in the post hybridization rinse, and preferably 50°C, 0.1 × SSC, and 0.1% SDS. High stringency conditions can be noted as more preferred hybridization conditions. High stringency conditions, for example, are 65°C, 5 × SSC, and 0.1% SDS. Under these conditions, as the temperature increases it can be expected that a polynucleotide having a higher degree of homology will be efficiently obtained. However, a plurality of factors affecting the stringency of hybridization such as temperature, salt concentration and the like can be noted, and a person skilled in the art can realize the same level of stringency by properly selecting these factors.

An antibody that is functionally equivalent to the antibody of the present invention and encoded by the polynucleotide obtained by these hybridization and gene amplification techniques will normally have a high degree of homology in its amino acid sequence. The antibody of the present invention includes an antibody that is functionally equivalent to the antibody of the present invention and has a high level of homology with the amino acid sequence of that antibody. The term "high degree of homology" normally indicates identity of at least 50% or more, preferably 75% or more, more preferably 85% or more, and even more preferably 95% or more at the amino acid level. The algorithm described in the following reference document can be followed to determine the homology of a polypeptide: Wilbur, W.J. and Lipman, D.J. Proc. Natl. Acad. Sci. USA (1983) 80, 726-730.

### Pharmaceutical composition

From a different standpoint, the present invention provides a pharmaceutical composition comprising an antibody capable of binding to the PRG-3 protein as an active ingredient. Moreover, the present invention relates to a cell growth inhibitor, especially an anticancer agent, comprising an antibody capable of binding to the PRG-3 protein as an active ingredient. Preferably, the cell growth inhibitor and the anticancer agent of the present invention are administered to a subject with cancer or a subject likely to get cancer. The cancer that the anticancer agent of the present invention targets is not particularly limited herein, but HCC, lung cancer, colon cancer, and glioblastoma are preferred, and HCC is particularly preferred. The anticancer agent of the present invention can be used to treat both primary lesions and metastatic lesions. Therefore, the anticancer agent of the present invention can be used for both primary cancer and metastatic cancer.

The present invention provides a cell growth inhibitor comprising the antibody capable of binding to the PRG-3 protein as an active ingredient. In other words, provided is a method for inhibiting cell growth comprising the step of administering to a subject the antibody capable of binding to the PRG-3 protein, or the use of the antibody capable of binding to the PRG-3 protein in the manufacture of a cell growth inhibitor.

In addition, the present invention provides an anticancer agent comprising the antibody capable of binding to the PRG-3 protein as an active ingredient. In other words, provided is a method for the prevention or treatment of cancer comprising the step of administering to a subject the antibody capable of binding to the PRG-3 protein, or the use of the antibody capable of binding to the PRG-3 protein in the manufacture of the anticancer agent.

As used herein the expression "comprise an antibody capable of binding to the PRG-3 protein as an active ingredient" means that the composition comprises an anti-PRG-3 antibody as a primary active ingredient, and the content ratio of the anti-PRG-3 antibody is not particularly limited.

The antibody contained in the pharmaceutical composition (for example, a cell growth inhibitor or anticancer agent, also hereinafter) of the present invention is not particularly limited provided it is capable of binding to the PRG-3 protein, and any of the antibodies described herein may be included.

The pharmaceutical composition of the present invention can be administered to a patient either orally or parenterally. Parenteral administration is preferred. More specifically, injection administration, transnasal administration, transpulmonary administration, transdermal administration and the like can be noted as methods of administration. The pharmaceutical composition of the present invention can be administered systemically or locally by intravenous injection, intramuscular injection, intraperitoneal administration, hypodermic injection and the like as examples of injection administration. Moreover, the administration method can be properly selected based on the patient's age and condition. For example, the amount of a single dose can be selected from within a range of 0.0001 mg to 1000 mg per kilogram of body weight. Alternatively, for example, the dose can be selected from within the range of 0.001 to 100000 mg/body per patient. However, the pharmaceutical composition of the present invention is by no means limited to these doses.

The pharmaceutical composition of the present invention can be formulated in accordance with conventional methods (e.g., Remington's Pharmaceutical Science, latest edition, Mark Publishing Company, Easton, U.S.A.) and can also contain both pharmaceutically acceptable excipients and additives. Examples include a surfactant, excipient, coloring, flavoring agent, preservative, stabilizer, buffer, suspending agent, tonicity agent, binder, disintegrating agent, lubricant, fluidity promoter, and flavor improving agent, etc. In addition, the excipients and additives are not limited, and other conventionally used carriers can be used. More specifically, examples of such carriers include light anhydrous silicic acid, lactose, crystalline cellulose, mannitol, starch, carmellose calcium, carmellose sodium, hydroxypropyl cellulose, hydroxypropyl methylcellulose, polyvinyl acetal diethyl amino acetate, polyvinyl pyrrolidone, gelatin, medium chain fatty acid triglycerides, polyoxyethylene hydrogenated castor oil 60, sucrose, carboxymethyl cellulose, cornstarch, inorganic salts, etc.

Moreover, the present invention provides a method for causing damage to and inhibiting cell growth of cells expressing PRG-3 by contacting the cells with an antibody capable of binding to the PRG-3 protein.

More specifically, the present invention includes the following modes:
- A method for damaging a cell that expresses PRG-3 comprising contacting the cell with an antibody capable of binding to the PRG-3 protein;
- A method for inhibiting a cell that expresses PRG-3 comprising contacting the cell with an antibody capable of binding to the PRG-3 protein;
- The aforementioned methods wherein the antibody capable of binding to the PRG-3 protein is an antibody having cytotoxic activity;
- The aforementioned methods wherein the antibody capable of binding to the PRG-3 protein is an antibody described in any of (1) to (34) below:
   (1) An antibody comprising an H-chain having the amino acid sequence represented by SEQ ID NO: 2 as CDR1, the amino acid sequence represented by SEQ ID NO: 4 as CDR2, and the amino acid sequence represented by SEQ ID NO: 6 as CDR3;
   (2) An antibody comprising the H-chain according to (1) having the amino acid sequence represented by SEQ ID NO: 8 as a CH;
   (3) An antibody comprising the H-chain according to (1) having the amino acid sequence represented by SEQ ID NO: 10 as a CH;
   (4) An antibody comprising an L-chain having the amino acid sequence represented by SEQ ID NO: 12 as CDR1, the amino acid sequence represented by SEQ ID NO: 14 as CDR2, and the amino acid sequence represented by SEQ ID NO: 16 as CDR3;
   (5) An antibody comprising the L-chain according to (4) having the amino acid sequence represented by SEQ ID NO: 18 as a CL;
   (6) An antibody comprising the L-chain according to (4) having the amino acid sequence represented by SEQ ID NO: 20 as a CL;
   (7) An antibody comprising the H-chain according to (1) and the L-chain according to (4);
   (8) An antibody comprising the H-chain according to (2) and the L-chain according to (5);
   (9) An antibody comprising the H-chain according to (3) and the L-chain according to (6);
   (10) An antibody wherein one or more amino acids is substituted, deleted from, added to, and/or inserted into the antibody according to any of (1) to (9), and that has activity equivalent to the antibody according to any of (1) to (9);
   (11) An antibody capable of binding to the same epitope as the epitope on the PRG-3 protein to which the antibody according to any of (1) to (10) is capable of binding;
   (12) An antibody comprising an H-chain having the amino acid sequence represented by SEQ ID NO: 22 as CDR1, the amino acid sequence represented by SEQ ID NO: 24 as CDR2, and the amino acid sequence represented by SEQ ID NO: 26 as CDR3;
   (13) An antibody comprising the H-chain according to (12) having the amino acid sequence represented by SEQ ID NO: 34 as a CH;
   (14) An antibody comprising the H-chain according to (12) having the amino acid sequence represented by SEQ ID NO: 10 as a CH;
   (15) An antibody comprising an L-chain having the amino acid sequence represented by SEQ ID NO: 28 as CDR1, the amino acid sequence represented by SEQ ID NO: 30 as CDR2, and the amino acid sequence represented by SEQ ID NO: 32 as CDR3;
   (16) An antibody comprising the L-chain according to (15) having the amino acid sequence represented by SEQ ID NO: 18 as a CL;
   (17) An antibody comprising the L-chain according to (15) having the amino acid sequence represented by SEQ ID NO: 20 as a CL;
   (18) An antibody comprising the H-chain according to (12) and the L-chain according to (15);
   (19) An antibody comprising the H-chain according to (13) and the L-chain according to (16);
   (20) An antibody comprising the H-chain according to (14) and the L-chain according to (17);
   (21) An antibody wherein one or more amino acids is substituted, deleted from, added to, and/or inserted into the antibody according to any of (12) to (20), and that has activity equivalent to the antibody according to any of (12) to (20);
   (22) An antibody capable of binding to the same epitope as the epitope on the PRG-3 protein to which the antibody according to any of (12) to (21) is capable of binding;
   (23) An antibody comprising an H-chain having the amino acid sequence represented by SEQ ID NO: 36 as CDR1, the amino acid sequence represented by SEQ ID NO: 38 as CDR2, and the amino acid sequence represented by SEQ ID NO: 40 as CDR3;
   (24) An antibody comprising the H-chain according to (23) having the amino acid sequence represented by SEQ ID NO: 34 as a CH;
   (25) An antibody comprising the H-chain according to (23) having the amino acid sequence represented by SEQ ID NO: 10 as a CH;
   (26) An antibody comprising an L-chain having the amino acid sequence represented by SEQ ID NO: 42 as CDR1, the amino acid sequence represented by SEQ ID NO: 44 as CDR2, and the amino acid sequence represented by SEQ ID NO: 46 as CDR3
   (27) An antibody comprising the L-chain according to (26) having the amino acid sequence represented by SEQ ID NO: 18 as a CL;
   (28) An antibody comprising the L-chain according to (26) having the amino acid sequence represented by SEQ ID NO: 20 as a CL;
   (29) An antibody comprising the H-chain according to (23) and the L-chain according to (26);
   (30) An antibody comprising the H-chain according to (24) and the L-chain according to (27);
   (31) An antibody comprising the H-chain according to (25) and the L-chain according to (28);
   (32) An antibody wherein one or more amino acids is substituted, deleted from, added to, and/or inserted into the antibody according to any of (23) to (31), and that has activity equivalent to the antibody according to any of (23) to (31);
   (33) An antibody capable of binding to the same epitope as the epitope on the PRG-3 protein to which the antibody according to any of (23) to (32) is capable of binding; and
   (34) A low molecular antibody of the antibody according to any of (1) to (33); and
- The aforementioned methods wherein the cell expressing the PRG-3 protein is a cancer cell.

As noted above, the antibody used in the methods of the present invention is that antibody capable of binding to the PRG-3 protein and is contained in the cell growth inhibitor of the present invention. The cells to which the anti-PRG-3 antibody binds are not particularly limited herein provided they are cells expressing PRG-3. The preferred cells expressing PRG-3 in the present invention are cancer cells. More preferred are HCC cells, lung cancer cells, colon cancer cells, and glioblastoma cells. The methods of the present invention can be used to treat both primary lesions and metastatic lesions of these cancers. Even more preferred cancer cells are primary and metastatic HCC cells.

As used herein, "contact" is carried out, for example, by adding the antibody to the culture medium of cells expressing PRG-3 cultured *in vitro.* In this case, a solution of the antibody, a solid form obtained by lyophilization, and the like can be suitably used as the form of the added antibody. When the antibody is added as an aqueous solution, it can be a pure aqueous solution that contains only the antibody. Alternatively, it can be a solution containing the aforementioned surfactant, excipient, coloring, flavoring agent, preservative, stabilizer, buffer, suspending agent, tonicity agent, binder, disintegrating agent, lubricant, fluidity promoter, and flavor improving agent, etc. The antibody concentration to be added is not particularly limited herein, but a range of preferably 1 pg/mL to 1 g/mL, more preferably 1 ng/mL to 1 mg/mL, and even more preferably 1 µg/mL to 1 mg/mL can be suitably used as the final concentration in the culture solution.

In another embodiment of the present invention, "contact" is carried out by administering the antibody to a non-human animal wherein cells expressing PRG-3 have been transplanted or to an animal that endogenously has cancer cells expressing PRG-3. Administration can be carried out either orally or parenterally. Parenteral administration is particularly preferred as the method of administration. More specifically, injection administration, transnasal administration, transpulmonary administration, transdermal administration and the like can be noted as these methods of administration. The pharmaceutical composition comprising the cell growth inhibitor and anticancer agent of the present invention can be administered systemically or locally by intravenous injection, intramuscular injection, intraperitoneal administration, hypodermic injection and the like as examples of injection administration. Moreover, the administration method can be properly selected based on the patient's age and condition. When the antibody is administered as an aqueous solution, it can be a pure aqueous solution that contains only the antibody. Alternatively, it can be a solution containing the aforementioned surfactant, excipient, coloring, flavoring agent, preservative, stabilizer, buffer, suspending agent, tonicity agent, binder, disintegrating agent, lubricant, fluidity promoter, and flavor improving agent, etc. For example, the amount of a single dose can be selected from within a range of 0.0001 mg to 1000 mg per kilogram of body weight. Alternatively, for example, the dose can be selected from within the range of 0.001 to 100000 mg/body per patient. However, the dosage of the antibody of the present invention is by no means limited thereto.

The following methods are suitably used as a method for evaluating or measuring cell damage caused to cells expressing PRG-3 by the contact with the anti-PRG-3 antibody. Methods that measure the aforementioned antibody-dependent cell-mediated cytotoxicity (ADCC) activity, complement-dependent cytotoxicity (CDC) and the like can be noted as methods for evaluating or measuring cytotoxic activity in vitro. Whether or not an anti-PRG-3 antibody has ADCC activity or CDC activity can be measured by a publicly known method (For example, Current Protocols in Immunology, Chapter 7. Immunologic studies in humans, Editor, John E. Coligan, et al., John Wiley & Sons, Inc. (1993), etc.) In measuring the activity, an antibody with the same isotype as the anti-PRG-3 antibody but not having cytotoxic activity can be used as a control antibody similarly to the anti-PRG-3 antibody, and activity can be evaluated by stronger cytotoxic activity being demonstrated by the anti-PRG-3 antibody than by the control antibody.

The isotype of an antibody is stipulated by the sequence of the H-chain constant region of that antibody's amino acid sequence. In the body an antibody's isotype is ultimately determined by a class switch due to gene recombination on the chromosome that occurs during the maturation process of antibody-producing B cells. An isotype difference reflects a difference in the antibody's physiological and pathological function. More specifically, it is known that the potency of cytotoxic activity, for example is affected by both the amount of antigen expression and the antibody isotype. Therefore, when measuring the cytotoxic activity described above, it is preferable to use as a control an antibody having the same isotype as the test antibody.

For the evaluation or measurement of cytotoxic activity in the body, for example, after cancer cells expressing PRG-3 are transplanted intracutaneously or subcutaneously into a non-human animal, the test antibody is administered intravenously or intraperitoneally every day or at intervals of a few days starting on the day of transplantation or on the following day. By measuring the time-course changes in tumor size it is possible to evaluate the cytotoxic activity of the test antibody. Just as in the case of an in vitro evaluation, a control antibody of the same isotype is administered, and the presence of cytotoxic activity can be determined by the fact that the size of the tumor in the group administered the anti-PRG-3 antibody is significantly smaller than the size of the tumor in the group administered the control antibody. When a mouse serves as the non-human test animal, a nude (nu/nu) mouse wherein the thymus is genetically deficient and T lymphocyte function is absent can be most suitably used. By using such a mouse it is possible to exclude the involvement of the test animal's T lymphocytes in the evaluation and measurement of cytotoxic activity due to the administered antibody.

### Screening method

The inventors found that PRG-3 expression is specifically enhanced in cancer cells. Therefore, treatment of the cancer can be realized by suppressing the level of PRG-3 expression in cells that express PRG-3. In other words, based on the revelations of the present invention, it is believed that a compound that suppresses the level of PRG-3 expression in cancer cells will be useful as a candidate compound to be used in a therapeutic agent for cancer. Based on this knowledge, the present invention provides a screening method for a candidate compound to be used in a therapeutic agent for cancer comprising the following steps of:
(1) contacting a test compound with cells expressing PRG-3;
(2) measuring the level of PRG-3 expression in the cells expressing PRG-3; and
(3) selecting a compound wherein the level of PRG-3 expression is decreased in comparison with the control as a candidate compound to be used in a therapeutic agent for cancer.

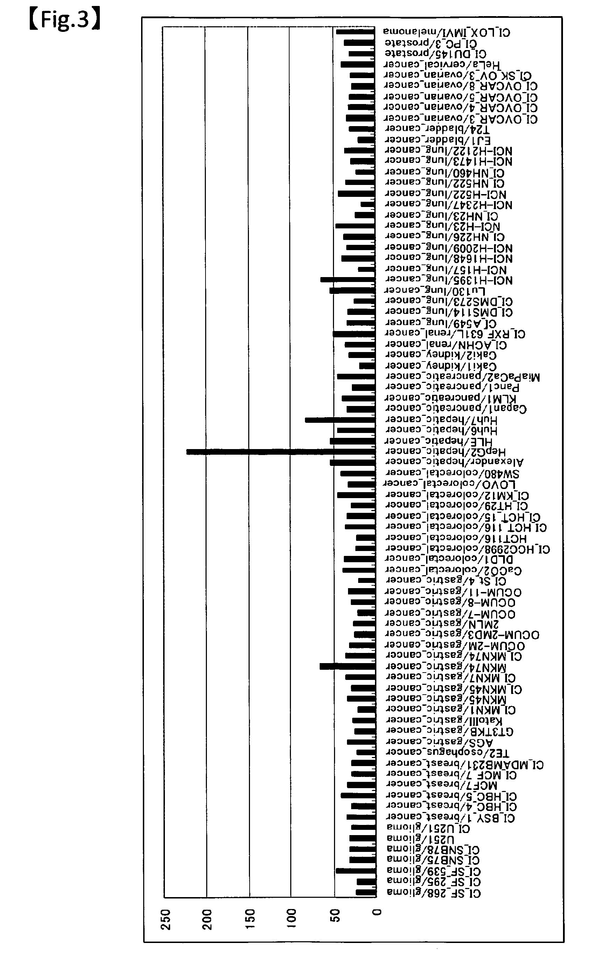

In the present invention cancer cells that express PRG-3 can be used, for example, as the cells expressing PRG-3. More specifically, any of the cancer cell lines that exhibit enhanced PRG-3 expression in Fig. 3, for example, can be used in the screening method. The following cancer cell lines can be noted as cells that can be used in the screening method of the present invention. All cell lines listed below can be obtained from a cell bank. The culturing conditions for these cell lines have already been established. The cells can be cultured using medium and culturing conditions recommended by these cell banks.

| | |
|---|---|
| HCC cell lines | :Huh6 |
| | :HuH-7 |
| | :HepG2 |
| Lung cancer cell lines | :Lu130 |
| | :H1395 |

These cell lines are cultured in the presence of the test compound and the level of PRG-3 expression in the cell lines is measured. The level of PRG-3 expression can be evaluated by measuring the amount of mRNA in the cells, and the amount of the PRG-3 protein within and on the surfaces of the cells. Any methods described herein can be used as the method for measuring PRG-3 mRNA and the PRG-3 protein.

In the present invention a test compound causing a level of PRG-3 expression lower than the control is selected as a candidate compound. The same cell line cultured in the absence of the test substance, for example, can be used as a control in the screening method of the present invention. Alternatively, a cell line cultured in the presence of a compound that has been clearly shown beforehand to affect the level of expression of PRG-3 can serve as a control. A compound whose effect is greater than a known compound can be selected by using such a subject.

The candidate compound selected by the screening method of the present invention is useful as a candidate compound to be used as a therapeutic drug in an anticancer agent wherein the mechanism of action is the inhibition of the action of PRG-3. The therapeutic efficacy against cancer of an antibody to PRG-3 has been verified in the present invention. Therefore, an effect similar to that of the antibody can also be expected of a compound selected by the screening method of the present invention. The effect of the candidate compound selected by the screening procedure of the present invention on other cancer cell lines or primary cultured cells, as well as effects such as toxicity on normal cells can be evaluated as needed. A useful compound can be selected as a therapeutic drug for cancer through these evaluations. A series of methods for evaluating therapeutic drugs for cancer has already been established.

In the screening method of the present invention, any natural or artificially synthesized compound can be used as a test substance. For example, protein libraries and antibody libraries are preferred as test compound libraries in the present invention. Alternatively, a phage library wherein proteins and antibodies are presented can be used as test compounds. In addition, a library of artificially synthesized compounds from a combinatorial library, etc., can be used as test compounds.

The entire content of all patents and reference documents explicitly referred to in the present description are hereby incorporated by reference. Moreover, the entire content presented in the description and drawings of Japanese Patent Application No. 2007-000804, which serves as the basis of the priority claim of the present application, are hereby incorporated by reference.

The present invention is explained in greater detail below through examples, but the present invention is by no means limited to these examples.

### [Example 1]

### Analysis of human PRG-3 gene expression in various cancers

### 1-1. Analysis of human PRG-3 gene expression using a GeneChip

To specifically search for genes with enhanced expression in liver cancer tissue, a comprehensive analysis of gene expression in normal tissue, cancer tissue, and cancer cell lines was performed using a GeneChip U-133A (Affymetrix).

Initially total RNA was prepared from the normal tissue, cancer tissue, and cancer cell lines shown in Tables 1, 2, and 3 in a conventional manner using ISOGEN (Nippon Gene). Then gene expression analysis was performed using 10 µg of each total RNA with a GeneChip U-133A (Affymetrix) in accordance with the Expression Analysis Technical Manual (Affymetrix). The mean value of the expression score of all genes was set at 100, and a search for the genes with enhanced expression in cancer tissue or cancer cells was carried out.

**[Table 1]**

| Tissues analyzed for the expression of PRG-3 gene | |
|---|---|
| Sample | Origin |
| Whole_Brain | Clontech 64020-1 |
| Brain_Amygdala | Clontech 6574-1 |
| Brain_corpus_callosum | Clontech 6577-1 |
| Brain_caudate_nuclers | Clontech 6575-1 |
| Brain_thalamus | Clontech 6582-1 |
| Brain_hippo campus | Clontech 6578-1 |
| Brain_cerebellum | Clontech 64035-1 |
| Spinal_Cord | Clontech 6593-1 |
| Retina | Clonetch636579 |
| Pituitary_Gland | Clontech 6584-1 |
| Thymus | Ambion 7964 |
| Thyroid | Stratagene 735040 |
| Salivary_Gland | Clontech 64026-1 |
| Lung | Clinical Sample |
| Trachea | Clontech 64091-1 |
| Skin | Stratagene 735031 |
| Breast | Stratagene 735044 |
| Skeltal_muscle | Ambion 7982 |
| Heart | Ambion7966 |
| Atrium | Stratagene835007 |
| Kidney | Ambion 7976 |
| Kidney1 N/normal | Clinical Sample |
| Kidney3N/normal | Clinical Sample |
| Adrenal_gland | Clontech64096-1 |
| Liver | Clinical Sample |
| Pancreas | Ambion 7954 |
| Spleen | Ambion 7970 |
| Stomach | Clinical Sample |
| Small_Intestine | Ambion 7984 |
| Colon | Ambion 7986 |
| Colon1 N/normal | Clinical Sample |
| Colon3N/normal | Clinical Sample |
| Bladder | Ambion 7990 |
| Bone_marrow | Clontech64106-1 |
| Peripheral_blood | Peripheral_blood |
| Testis | Clontech64027-1 |
| Prostate | Ambion 7988 |
| Ovary | Ambion 7974 |
| Uterus | Stratagene 735042 |
| Placenta | Ambion 7950 |
| Liver_Cirrhosis | Clinical Sample |
| Fetal_Brain | Clontech64094-1 |
| Fetal_Liver | CHEMICON356 |

**[Table 2]**

| Cancer tissues alalyzed for the expression of PRG-3 gene | |
|---|---|
| Sample | Origin |
| Glioblastoma | Clinical Sample |
| lung_ade no carcino ma/pool | Clinical Sample |
| hepatic_cancer/moderately | Clinical Sample |
| hepatic_cancer/poorly | Clinical Sample |
| stomach cancer | Clinical Sample |
| Lung1 Ca/small cell lung carcinoma | Clinical Sample |
| Lung2Ca/small cell lung carcinoma | Clinical Sample |
| Lung3Ca/small cell lung carcinoma | Clinical Sample |
| Lung4Ca/small cell lung carcinoma | Clinical Sample |
| Lung5Ca/small cell lung carcinoma | Clinical Sample |
| Lung5Ca/small cell lung carcinoma | Clinical Sample |
| Lung7Ca/small cell lung carcinoma | Clinical Sample |
| Lung8Ca/small cell lung carcinoma | Clinical Sample |
| Luns9Ca/small cell lung carcinoma | Clinical Sample |
| Lung10Ca/small cell lung carcinoma | Clinical Sample |
| Lung11Ca/squamous cell carcinoma | Clinical Sample |
| Lung12Ca/squamous cell carcinoma | Clinical Sample |
| Lung13Ca/squamous cell carcinoma | Clinical Sample |
| Lung14Ca/squamous cell carcinoma | Clinical Sample |
| Lung15Ca/squamous cell carcinoma | Clinical Sample |
| Lung16Ca/ade no carcinoma | Clinical Sample |
| Lung17Ca/ade no carcinoma | Clinical Sample |
| Lung18Ca/ade no carcinoma | Clinical Sample |
| Lung19Ca/ade no carcinoma | Clinical Sample |
| Lung20Ca/ade no carcinoma | Clinical Sample |
| Kidney2Ca/cancer | Clinical Sample |
| Kidney4Ca/cancer | Clinical Sample |
| CR34/primary | Clinical Sample |
| CR61/primary | Clinical Sample |
| CR67/primary | Clinical Sample |
| Colon_Ca/pool | Clinical Sample (mixture of CR34, CR61, and Cr67) |
| Colon2Ca/cancer | Clinical Sample |
| Colon4Ca/cancer | Clinical Sample |
| Colon5Ca/metastasis | Clinical Sample |
| Colon6Ca/metastasis | Clinical Sample |
| Coolon7Ca/metastasis | Clinical Sample |
| Colon8Ca/metastasis | Clinical Sample |
| Colon9Ca/metastasis | Clinical Sample |
| Colon10Ca/metastasis | Clinical Sample |
| Colon11Ca/metastasis | Clinical Sample |
| Colon12Ca/primary | Clinical Sample |
| Colon13Ca/primary | Clinical Sample |
| Pancreas1Ca/cancer | Clinical Sample |
| Pancreas2Ca/cancer | Clinical Sample |
| Pancreas30a/cancer | Clinical Sample |
| Pancreas4Ca/cancer | Clinical Sample |

**[Table 3-1]**

| Cancer cell lines analyzed for the expression of PRG-3 gene | |
|---|---|
| Sample | Origin |
| CI_SF_268/glioma | cell line derived from human glioma |
| CI_SF_295/glioma | cell line derived from human glioma |
| CI_SF_539/glioma | cell line derived from human glioma |
| CI_SNB75/glioma | cell line derived from human glioma |
| CI_SNB78/glioma | cell line derived from human glioma |
| U251/glioma | cell line derived from human glioma |
| CI_U251/glioma | cell line derived from human glioma |
| CI_BSY_1/breast_cancer | cell line derived from human breast cancer |
| CI_HBC_4/breast_cancer | cell line derived from human breast cancer |
| CI_HBC_5/breast_cancer | cell line derived from human breast cancer |
| MCF7/breast_cancer | cell line derived from human breast cancer |
| CI_MCF_7/breast_cancer | cell line derived from human breast cancer |
| CI_MDAMB231/breast_cancer | cell line derived from human breast cancer |
| TE2/esophagus_cancer | cell line derived from human esophagus cancer |
| AGS/gastric_cancer | cell line derived from human gastric cancer |
| GT3TKB/gastric_cancer | cell line derived from human gastric cancer |
| KatoIII/gastric_cancer | cell line derived from human gastric cancer |
| CI_MKN1/gastric_cancer | cell line derived from human gastric cancer |
| MKN45/gastric_cancer | cell line derived from human gastric cancer |
| CI_MKN45/gastric_cancer | cell line derived from human gastric cancer |
| CI_MKN7/gastric Cancer | cell line derived from human gastric cancer |
| MKN74/gastric_cancer | cell line derived from human gastric cancer |
| CI_MKN74/gastric_cancer | cell line derived from human gastric cancer |
| OCUM-2M/gastric_cancer | cell line derived from human gastric cancer |
| OCUM-2MD3/gastric_cancer | cell line derived from human gastric cancer |
| 2MLN/gastric_cancer | cell line of human gastric cancer |
| OCUM-7/gastric_cancer | cell line derived from human gastric cancer |
| OCUM-8/gastric_cancer | cell line derived from human gastric cancer |
| OCUM-11/gastric_cancer | cell line derived from human gastric cancer |
| CI_St_4/gastric_cancer | cell line derived from human gastric cancer |
| CaCO2/coloreectal_cancer | cell line derived from human colorectal cancer |
| DLD1/colorectal_cancer | cell line derived from human colorectal cancer |
| CI_HCC2998/colorectal_cancer | cell line derived from human colorectal cancer |
| HCT1116/colorectal_cancer | cell line derived from human colorectal cancer |
| CI_HCT_116/colorectal_cancer | cell line derived from human colorectal cancer |
| CI_HCT_15/colorectal_cancer | cell line derived from human colorectal cancer |
| CI_HT29/colorectal_cancer | cell line derived from human colorectal cancer |
| CI_KM12/colorectal_cancer | cell line derived from human colorectal cancer |
| LOVO/colorectal_cancer | cell line derived from human colorectal cancer |
| SW48O/colorectal_cancer | cell line derived from human colorectal cancer |

**[Table 3-2]**

| Cancer cell lines analyzed for the expression of PRG-3 gene | |
|---|---|
| Sample | Origin |
| Alexander/hepatic_cancer | cell line derived from human hepatic cancer |
| HepG2/hepatic_cancer | cell line of human hepatic cancer |
| HLE/hepatic_cancer | cell line derived from human hepatic cancer |
| Huh6/hepatic_cancer | cell line derived from human hepatic cancer |
| Huh7/hepatic_cancer | cell line derived from human hepatic cancer |
| Capan1/pancreatic_cancer | cell line derived from human pancreatic cancer |
| KLM1/pancreatic_cancer | cell line of human pancreatic cancer |
| Panc1/pancreatic_cancer | cell line of human pancreatic cancer |
| MiaPaCa2/pancreatic_cancer | cell line derived from human pancreatic cancer |
| Caki1/kidney_cancer | cell line derived from human kidney cancer |
| Cak12/kidney_cancer | cell line derived from human kidney cancer |
| CI_ACHN/renal_cancer | cell line derived from human renal cancer |
| CI_RXF_631L/renal_cancer | cell line derived from human renal cancer |
| CI_A549/lung_cancer | cell line derived from human lung cancer |
| CI_DMS114/lung_cancer | cell line derived from human lung cancer |
| CI_DMS273/lun_cancer | cell line derived from human lung cancer |
| Lu1_30/lung_cancer | cell line derived from human lung cancer |
| NCI-H1395/lung_cancer | cell line derived from human lung cancer |
| NCI-H157/lung_cancer | cell line derived from human lung cancer |
| NCI-H1648/lung cancer | cell line derived from human lung cancer |
| NCI-H2009/lung_cancer | cell line derived from human lung cancer |
| CI_NH226/lung_cancer | cell line derived from human lung cancer |
| NCI-H23/lung_cancer | cell line derived from human lung cancer |
| CI_NH23/lung_cancer | cell line derived from human lung cancer |
| NCI-H2347/lung_cancer | cell line derived from human lung cancer |
| NCI-H522/lung_cancer | cell line derived from human lung cancer |
| CI_NH522/lung_cancer | cell line derived from human lung cancer |
| CI_NH460/lung_cancer | cell line derived from human lung cancer |
| NCI-H1473/lung_cancer | cell line derived from human lung cancer |
| NCI-H2122/lung_cancer | cell line derived from human lung cancer |
| EJ1/bladder_cancer | cell line derived from human bladder cancer |
| T24/bladder_cancer | cell line derived from human bladder cancer |
| CI_OVCAR_3/ovarian_cancer | cell line derived from human ovarian cancer |
| CI_OVCAR_4/ovarian_cancer | cell line derived from human ovarian cancer |
| CI_OVCAR_5/ovarian_cancer | cell line derived from human ovarian cancer |
| CI_OVCAR_8/ovarian_cancer | cell line derived from human ovarian cancer |
| CI_SK_OV_3/ovarian_cancer | cell line derived from human ovarian cancer |
| HeLa/cervical_cancer | cell line of human cervical cancer |
| CI_DU145/prostate | cell line derived from human prostate cancer |
| CI_PC_3/prostate | cell line derived from human prostate cancer |
| CI_LOX_IMVI/melanoma | cell line derived from human melanoma |

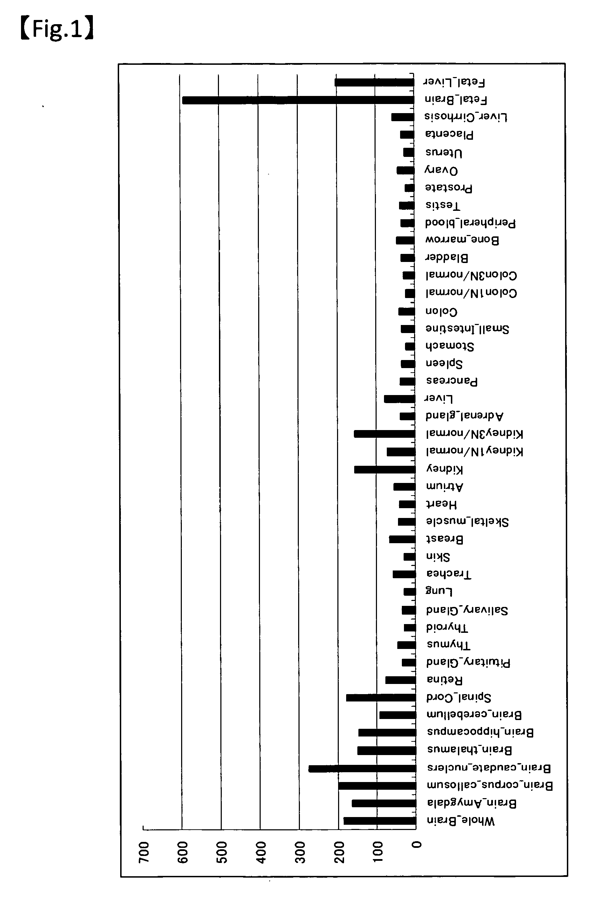

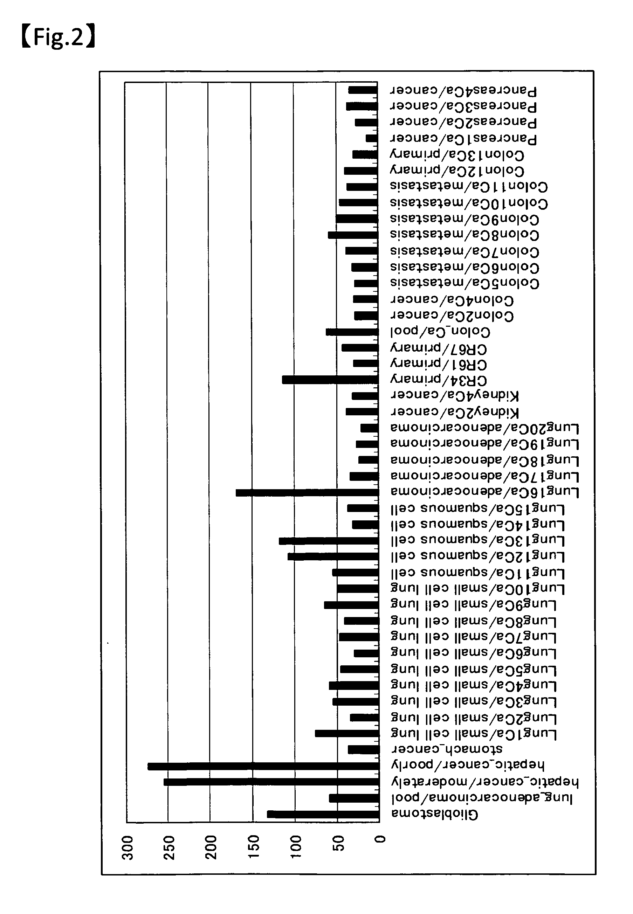

The results of that analysis clearly revealed that human PRG-3 mRNA (probe ID:219732_at HG-U133A) expression is enhanced in normal fetal brain tissue, and in liver cancer, lung cancer, colon cancer, and the liver cancer cell line (HepG2) among the clinical cancer tissues (Figures 1, 2, and 3).

### [Example 2]

### Preparation of anti-PRG-3 monoclonal antibody

### 2-1. Establishment of cell line expressing whole human PRG-3

Whole human PRG-3 cDNA (polynucleotide sequence SEQ ID NO: 59 and amino acid sequence SEQ ID NO: 60) was isolated by PCR using the sequence NCBI Accession No. AK000307: Homo sapiens cDNA FLJ20300 fis, clone HEP06465 as a basis, and it was cloned to a mammalian cell expression vector (pcDNA5/FRT/TO, Invitrogen). The vector pcDNA5/FRT/TO enables inducible expression of a transferred gene under the hybrid human CMV/Tet02 promoter, and it is a vector containing a neomycin resistance gene as a drug resistance marker. Additionally, using the FlpIn expression system (Invitrogen), which enables inducible expression only in the presence of tetracycline or doxycycline, the whole human PRG-3 cDNA gene was transferred into 293FlpIn T-Rex cells. Fugene6 (Roche) was used for transfer of the expression vector into the 293FlpIn T-Rex cells cultured with DMEM (high glucose)/10% FBS/100 µg/mL Zeocin^{™} (Invitrogen)/15 µg/mL blasticidin (Invitrogen). Gene transfer of the pcDNA5/FRT/PRG-3 vector together with the OG44 vector that expresses Flp recombinase was carried out following the instruction manual, and a line of PRG-3/293T-Rex cells carrying whole human PRG-3 was established by selection using 50 µg/mL hygromycin B (Invitrogen). Because a V5 tag was inserted at the C-terminus of the PRG-3 gene inserted into the expression vector, the selected cells were detected by an anti-V5 antigen (Invitrogen).

In addition, a vector inserted with the PRG-3 gene was constructed for DNA immunization. The expression vector pMCN enables inducible expression of a transferred gene under the mouse CMV promoter (Accession No. U68299), and it is a vector having a neomycin resistance gene incorporated as a drug resistance marker. The PRG-3 gene was cloned by a conventional method into pMCN to prepare the PRG-3 inducible expression vector pMCN-PRG-3.

### 2-2. Preparation of anti-PRG-3 monoclonal antibody

DNA immunization by gene transfer to mice was carried out by the GeneGun Particle method. The procedure was performed according to the Bio-Rad manual. The bullets for DNA immunization were prepared by mixing 1 mm gold particles (Bio-Rad) and pMCN-PRG-3 DNA, and coating the interior of a tube. Gene transfer was carried out by shooting the bullets coated with pMCN-PRG-3 DNA into the abdominal skin of a 5-week-old female MRL/lpr mice using a Helios Gene Gun (Bio-Rad) at a pressure of 200 to 300 psi. The gene transferred to the keratinocytes, Langerhans cells, and dermal dendritic cells in the mouse skin is thought to attract immunoreaction because these cells become antigen-presenting cells (APC) by expressing the PRG-3 protein (Peachman K.K., Rao M., and Alving C.R., Immunization with DNA through the skin, Methods 31, 232-242 (2003)). DNA immunization was carried out 2 to 3 times at 1 week intervals. As the final immunization, 5 × 10⁵ 293T-Rex cells expressing PRG-3 were diluted in PBS and injected via the tail vein. Measurement of the antibody titer was performed by FACS analysis using PRG-3/293T-Rex cells. A comparison was made of the reactivity of sera from the immunized mice to PRG-3 protein induced by doxycycline and expressed on cell membrane surfaces. Spleen cells were extracted from the mouse showing the highest response and subjected to cell fusion by the PEG method. The spleen cells were resected 3 days after the final immunization and mixed with P3-X63Ag8U1 mouse myeloma cells (P3U1, purchased from ATCC) at a 4:1 ratio. Cell fusion was carried out by gradually adding PEG1500 (Roche Diagnostics), and hybridomas were prepared. After the PEG1500 concentration was diluted by carefully adding RPMI-1640 medium (Gibco BRL), the PEG1500 was removed by a centrifugation procedure. Next, the hybridoma cells were suspended in RPMI-1640 medium containing 10% FBS, 1 × Hat media supplement (SIGMA), and 0.5 × BM-Condimed H1 Hybridoma cloning supplement (Roche Diagnostics) (hereinafter, HAT medium), and inoculated into a 96-well culture plate to a total volume of 200 µL/well. The cell density at the time of inoculation was diluted depending on the P3U1 cell count, and the hybridoma cells were cultured about 1 week in HAT medium in the 96-well plate at 37°C and 5% CO₂. Screening for hybridomas that secreted antibodies into the culture supernatant was performed by flow cytometry.

### 2-3. Evaluation of binding activity by flow cytometry

The antibody binding to the human PRG-3/293T-Rex cells was evaluated by flow cytometry using the obtained hybridomas. The cell line expressing human PRG-3 suspended in FACS buffer (2% FBS/PBS) was diluted to 1 × 10⁶ cells/mL using FACS buffer, and aliquoted at 50 µL/well into a Falcon 353910 round-bottom 96-well plate. Hybridoma culture supernatant diluted to a suitable concentration was added to the wells containing the cells and reacted for 60 min on ice. Next, the cells were rinsed once with FACS buffer. Goat F(ab')₂ fragment anti-mouse IgG (H+L)-FITC (Beckman Coulter) was added to the wells containing the cells as a secondary antibody, and reacted for 30 min on ice. The supernatant was removed by centrifugation after the reaction, and the cells suspended in 100 µL of FACS buffer were subjected to flow cytometry. A FACS Calibur (Becton Dickinson) was used for the flow cytometry. A gate for the viable cell population was established with a forward scatter-side scatter dot blot, an FL1 histogram was made of the cells contained in the population, and binding activity thereof was evaluated.

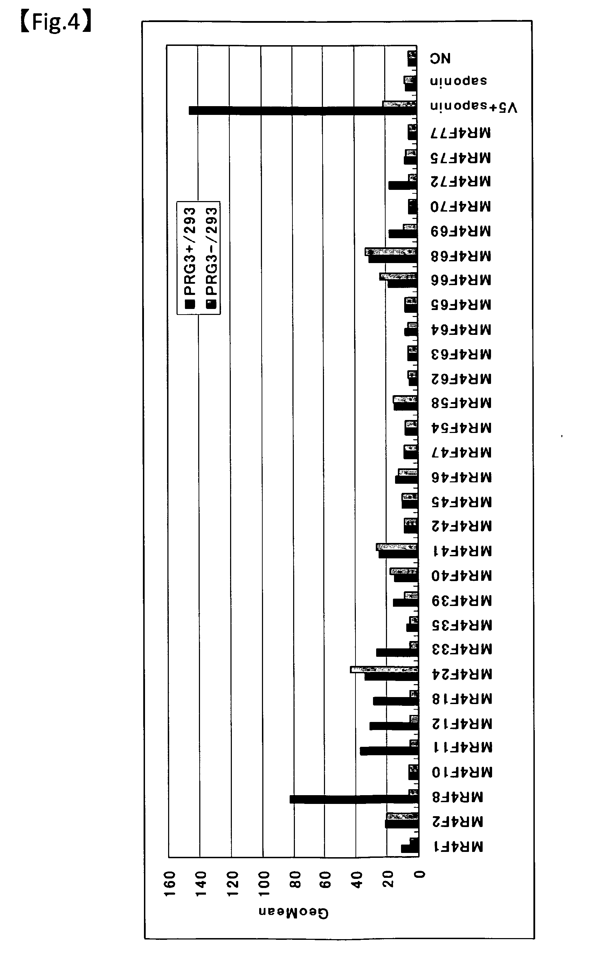

When the hybridoma supernatants were reacted with 293 cells wherein PRG-3 was inducibly expressed and 293 cells wherein PRG-3 was not inducibly expressed, hybridomas that specifically reacted with cells expressing PRG-3 was obtained. Figure 4 shows one example. PRG-3/293 cells could be detected by the V5 tag added to the C-terminus of PRG-3. Because the C-terminus region lies within the cell, the cells were treated with saponin so that the antibody could react intracellularly, and flow cytometry was performed in that state. The samples with V5 + saponin illustrates that the expression of PRG-3 was induced. Among the wells of hybridomas it was discovered that MR4F1, MR4F8, MR4F11, MR4F12, MR4F33, and MR4F72 contained hybridomas producing an antibody that reacts with PRG-3.

The hybridomas from these wells were made into monoclones by the limiting dilution method. Culturing of the hybridomas made into monoclones was scaled up, and the antibodies from the culture supernatant were purified using a protein G column in accordance with the manual. The isotype of each antibody was analyzed using an IsoStrip™ (Roche Diagnostics) mouse monoclonal antibody isotyping kit. As a result, antibodies 4F1 and 4F11 were IgG2b, and the other antibodies were all IgG2a.

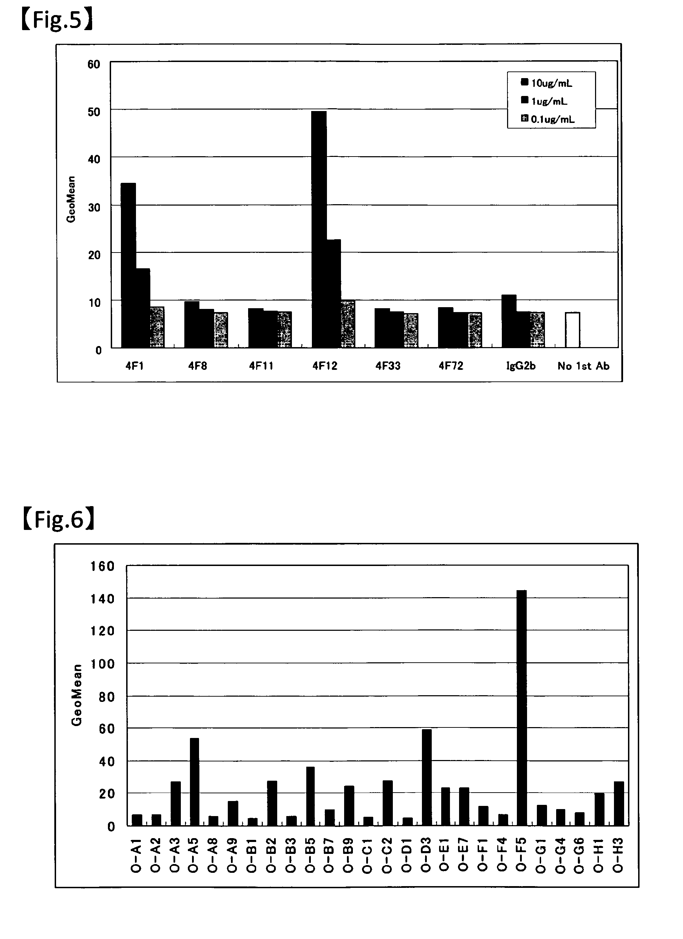

Protein quantitation of the purified antibodies was carried out, and the reactivity with HepG2 cells, wherein the expression of the human PRG-3 gene is enhanced, was analyzed by flow cytometry at concentrations of 10 µg/mL, 1 µg/mL, and 0.1 µg/mL respectively. The results revealed that only 4F1 and 4F12 react with HepG2 cells (Fig. 5).

### [Example 3]

### Measurement of complement-dependent cytotoxicity (CDC) activity and antibody-dependent cell-mediated cytotoxicity (ADCC) activity of anti-PRG-3 monoclonal antibodies

### 3-1. Measurement of CDC activity of the anti-PRG-3 monoclonal antibodies

The CDC activity was measured using the extent of uptake of 7-AAD by cells wherein cytotoxicity had occurred as an indicator.

A Ba/F3 cell transformant line was established by gene transfer of the pMCN-PRG-3 expression vector constructed in example 2 into Ba/F3 cells, which is a murine pro B cell line, by electroporation under conditions of 0.33 kV and 950 µF. The Ba/F3 cells are IL-3 dependent cells, and they were cultured in RPMI-1640/10% FBS/penicillin-streptomycin containing 1 ng/mL IL-3. The cells that underwent gene transfer were selected by RPMI-1640/10% FBS/penicillin-streptomycin containing IL-3 and 500 µg/mL Geneticin^{®} (Invitrogen). Cells expressing PRG-3 from the Geneticin^{®}-resistant lines were selected by flow cytometry. Because the C-terminus of the PRG-3 gene inserted into the pMCN-PRG-3 expression vector did not have a V5 tag, cells reacting with a mixture of monoclonal antibodies 4F1 and 4F12 established by immunization were selected as cells expressing PRG-3. Lines O-A5, O-D3, and O-F5 were established as Ba/F3 cell lines expressing PRG-3 (Fig. 6). Additionally, line O-F5 was cloned by the limiting dilution method and the OF5-G8 line with a high level of expression was also obtained.

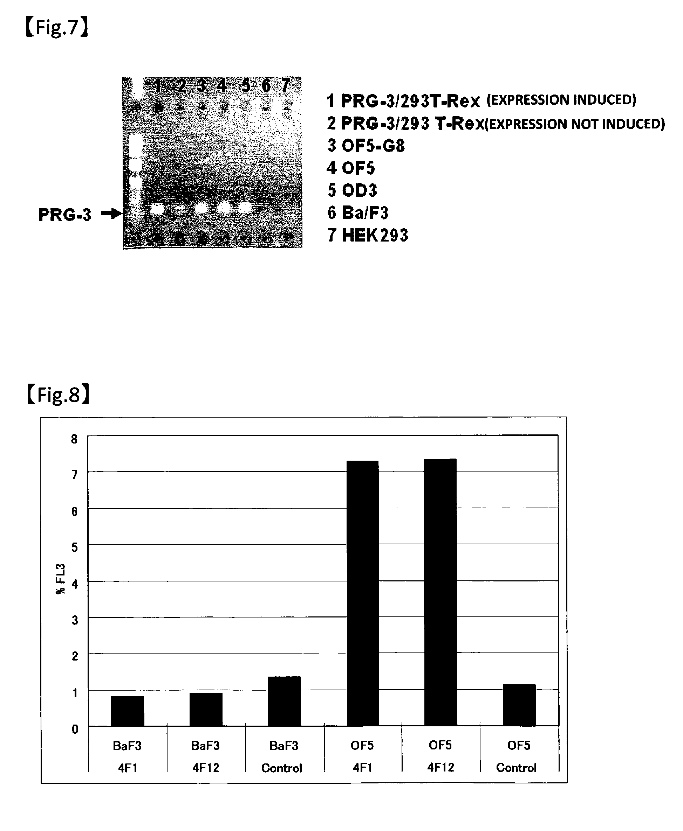

RT-PCR was used to confirm that the established cell line expresses PRG-3 (Fig. 7). Because it could be verified on the mRNA level that PRG-3 is expressed, while a PRG-3 band could not be identified in the parent Ba/F3 cells, it was concluded that the established cells express PRG-3.

Ba/F3 cells and O-F5 cells were reacted with the monoclonal antibodies at a concentration of 10 µg/mL at 4°C for 30 min. Next, baby rabbit complement (Cedarlane Laboratories) was added to a final concentration of 1% or 5%, and the reaction was continued for 90 min at 37°C. After 7-AAD (Beckman Coulter) was added to make a final concentration of 1 µg/mL, the reaction mixture was let stand for 10 min at room temperature. Then after the cells had been rinsed with FACS buffer, the ratio of cells showing a cytotoxic response was analyzed by FACS Calibur. The value of %FL3 shows the ratio of cells stained by 7-AAD wherein cytotoxicity had occurred, and complement-dependent cytotoxicity (CDC) specifically caused by the anti-PRG-3 antibody in O-F5 cells expressing PRG-3 was found (Fig. 8). On the other hand, no CDC activity was seen at the same concentration with the mouse IgG2a antibody controls.

### 3-2. Measurement of ADCC activity of the anti-PRG-3 monoclonal antibodies

The measurement of ADCC activity was performed by using OF5-G8, which are PRG-3 forced expression Ba/F3 cells. ADCC activity was measured using a predetermined method. More specifically, after the OF5-G8 cell line had been reacted with chromium-51 at 37°C for 1 hour, they were rinsed with RPMI-1640 medium (Invitrogen) with 10% FBS, adjusted to a concentration of 2 × 10⁵ cells/mL, and 50 µL aliquots were added to each well of a 96-well round-bottom plate. Next 50 µL of anti-PRG-3 monoclonal antibody (4F1, 4F8, 4F12) diluted in medium and a control (no antibody) were added to each well. The antibodies were diluted to final concentrations of 1 µg/mL, 0.1 µg/mL, 0.01 µg/mL, and 0.001 µg/mL. Next, 100 µL of an effector cell solution (1.9 × 10⁶ cells/mL) was added to each well. After the plate was let stand 6 hours at 37°C in a 5% CO₂ gas incubator, the specific chromium release rate was determined. CSH mouse (Charles River Japan) spleen cells cultured for 4 days in a medium containing 50 ng/mL of recombinant human interleukin-2 (Cat. No. 200-02, Peprotech) were used as the effector cells.

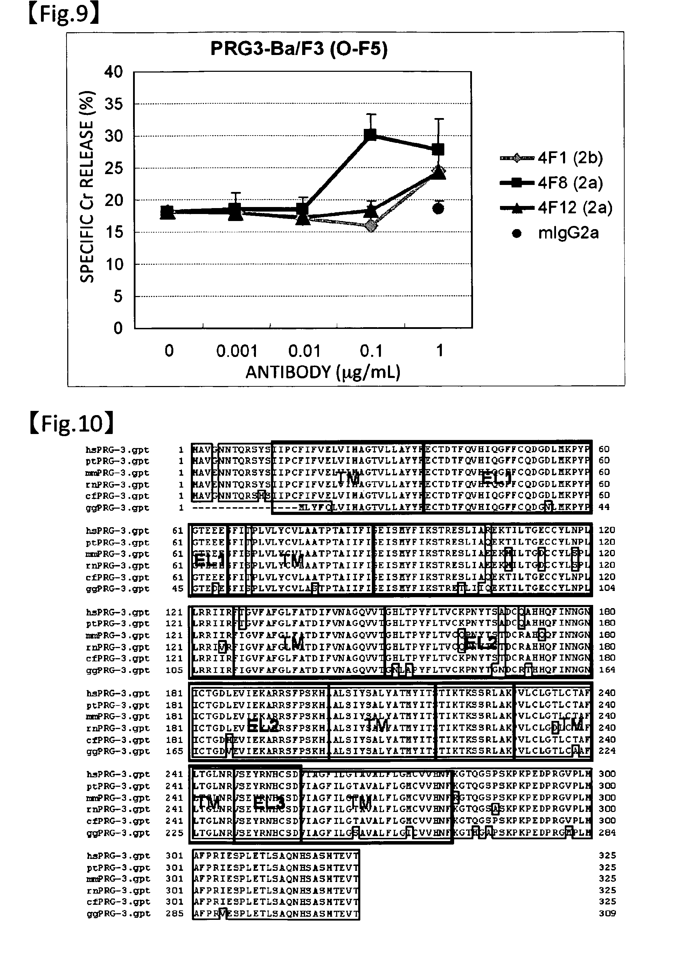

The results clearly revealed that all anti-PRG-3 monoclonal antibodies used induced ADCC in OF5-G8 cells (Fig. 9).

### [Example 4]

### Determination of variable region gene sequences of the 4F1, 4F8, and 4F12 anti-PRG-3 monoclonal antibodies

The antibody variable region gene sequences of the three hybridomas (4F1, 4F8, and 4F12) that showed ADCC activity and CDC activity were determined. The antibody genes were amplified by RT-PCR using total RNA extracted from the hybridomas producing the anti-PRG-3 antibodies (4F1, 4F8, and 4F12). The total RNA was extracted from 1 × 10⁷ hybridoma cells using RNeasy Plant Mini Kits (Qiagen). A RACE library was constructed using 1 µg of total RNA with a SMART RACE cDNA Amplification Kit (Clontech). By using MHC-IgG2a (SEQ ID NO: 61, a synthetic oligonucleotide complementary to the murine IgG2a constant region sequence), MHC-IgG2b (SEQ ID NO: 62, a synthetic oligonucleotide complementary to the murine IgG2b constant region sequence), and kappa (SEQ ID NO: 63, a synthetic oligonucleotide complementary to the murine κ-chain constant region base sequence), the 5' end gene fragment of the gene encoding the antibody produced by the hybridoma was amplified. The reverse transcription reaction was carried out at 42°C for 1.5 hours. Each 50 µL of PCR solution contained 5 µL of 10 × Advantage 2 PCR Buffer, 5 µL of 10 × Universal Primer A Mix, 0.2 mM dNTPs (dATP, dGTP, dCTP, dTTP), 1 µL of Advantage 2 Polymerase Mix (all from Clontech), 2.5 µL of reverse transcription reaction product, and 10 pmol of the synthetic oligonucleotide MHC-IgG2a, MHC-IgG2b, or kappa. The PCR reaction was carried out as follows: 5 repetitions of a cycle with a 30 sec reaction at a starting temperature of 94°C, 5 sec at 94°C, and 3 min at 72°C; next 5 repetitions of a cycle with 5 sec at 94°C, 10 sec at 70°C, and 3 min at 72°C; and then 25 repetitions of a cycle with 5 sec at 94°C, 10 sec at 68°C, and 3 min at 72°C. Finally, the reaction product was heated for 7 min at 72°C. Each PCR product was purified from agarose gel using a QIAquick Gel Extraction Kit (Qiagen). Then the PCR product was cloned to a pGEM-T Easy vector (Promega) and the nucleotide sequence was determined. For 4F1, SEQ ID NO: 47 shows the nucleotide sequence and SEQ ID NO: 48 shows the amino acid sequence of the H-chain variable region; and SEQ ID NO: 49 shows the nucleotide sequence and SEQ ID NO: 50 shows the amino acid sequence of the L-chain variable region. For 4F8, SEQ ID NO: 51 shows the nucleotide sequence and SEQ ID NO: 52 shows the amino acid sequence of the H-chain variable region; and SEQ ID NO: 53 shows the nucleotide sequence and SEQ ID NO: 54 shows the amino acid sequence of the L-chain variable region. For 4F12, SEQ ID NO: 55 shows the nucleotide sequence and SEQ ID NO: 56 shows the amino acid sequence of the H-chain variable region; and SEQ ID NO: 57 shows the nucleotide sequence and SEQ ID NO: 58 shows the amino acid sequence of the L-chain variable region. Additionally, SEQ ID NOs: 77 to 88 show the sequences without the signal sequences attached thereto (SEQ ID NO: 77: nucleotide sequence H-chain variable region of 4F1 and SEQ ID NO: 78: amino acid sequence of H-chain variable region of 4F1; SEQ ID NO: 79: nucleotide sequence of L-chain variable region of 4F1 and SEQ ID NO: 80: amino acid sequence of L-chain variable region of 4F1, SEQ ID NO: 81: nucleotide sequence H-chain variable region of 4F8 and SEQ ID NO: 82: amino acid sequence of H-chain variable region of 4F8; SEQ ID NO: 83: nucleotide sequence of L-chain variable region of 4F8 and SEQ ID NO: 84: amino acid sequence of L-chain variable region of 4F8, SEQ ID NO: 85: nucleotide sequence H-chain variable region of 4F12 and SEQ ID NO: 86: amino acid sequence of H-chain variable region of 4F12; SEQ ID NO: 87: nucleotide sequence of L-chain variable region of 4F12 and SEQ ID NO: 88: amino acid sequence of L-chain variable region of 4F12).

### INDUSTRIAL APPLICABILITY

The antibody specific to the PRG-3 protein in the present invention can be used as a diagnostic agent for HCC, lung cancer, colon cancer, or glioblastoma, etc. The diagnostic agent of the present invention is useful for the diagnosis of primary or metastatic cancers. More specifically, the likelihood of cancer can be evaluated by detecting the PRG-3 protein or mRNA encoding PRG-3 contained in a biological sample collected from a patient. Alternatively, the presence of primary HCC, metastatic HCC, lung cancer, colon cancer or glioblastoma can be detected in vivo by detecting the localization of cells expressing PRG-3 in the body.

Moreover, the anti-PRG-3 antibody having cytotoxic activity of the present invention is useful for the prevention or treatment of cancers wherein the PRG-3 protein is expressed. More specifically, the present invention provides a cytotoxic agent or cell growth inhibitor toward various cancer cells such as HCC, lung cancer, colon cancer, glioblastoma, etc. The cytotoxic agent or cell growth inhibitor of the present invention can be used to treat both primary and metastatic cancers.

In addition, the anti-PRG-3 antibody having cytotoxic activity of the present invention can be used as a therapeutic drug for various cancers such as HCC, lung cancer, colon cancer, glioblastoma, etc. In the present invention, the anti-PRG-3 antibody is useful as a therapeutic drug for the treatment of both primary and metastatic cancers.

Additionally, a gene encoding the antibody of the present invention and recombinant cells transformed by that gene can be used for the effects disclosed above or to prepare a recombinant antibody providing an even more preferable effect.

Moreover, it can be said that a compound selected by the screening method of the present invention is a compound that inhibits the proliferation of cancer. A compound selected according to the present invention is useful as a candidate compound for elucidating its utility as an anticancer agent through evaluations of safety and specificity to cancer.

## Claims

1. An antibody which binds to PRG-3 protein and has cell growth inhibitory activity toward a cell expressing the PRG-3 protein.

2. The antibody according to claim 1, wherein the cell growth inhibitory activity is cytotoxic activity.

3. The antibody according to claim 2, wherein the cytotoxic activity is antibody-dependent cell-mediated cytotoxicity (ADCC).

4. The antibody according to claim 2, wherein the cytotoxic activity is complement-dependent cytotoxicity (CDC).

5. A low molecular antibody prepared from the antibody according to claim 1.

6. The antibody according to any of claims 1 to 5, conjugated with a chemotherapeutic agent or a toxic peptide.

7. An antibody which binds to PRG-3 protein and is conjugated with a substance selected from the group consisting of a chemotherapeutic agent, a toxic peptide, and a radioisotope.

8. An antibody according to any of (1) to (34) below:
(1) an antibody comprising an H-chain having the amino acid sequence represented by SEQ ID NO: 2 as CDR1, the amino acid sequence represented by SEQ ID NO: 4 as CDR2, and the amino acid sequence represented by SEQ ID NO: 6 as CDR3;
(2) an antibody comprising the H-chain according to (1) having the amino acid sequence represented by SEQ ID NO: 8 as a CH;
(3) an antibody comprising the H-chain according to (1) having the amino acid sequence represented by SEQ ID NO: 10 as a CH;
(4) an antibody comprising an L-chain having the amino acid sequence represented by SEQ ID NO: 12 as CDR1, the amino acid sequence represented by SEQ ID NO: 14 as CDR2, and the amino acid sequence represented by SEQ ID NO: 16 as CDR3;
(5) an antibody comprising the L-chain according to (4) having the amino acid sequence represented by SEQ ID NO: 18 as a CL;
(6) an antibody comprising the L-chain according to (4) having the amino acid sequence represented by SEQ ID NO: 20 as a CL;
(7) an antibody comprising the H-chain according to (1) and the L-chain according to (4);
(8) an antibody comprising the H-chain according to (2) and the L-chain according to (5);
(9) an antibody comprising the H-chain according to (3) and the L-chain according to (6);
(10) an antibody in which one or a plurality of amino acids is substituted, deleted from, added to, and/or inserted into the antibody according to any of (1) to (9), and which has activity equivalent to the antibody according to any of (1) to (9);
(11) an antibody capable of binding to the same epitope as the epitope on the PRG-3 protein to which the antibody according to any of (1) to (10) is capable of binding;
(12) an antibody comprising an H-chain having the amino acid sequence represented by SEQ ID NO: 22 as CDR1, the amino acid sequence represented by SEQ ID NO: 24 as CDR2, and the amino acid sequence represented by SEQ ID NO: 26 as CDR3;
(13) an antibody comprising the H-chain according to (12) having the amino acid sequence represented by SEQ ID NO: 34 as a CH;
(14) an antibody comprising the H-chain according to (12) having the amino acid sequence represented by SEQ ID NO: 10 as a CH;
(15) an antibody comprising an L-chain having the amino acid sequence represented by SEQ ID NO: 28 as CDR1, the amino acid sequence represented by SEQ ID NO: 30 as CDR2, and the amino acid sequence represented by SEQ ID NO: 32 as CDR3;
(16) an antibody comprising the L-chain according to (15) having the amino acid sequence represented by SEQ ID NO: 18 as a CL;
(17) an antibody comprising the L-chain according to (15) having the amino acid sequence represented by SEQ ID NO: 20 as a CL;
(18) an antibody comprising the H-chain according to (12) and the L-chain according to (15);
(19) an antibody comprising the H-chain according to (13) and the L-chain according to (16);
(20) an antibody comprising the H-chain according to (14) and the L-chain according to (17);
(21) an antibody in which one or a plurality of amino acids is substituted, deleted from, added to, and/or inserted into the antibody according to any of (12) to (20), and which has activity equivalent to the antibody according to any of (12) to (20);
(22) an antibody capable of binding to the same epitope as the epitope on the PRG-3 protein to which the antibody according to any of (12) to (21) is capable of binding;
(23) an antibody comprising an H-chain having the amino acid sequence represented by SEQ ID NO: 36 as CDR1, the amino acid sequence represented by SEQ ID NO: 38 as CDR2, and the amino acid sequence represented by SEQ ID NO: 40 as CDR3;
(24) an antibody comprising the H-chain according to (23) having the amino acid sequence represented by SEQ ID NO: 34 as a CH;
(25) an antibody comprising the H-chain according to (23) having the amino acid sequence represented by SEQ ID NO: 10 as a CH;
(26) an antibody comprising an L-chain having the amino acid sequence represented by SEQ ID NO: 42 as CDR1, the amino acid sequence represented by SEQ ID NO: 44 as CDR2, and the amino acid sequence represented by SEQ ID NO: 46 as CDR3;
(27) an antibody comprising the L-chain according to (26) having the amino acid sequence represented by SEQ ID NO: 18 as a CL;
(28) an antibody comprising the L-chain according to (26) having the amino acid sequence represented by SEQ ID NO: 20 as a CL;
(29) an antibody comprising the H-chain according to (23) and the L-chain according to (26);
(30) an antibody comprising the H-chain according to (24) and the L-chain according to (27);
(31) an antibody comprising the H-chain according to (25) and the L-chain according to (28);
(32) an antibody in which one or a plurality of amino acids is substituted, deleted from, added to, and/or inserted into the antibody according to any of (23) to (31), and which has activity equivalent to the antibody according to any of (23) to (31);
(33) an antibody which binds to the same epitope as the epitope on the PRG-3 protein to which the antibody according to any of (23) to (32) binds; and
(34) a low molecular antibody of the antibody according to any of (1) to (33).

9. A pharmaceutical composition comprising the antibody according to any of claims 1 to 8 as an active ingredient.

10. A cell growth inhibitor comprising the antibody according to any of claims 1 to 8 as an active ingredient.

11. An anticancer agent comprising the antibody according to any of claims 1 to 8 as an active ingredient.

12. The anticancer agent according to claim 11, wherein the cancer is selected from the group consisting of hepatocellular carcinoma, lung cancer, colon cancer, and glioblastoma.

13. The anticancer agent according to claim 12, wherein the cancer is a primary cancer.

14. The anticancer agent according to claim 12, wherein the cancer is a metastatic cancer.

15. A diagnostic method for cancer comprising detecting the expression of a gene encoding PRG-3 protein.

16. A diagnostic method for cancer comprising detecting PRG-3 protein.

17. The diagnostic method according to claim 16, wherein the PRG-3 protein is detected using an antibody which binds to the PRG-3 protein.

18. A diagnostic method for cancer, the method comprising the steps of:
(a) providing a test sample collected from a subject; and
(b) detecting PRG-3 protein contained in the test sample of
(a) using an antibody which binds to the PRG-3 protein.

19. A diagnostic method for cancer, the method comprising the steps of:
(1) administering to a subject an antibody that has binding activity toward PRG-3 protein and labeled with a radioisotope; and
(2) detecting the accumulation of the radioisotope.

20. The diagnostic method according to any of claims 17 to 19, wherein the antibody according to claim 8 is employed.

21. The diagnostic method according to any of claims 15 to 20, wherein the cancer is selected from the group consisting of hepatocellular carcinoma, lung cancer, colon cancer, and glioblastoma.

22. The diagnostic method according to claim 21, wherein the cancer is a primary cancer.

23. The diagnostic method according to claim 21 wherein the cancer is a metastatic cancer.

24. A diagnostic agent for use in the diagnosis of cancer comprising an antibody which binds to PRG-3 protein.

25. A kit for use in the diagnosis of cancer comprising an antibody which binds to PRG-3 protein and a reagent for detecting the binding between the antibody and the PRG-3 protein.

26. Use of PRG-3 protein, PRG-3 mRNA, or both as a marker for the diagnosis of cancer.

27. Use of an antibody which binds to PRG-3 for the diagnosis of cancer.

28. A method of screening for a candidate compound to be used in an anticancer agent, the method comprising the steps of:
(1) contacting a test compound with cells that express PRG-3;
(2) measuring the level of PRG-3 expression in the cells that express PRG-3; and
(3) selecting a compound that decreases the level of PRG-3 expression compared with a control, as a candidate compound to be used in an anticancer agent.


SEQUENCE LISTING
<110> The University of Tokyo; Forerunner Pharma Research Co., Ltd.
<120> Diagnosis and treatment of cancer by using anti-PRG-3 antibody
<130> PCG-9020WO
<150> JP 2007-000804
<151> 2007-01-05
<160> 88
<170> PatentIn version 3.1
<210> 1
<211> 15
<212> DNA
<213> Mus musculus
<400> 1
agctactgga tgaac 15
<210> 2
<211> 5
<212> PRT
<213> Mus musculus
<400> 2
Ser Tyr Trp Met Asn
1               5
<210> 3
<211> 51
<212> DNA
<213> Mus musculus
<400> 3
aagattcatc cttctgatag tgaaactcac tacaatcaaa agttcaagag c 51
<210> 4
<211> 17
<212> PRT
<213> Mus musculus
<400> 4
Lys Ile His Pro Ser Asp Ser Glu Thr His Tyr Asn Gln Lys Phe Lys
1               5                   10                  15
Ser

<210> 5
<211> 24
<212> DNA
<213> Mus musculus
<400> 5
gggtataata actaccttga ctac 24
<210> 6
<211> 8
<212> PRT
<213> Mus musculus
<400> 6
Gly Tyr Asn Asn Tyr Leu Asp Tyr
1               5
<210> 7
<211> 1011
<212> DNA
<213> Mus musculus
<400> 7
gccaaaacaa cacccccatc agtctatcca ctggcccctg ggtgtggaga tacaactggt 60
tcctctgtga ctctgggatg cctggtcaag ggctacttcc ctgagtcagt gactgtgact 120
tggaactctg gatccctgtc cagcagtgtg cacaccttcc cagctctcct gcagtctgga 180
ctctacacta tgagcagctc agtgactgtc ccctccagca cttggccaag tcagaccgtc 240
acctgcagcg ttgctcaccc agccagcagc accacggtgg acaaaaaact tgagcccagc 300
gggcccattt caacaatcaa cccctgtcct ccatgcaagg agtgtcacaa atgcccagct 360
cctaacctcg agggtggacc atccgtcttc atcttccctc caaatatcaa ggatgtactc 420
atgatctccc tgacacccaa ggtcacgtgt gtggtggtgg atgtgagcga ggatgaccca 480
gacgtccgga tcagctggtt tgtgaacaac gtggaagtac acacagctca gacacaaacc 540
catagagagg attacaacag tactatccgg gtggtcagtg ccctccccat ccagcaccag 600
gactggatga gtggcaagga gttcaaatgc aaggtcaaca acaaagacct cccatcaccc 660
atcgagagaa ccatctcaaa aattaaaggg ctagtcagag ctccacaagt atacatcttg 720
ccgccaccag cagagcagtt gtccaggaaa gatgtcagtc tcacttgcct ggtcgtgggc 780
ttcaaccctg gagacatcag tgtggagtgg accagcaatg ggcatacaga ggagaactac 840
aaggacaccg caccagtcct agactctgac ggttcttact tcatatacag caagctcgat 900
ataaaaacaa gcaagtggga gaaaacagat tccttctcat gcaacgtgag acacgagggt 960
ctgaaaaatt actacctgaa gaagaccatc tcccggtctc cgggtaaatg a 1011
<210> 8
<211> 336
<212> PRT
<213> Mus musculus
<400> 8
Ala Lys Thr Thr Pro Pro Ser Val Tyr Pro Leu Ala Pro Gly Cys Gly

1                   5                   10                  15
Asp Thr Thr Gly Ser Ser Val Thr Leu Gly Cys Leu Val Lys Gly Tyr
                20                  25                  30
Phe Pro Glu Ser Val Thr Val Thr Trp Asn Ser Gly Ser Leu Ser Ser
            35                  40                  45
Ser Val His Thr Phe Pro Ala Leu Leu Gln Ser Gly Leu Tyr Thr Met
    50                  55                  60
Ser Ser Ser Val Thr Val Pro Ser Ser Thr Trp Pro Ser Gln Thr Val
65                  70                  75                  80
Thr Cys Ser Val Ala His Pro Ala Ser Ser Thr Thr Val Asp Lys Lys
                85                  90                  95
Leu Glu Pro Ser Gly Pro Ile Ser Thr Ile Asn Pro Cys Pro Pro Cys
            100                 105                 110
Lys Glu Cys His Lys Cys Pro Ala Pro Asn Leu Glu Gly Gly Pro Ser
        115                 120                 125
Val Phe Ile Phe Pro Pro Asn Ile Lys Asp Val Leu Met Ile Ser Leu
    130                 135                 140
Thr Pro Lys Val Thr Cys Val Val Val Asp Val Ser Glu Asp Asp Pro
145                 150                 155                 160
Asp Val Arg Ile Ser Trp Phe Val Asn Asn Val Glu Val His Thr Ala
                165                 170                 175
Gln Thr Gln Thr His Arg Glu Asp Tyr Asn Ser Thr Ile Arg Val Val
            180                 185                 190
Ser Ala Leu Pro Ile Gln His Gln Asp Trp Met Ser Gly Lys Glu Phe
        195                 200                 205
Lys Cys Lys Val Asn Asn Lys Asp Leu Pro Ser Pro Ile Glu Arg Thr
    210                 215                 220
Ile Ser Lys Ile Lys Gly Leu Val Arg Ala Pro Gln Val Tyr Ile Leu
225                 230                 235                 240
Pro Pro Pro Ala Glu Gln Leu Ser Arg Lys Asp Val Ser Leu Thr Cys
                245                 250                 255
Leu Val Val Gly Phe Asn Pro Gly Asp Ile Ser Val Glu Trp Thr Ser
            260                 265                 270
Asn Gly His Thr Glu Glu Asn Tyr Lys Asp Thr Ala Pro Val Leu Asp
        275                 280                 285
Ser Asp Gly Ser Tyr Phe Ile Tyr Ser Lys Leu Asp Ile Lys Thr Ser
    290                 295                 300
Lys Trp Glu Lys Thr Asp Ser Phe Ser Cys Asn Val Arg His Glu Gly
305                 310                 315                 320
Leu Lys Asn Tyr Tyr Leu Lys Lys Thr Ile Ser Arg Ser Pro Gly Lys
                325                 330                 335
<210>  9
<211>  993
<212>  DNA
<213>  homo sapiens
<400>  9
gctagcacca agggcccatc ggtcttcccc ctggcaccct cctccaagag cacctctggg      60
ggcacagcgg ccctgggctg cctggtcaag gactacttcc ccgaaccggt gacggtgtcg     120
tggaactcag gcgccctgac cagcggcgtg cacaccttcc cggctgtcct acagtcctca     180
ggactctact ccctcagcag cgtggtgacc gtgccctcca gcagcttggg cacccagacc     240
tacatctgca acgtgaatca caagcccagc aacaccaagg tggacaagaa agttgagccc     300
aaatcttgtg acaaaactca cacatgccca ccgtgcccag cacctgaact cctgggggga     360
ccgtcagtct tcctcttccc cccaaaaccc aaggacaccc tcatgatctc ccggacccct     420
gaggtcacat gcgtggtggt ggacgtgagc cacgaagacc ctgaggtcaa gttcaactgg     480
tacgtggacg gcgtggaggt gcataatgcc aagacaaagc cgcgggagga gcagtacaac     540
agcacgtacc gtgtggtcag cgtcctcacc gtcctgcacc aggactggct gaatggcaag     600
gagtacaagt gcaaggtctc caacaaagcc ctcccagccc ccatcgagaa aaccatctcc     660
aaagccaaag ggcagccccg agaaccacag gtgtacaccc tgcccccatc ccgggatgag     720
ctgaccaaga accaggtcag cctgacctgc ctggtcaaag gcttctatcc cagcgacatc     780
gccgtggagt gggagagcaa tgggcagccg gagaacaact acaagaccac gcctcccgtg     840
ctggactccg acggctcctt cttcctctac agcaagctca ccgtggacaa gagcaggtgg     900
cagcagggga acgtcttctc atgctccgtg atgcatgagg ctctgcacaa ccactacacg     960
cagaagagcc tctccctgtc tccgggtaaa tga                                  993
<210>  10
<211>  330
<212>  PRT
<213>  homo sapiens
<400>  10
<210>  11
<211>  45
<212>  DNA
<213>  Mus musculus
<400>  11
agagccagtg aaagtgttga atattatggc actagtttaa tgcag                    45
<210>  12
<211>  15
<212>  PRT
<213>  Mus musculus
<400>  12
Arg Ala Ser Glu Ser Val Glu Tyr Tyr Gly Thr Ser Leu Met Gln
1               5                   10                  15
<210>  13
<211>  21
<212>  DNA
<213>  Mus musculus
<400>  13
ggtgcatcca acgtagaatc t                                              21
<210>  14
<211>  7
<212>  PRT
<213>  Mus musculus
<400>  14
Gly Ala Ser Asn Val Glu Ser
1               5
<210>  15
<211>  27
<212>  DNA
<213>  Mus musculus
<400>  15
cagcaaagta ggaaggttcc ttcgacg                                        27
<210>  16
<211>  9
<212>  PRT
<213>  Mus musculus
<400>  16
Gln Gln Ser Arg Lys Val Pro Ser Thr
1               5
<210>  17
<211>  324
<212>  DNA
<213>  Mus musculus
<400>  17

cgggctgatg ctgcaccaac tgtatccatc ttcccaccat ccagtgagca gttaacatct 60
ggaggtgcct cagtcgtgtg cttcttgaac aacttctacc ccaaagacat caatgtcaag 120
tggaagattg atggcagtga acgacaaaat ggcgtcctga acagttggac tgatcaggac 180
agcaaagaca gcacctacag catgagcagc accctcacgt tgaccaagga cgagtatgaa 240
cgacataaca gctatacctg tgaggccact cacaagacat caacttcacc cattgtcaag 300
agcttcaaca ggaatgagtg ttag 324
<210> 18
<211> 107
<212> PRT
<213> Mus musculus
<400> 18
Arg Ala Asp Ala Ala Pro Thr Val Ser Ile Phe Pro Pro Ser Ser Glu
1               5                   10                  15
Gln Leu Thr Ser Gly Gly Ala Ser Val Val Cys Phe Leu Asn Asn Phe
            20                  25                  30
Tyr Pro Lys Asp Ile Asn Val Lys Trp Lys Ile Asp Gly Ser Glu Arg
        35                  40                  45
Gln Asn Gly Val Leu Asn Ser Trp Thr Asp Gln Asp Ser Lys Asp Ser
    50                  55                  60
Thr Tyr Ser Met Ser Ser Thr Leu Thr Leu Thr Lys Asp Glu Tyr Glu
65                  70                  75                  80
Arg His Asn Ser Tyr Thr Cys Glu Ala Thr His Lys Thr Ser Thr Ser
                85                  90                  95
Pro Ile Val Lys Ser Phe Asn Arg Asn Glu Cys
            100                 105
<210> 19
<211> 324
<212> DNA
<213> homo sapiens
<400> 19
cgtacggtgg ctgcaccatc tgtcttcatc ttcccgccat ctgatgagca gttgaaatct 60
ggaactgcct ctgttgtgtg cctgctgaat aacttctatc ccagagaggc caaagtacag 120
tggaaggtgg ataacgccct ccaatcgggt aactcccagg agagtgtcac agagcaggac 180
agcaaggaca gcacctacag cctcagcagc accctgacgc tgagcaaagc agactacgag 240
aaacacaaag tctacgcctg cgaagtcacc catcagggcc tgagctcgcc cgtcacaaag 300
agcttcaaca ggggagagtg ttga 324
<210> 20
<211> 107
<212> PRT
<213> homo sapiens
<400> 20
<210> 21
<211> 15
<212> DNA
<213> Mus musculus
<400> 21
agctactgga tgaac 15
<210> 22
<211> 5
<212> PRT
<213> Mus musculus
<400> 22
Ser Tyr Trp Met Asn
1               5
<210> 23
<211> 51
<212> DNA
<213> Mus musculus
<400> 23
aagattcatc cttctgatag tgaaactcac tacaatcaaa agttcaagag c 51
<210> 24
<211> 17
<212> PRT

<213>  Mus musculus
<400>  24
Lys Ile His Pro Ser Asp Ser Glu Thr His Tyr Asn Gln Lys Phe Lys
1               5                   10                  15
Ser

<210>  25
<211>  33
<212>  DNA
<213>  Mus musculus
<400>  25
tcgggtggta actacggggc ctggtttgtt tac                                33
<210>  26
<211>  11
<212>  PRT
<213>  Mus musculus
<400>  26
Ser Gly Gly Asn Tyr Gly Ala Trp Phe Val Tyr
1               5                   10
<210>  27
<211>  45
<212>  DNA
<213>  Mus musculus
<400>  27
agagccagtg aaagtgttga atattatggc actagtttta tgcag                   45
<210>  28
<211>  15
<212>  PRT
<213>  Mus musculus
<400>  28
Arg Ala Ser Glu Ser Val Glu Tyr Tyr Gly Thr Ser Phe Met Gln
1               5                   10                  15
<210>  29
<211>  21
<212>  DNA
<213>  Mus musculus
<400>  29
ggtgcatcca acgtaggatc t                                             21
<210>  30
<211>  7
<212>  PRT
<213>  Mus musculus
<400>  30
Gly Ala Ser Asn Val Gly Ser
1               5
<210>  31
<211>  27
<212>  DNA
<213>  Mus musculus
<400>  31
cagcaaagta ggaaggttcc gtatacg                                       27
<210>  32
<211>  9
<212>  PRT
<213>  Mus musculus
<400>  32
Gln Gln Ser Arg Lys Val Pro Tyr Thr
1               5
<210>  33
<211>  993
<212>  DNA
<213>  Mus musculus
<400>  33
gccaaaacaa cagccccatc ggtctatcca ctggcccctg tgtgtggaga tacaactggc      60
tcctcggtga ctctaggatg cctggtcaag ggttatttcc ctgagccagt gaccttgacc     120
tggaactctg gatccctgtc cagtggtgtg cacaccttcc cagctgtcct gcagtctgac     180
ctctacaccc tcagcagctc agtgactgta acctcgagca cctggcccag ccagtccatc     240
acctgcaatg tggcccaccc ggcaagcagc accaaggtgg acaagaaaat tgagcccaga     300
gggcccacaa tcaagccctg tcctccatgc aaatgcccag cacctaacct cttgggtgga     360
ccatccgtct tcatcttccc tccaaagatc aaggatgtac tcatgatctc cctgagcccc     420
atagtcacat gtgtggtggt ggatgtgagc gaggatgacc cagatgtcca gatcagctgg     480
tttgtgaaca acgtggaagt acacacagct cagacacaaa cccatagaga ggattacaac     540
agtactctcc gggtggtcag tgccctcccc atccagcacc aggactggat gagtggcaag     600
gagttcaaat gcaaggtcaa caacaaagac ctcccagcgc ccatcgagag aaccatctca     660
aaacccaaag ggtcagtaag agctccacag gtatatgtct tgcctccacc agaagaagag     720
atgactaaga aacaggtcac tctgacctgc atggtcacag acttcatgcc tgaagacatt     780
tacgtggagt ggaccaacaa cgggaaaaca gagctaaact acaagaacac tgaaccagtc     840

ctggactctg atggttctta cttcatgtac agcaagctga gagtggaaaa gaagaactgg 900
gtggaaagaa atagctactc ctgttcagtg gtccacgagg gtctgcacaa tcaccacacg 960
actaagagct tctcccggac tccgggtaaa tga 993
<210> 34
<211> 330
<212> PRT
<213> Mus musculus
<400> 34
Ala Lys Thr Thr Ala Pro Ser Val Tyr Pro Leu Ala Pro Val Cys Gly
1               5                   10                  15
Asp Thr Thr Gly Ser Ser Val Thr Leu Gly Cys Leu Val Lys Gly Tyr
            20                  25                  30
Phe Pro Glu Pro Val Thr Leu Thr Trp Asn Ser Gly Ser Leu Ser Ser
        35                  40                  45
Gly Val His Thr Phe Pro Ala Val Leu Gln Ser Asp Leu Tyr Thr Leu
    50                  55                  60
Ser Ser Ser Val Thr Val Thr Ser Ser Thr Trp Pro Ser Gln Ser Ile
65                  70                  75                  80
Thr Cys Asn Val Ala His Pro Ala Ser Ser Thr Lys Val Asp Lys Lys
                85                  90                  95
Ile Glu Pro Arg Gly Pro Thr Ile Lys Pro Cys Pro Pro Cys Lys Cys
            100                 105                 110
Pro Ala Pro Asn Leu Leu Gly Gly Pro Ser Val Phe Ile Phe Pro Pro
        115                 120                 125
Lys Ile Lys Asp Val Leu Met Ile Ser Leu Ser Pro Ile Val Thr Cys
    130                 135                 140
Val Val Val Asp Val Ser Glu Asp Asp Pro Asp Val Gln Ile Ser Trp
145                 150                 155                 160
Phe Val Asn Asn Val Glu Val His Thr Ala Gln Thr Gln Thr His Arg
                165                 170                 175
Glu Asp Tyr Asn Ser Thr Leu Arg Val Val Ser Ala Leu Pro Ile Gln
            180                 185                 190
His Gln Asp Trp Met Ser Gly Lys Glu Phe Lys Cys Lys Val Asn Asn
        195                 200                 205
Lys Asp Leu Pro Ala Pro Ile Glu Arg Thr Ile Ser Lys Pro Lys Gly
    210                 215                 220
Ser Val Arg Ala Pro Gln Val Tyr Val Leu Pro Pro Pro Glu Glu Glu
225                 230                 235                 240
Met Thr Lys Lys Gln Val Thr Leu Thr Cys Met Val Thr Asp Phe Met
                245                 250                 255
Pro Glu Asp Ile Tyr Val Glu Trp Thr Asn Asn Gly Lys Thr Glu Leu
            260                 265                 270
Asn Tyr Lys Asn Thr Glu Pro Val Leu Asp Ser Asp Gly Ser Tyr Phe
        275                 280                 285
Met Tyr Ser Lys Leu Arg Val Glu Lys Lys Asn Trp Val Glu Arg Asn
    290                 295                 300
Ser Tyr Ser Cys Ser Val Val His Glu Gly Leu His Asn His His Thr
305                 310                 315                 320
Thr Lys Ser Phe Ser Arg Thr Pro Gly Lys
                325                 330
<210> 35
<211> 15
<212> DNA
<213> Mus musculus
<400> 35
agctactgga tgaac 15
<210> 36
<211> 5
<212> PRT
<213> Mus musculus
<400> 36
Ser Tyr Trp Met Asn
1               5
<210> 37
<211> 51
<212> DNA
<213> Mus musculus
<400> 37
aagattcatc cttctgatag tgaaactcac tacaatcaaa aattcaagag c 51
<210> 38
<211> 17
<212> PRT
<213> Mus musculus
<400> 38
Lys Ile His Pro Ser Asp Ser Glu Thr His Tyr Asn Gln Lys Phe Lys
1               5                   10                  15
Ser

<210> 39
<211> 24
<212> DNA
<213> Mus musculus
<400> 39
gggtatagta actaccttga ctac 24
<210> 40
<211> 8
<212> PRT
<213> Mus musculus
<400> 40
Gly Tyr Ser Asn Tyr Leu Asp Tyr
1               5
<210> 41
<211> 45
<212> DNA
<213> Mus musculus
<400> 41
agagccagtg aaagtgttga atattatggc actagtttaa tgcag 45
<210> 42
<211> 15
<212> PRT
<213> Mus musculus
<400> 42
Arg Ala Ser Glu Ser Val Glu Tyr Tyr Gly Thr Ser Leu Met Gln
1               5                   10                  15
<210> 43
<211> 21
<212> DNA
<213> Mus musculus
<400> 43
ggtgcatcca acgtagaatc t 21
<210> 44
<211> 7
<212> PRT
<213> Mus musculus
<400> 44
Gly Ala Ser Asn Val Glu Ser
1               5
<210> 45
<211> 27
<212> DNA
<213> Mus musculus
<400> 45
cagcaaagta ggaaggttcc ttcgacg 27
<210> 46
<211> 9
<212> PRT
<213> Mus musculus
<400> 46
Gln Gln Ser Arg Lys Val Pro Ser Thr
1               5
<210> 47
<211> 405
<212> DNA
<213> Mus musculus
<400> 47
atgggatgga gctgtatcat cctcttcttg atagcagcta caggtgtcca ctcccaggtc 60
caactgcagc agcctggggc tgaactggtg aagcctgggg cttcagtgaa gctgtcctgc 120
aaggcttctg gctacacttt caccagctac tggatgaact gggtgaagca gaggcctgga 180
cgaggcctcg agtggattgg aaagattcat ccttctgata gtgaaactca ctacaatcaa 240
aagttcaaga gcaaggccac actgactgta gacaaatcct ccagcacggc ctacatccaa 300
ctcaacagcc tgacatctga ggactctgcg gtctattatt gtacaagggg gtataataac 360
taccttgact actggggcca aggcaccact ctcacagtct cctca 405
<210> 48
<211> 135
<212> PRT
<213> Mus musculus
<400> 48
Met Gly Trp Ser Cys Ile Ile Leu Phe Leu Ile Ala Ala Thr Gly Val
1               5                   10                  15
His Ser Gln Val Gln Leu Gln Gln Pro Gly Ala Glu Leu Val Lys Pro
            20                  25                  30
Gly Ala Ser Val Lys Leu Ser Cys Lys Ala Ser Gly Tyr Thr Phe Thr
        35                  40                  45
Ser Tyr Trp Met Asn Trp Val Lys Gln Arg Pro Gly Arg Gly Leu Glu

50                  55                  60
Trp Ile Gly Lys Ile His Pro Ser Asp Ser Glu Thr His Tyr Asn Gln
65                  70                  75                  80
Lys Phe Lys Ser Lys Ala Thr Leu Thr Val Asp Lys Ser Ser Ser Thr
                85                  90                  95
Ala Tyr Ile Gln Leu Asn Ser Leu Thr Ser Glu Asp Ser Ala Val Tyr
            100                 105                 110
Tyr Cys Thr Arg Gly Tyr Asn Asn Tyr Leu Asp Tyr Trp Gly Gln Gly
        115                 120                 125
Thr Thr Leu Thr Val Ser Ser
    130                 135
<210>  49
<211>  392
<212>  DNA
<213>  Mus musculus
<400>  49
tggagacaga cacactcctg ctatgggtgc tgctgctctg ggttccaggc tccactggtg      60
acattgtgct cacccaatct ccagcttctt tggctgtgtc tctagggcag agtgtcacca     120
tctcctgcag agccagtgaa agtgttgaat attatggcac tagtttaatg cagtggtatc     180
aacagaaacc aggacagcca cccaaactcc tcatctatgg tgcatccaac gtagaatctg     240
gggtccctgc caggtttagt ggcagtgggt cttggacaga cttcagcctc aacatccatc     300
ctgtggagga ggatgatatt gcaatatatt tctgtcagca aagtaggaag gttccttcga     360
cgttcggtgg aggcaccaag ctggaaatca aa                                   392
<210>  50
<211>  130
<212>  PRT
<213>  Mus musculus
<400>  50
Glu Thr Asp Thr Leu Leu Leu Trp Val Leu Leu Leu Trp Val Pro Gly
1               5                   10                  15
Ser Thr Gly Asp Ile Val Leu Thr Gln Ser Pro Ala Ser Leu Ala Val
            20                  25                  30
Ser Leu Gly Gln Ser Val Thr Ile Ser Cys Arg Ala Ser Glu Ser Val
        35                  40                  45
Glu Tyr Tyr Gly Thr Ser Leu Met Gln Trp Tyr Gln Gln Lys Pro Gly
    50                  55                  60
Gln Pro Pro Lys Leu Leu Ile Tyr Gly Ala Ser Asn Val Glu Ser Gly
65                  70                  75                  80
Val Pro Ala Arg Phe Ser Gly Ser Gly Ser Trp Thr Asp Phe Ser Leu
                85                  90                  95
Asn Ile His Pro Val Glu Glu Asp Asp Ile Ala Ile Tyr Phe Cys Gln
            100                 105                 110
Gln Ser Arg Lys Val Pro Ser Thr Phe Gly Gly Gly Thr Lys Leu Glu
        115                 120                 125
Ile Lys
    130
<210>  51
<211>  416
<212>  DNA
<213>  Mus musculus
<400>  51
tgggatggag ctgtatcatc ctcttcttgg tagcagcagc tacaggtgtc cactcccagg      60
tccaactgca gcagcctggg gctgaactgg tgaagcctgg ggcttcagtg aagctgtcct     120
gcaaggcttc tggctacact ttcaccagct actggatgaa ctgggtgaag cagaggcctg     180
gacgaggcct cgagtggatt ggaaagattc atccttctga tagtgaaact cactacaatc     240
aaaagttcaa gagcaaggcc acactgactg tagacaaatc ctccagcaca gcctacatcc     300
aactcagcag cctgacatct gaggactctg cggtctatta ttgtgcaaga tcgggtggta     360
actacggggc ctggtttgtt tactggggcc aagggactct ggtcactgtc tctgca         416
<210>  52
<211>  139
<212>  PRT
<213>  Mus musculus
<400>  52
Met Gly Trp Ser Cys Ile Ile Leu Phe Leu Val Ala Ala Ala Thr Gly
1               5                   10                  15
Val His Ser Gln Val Gln Leu Gln Gln Pro Gly Ala Glu Leu Val Lys
            20                  25                  30
Pro Gly Ala Ser Val Lys Leu Ser Cys Lys Ala Ser Gly Tyr Thr Phe
        35                  40                  45
Thr Ser Tyr Trp Met Asn Trp Val Lys Gln Arg Pro Gly Arg Gly Leu
    50                  55                  60
Glu Trp Ile Gly Lys Ile His Pro Ser Asp Ser Glu Thr His Tyr Asn
65                  70                  75                  80
Gln Lys Phe Lys Ser Lys Ala Thr Leu Thr Val Asp Lys Ser Ser Ser
                85                  90                  95
Thr Ala Tyr Ile Gln Leu Ser Ser Leu Thr Ser Glu Asp Ser Ala Val
            100                 105                 110

Tyr Tyr Cys Ala Arg Ser Gly Gly Asn Tyr Gly Ala Trp Phe Val Tyr
        115                 120                 125
Trp Gly Gln Gly Thr Leu Val Thr Val Ser Ala
    130                 135
<210> 53
<211> 393
<212> DNA
<213> Mus musculus
<400> 53
atggagacag acacactcct gctatgggtg ctgctgctct gggttccagg ctccactggt 60
gacattgtgc tcacccaatc tccagcttct ttggctgtgt ctctagggca gagtgtcacc 120
atctcctgca gagccagtga aagtgttgaa tattatggca ctagttttat gcagtggtgc 180
caacagaaac caggacagcc acccaaactc ctcatctatg gtgcatccaa cgtaggatct 240
ggggtccctg ccaggtttag tggcagtggg tctggggcag acttcagcct caacatccat 300
cctgtggagg aggatgatat tgcaatgtat ttctgtcagc aaagtaggaa ggttccgtat 360
acgttcggat cggggaccag gctggaaata aaa 393
<210> 54
<211> 131
<212> PRT
<213> Mus musculus
<400> 54
Met Glu Thr Asp Thr Leu Leu Leu Trp Val Leu Leu Leu Trp Val Pro
1               5                   10                  15
Gly Ser Thr Gly Asp Ile Val Leu Thr Gln Ser Pro Ala Ser Leu Ala
            20                  25                  30
Val Ser Leu Gly Gln Ser Val Thr Ile Ser Cys Arg Ala Ser Glu Ser
        35                  40                  45
Val Glu Tyr Tyr Gly Thr Ser Phe Met Gln Trp Cys Gln Gln Lys Pro
    50                  55                  60
Gly Gln Pro Pro Lys Leu Leu Ile Tyr Gly Ala Ser Asn Val Gly Ser
65                  70                  75                  80
Gly Val Pro Ala Arg Phe Ser Gly Ser Gly Ser Gly Ala Asp Phe Ser
                85                  90                  95
Leu Asn Ile His Pro Val Glu Glu Asp Asp Ile Ala Met Tyr Phe Cys
            100                 105                 110
Gln Gln Ser Arg Lys Val Pro Tyr Thr Phe Gly Ser Gly Thr Arg Leu
        115                 120                 125
Glu Ile Lys
    130
<210> 55
<211> 408
<212> DNA
<213> Mus musculus
<400> 55
atgggatgga gctgtatcat cctcttcttg gtagcagcag ctacaggtgt ccactcccag 60
gtccaactgc agcagcctgg ggctgaactg gtgaagcctg gggcttcagt gaagctgtcc 120
tgcaaggctt ctggctacac tttcaccagc tactggatga actgggtgaa gcagaggcct 180
ggacgaggcc tcgagtggat tggaaagatt catccttctg atagtgaaac tcactacaat 240
caaaaattca agagcaaggc cacactgact gtagacaaat cctccagcac agcctacatc 300
caactcagca gcctgacatc tgaggactct gcggtctatt attgtgcaag ggggtatagt 360
aactaccttg actactgggg ccaaggcacc actctcacag tctcctca 408
<210> 56
<211> 136
<212> PRT
<213> Mus musculus
<400> 56
Met Gly Trp Ser Cys Ile Ile Leu Phe Leu Val Ala Ala Ala Thr Gly
1               5                   10                  15
Val His Ser Gln Val Gln Leu Gln Gln Pro Gly Ala Glu Leu Val Lys
            20                  25                  30
Pro Gly Ala Ser Val Lys Leu Ser Cys Lys Ala Ser Gly Tyr Thr Phe
        35                  40                  45
Thr Ser Tyr Trp Met Asn Trp Val Lys Gln Arg Pro Gly Arg Gly Leu
    50                  55                  60
Glu Trp Ile Gly Lys Ile His Pro Ser Asp Ser Glu Thr His Tyr Asn
65                  70                  75                  80
Gln Lys Phe Lys Ser Lys Ala Thr Leu Thr Val Asp Lys Ser Ser Ser
                85                  90                  95
Thr Ala Tyr Ile Gln Leu Ser Ser Leu Thr Ser Glu Asp Ser Ala Val
            100                 105                 110
Tyr Tyr Cys Ala Arg Gly Tyr Ser Asn Tyr Leu Asp Tyr Trp Gly Gln
        115                 120                 125
Gly Thr Thr Leu Thr Val Ser Ser
    130                 135
<210> 57
<211> 393
<212> DNA

<213>  Mus musculus
<400>  57
atggagacag acacactcct gctatgggtg ctgctgctct gggttccagg ttccactggt     60
gacattgtgc tcacccaatc tccagcttct ttggctgtgt ctctagggca gagagtcacc    120
atctcctgca gagccagtga aagtgttgaa tattatggca ctagtttaat gcagtggtac    180
caacagaaac caggacagcc acccaaactc ctcatctatg gtgcatccaa cgtagaatct    240
ggggtccctg ccaggtttag tggcagtggg tctgggacag acttcagcct caacatccat    300
cctgtggagg aggatgatat tgcaatgtat ttctgtcagc aaagtaggaa ggttccttcg    360
acgttcggtg gaggcaccaa gctggaaatc aaa                                 393
<210>  58
<211>  131
<212>  PRT
<213>  Mus musculus
<400>  58
Met Glu Thr Asp Thr Leu Leu Leu Trp Val Leu Leu Leu Trp Val Pro
1               5                   10                  15
Gly Ser Thr Gly Asp Ile Val Leu Thr Gln Ser Pro Ala Ser Leu Ala
            20                  25                  30
Val Ser Leu Gly Gln Arg Val Thr Ile Ser Cys Arg Ala Ser Glu Ser
        35                  40                  45
Val Glu Tyr Tyr Gly Thr Ser Leu Met Gln Trp Tyr Gln Gln Lys Pro
    50                  55                  60
Gly Gln Pro Pro Lys Leu Leu Ile Tyr Gly Ala Ser Asn Val Glu Ser
65                  70                  75                  80
Gly Val Pro Ala Arg Phe Ser Gly Ser Gly Ser Gly Thr Asp Phe Ser
                85                  90                  95
Leu Asn Ile His Pro Val Glu Glu Asp Asp Ile Ala Met Tyr Phe Cys
            100                 105                 110
Gln Gln Ser Arg Lys Val Pro Ser Thr Phe Gly Gly Gly Thr Lys Leu
        115                 120                 125
Glu Ile Lys
    130
<210>  59
<211>  978
<212>  DNA
<213>  homo sapiens
<400>  59
   atggctgtag gaaacaacac tcaacgaagt tattccatca tcccgtgttt tatatttgtt     60
   gagcttgtca tcatggctgg gacagtgctg cttgcctact acttcgaatg cactgacact    120
   tttcaggtgc atatccaagg attcttctgt caggacggag acttaatgaa gccttaccca    180
   gggacagagg aagaaagctt catcacccct ctggtgctct attgtgtgct ggctgccacc    240
   ccaactgcta ttatttttat tggtgagata tccatgtatt tcataaaatc aacaagagaa    300
   tccctgattg ctcaggagaa aacaattctg accggagaat gctgttacct gaacccctta    360
   cttcgaagga tcataagatt cacaggggtg tttgcatttg gactttttgc tactgacatt    420
   tttgtaaacg ccggacaagt ggtcactggg cacttaacgc catacttcct gactgtgtgc    480
   aagccaaact acaccagtgc agactgccaa gcgcaccacc agtttataaa caatgggaac    540
   atttgtactg ggacctggaa agtgatagaa aaggctcgga gatcctttcc ctccaaacac    600
   gctgctctga gcatttactc cgccttatat gccacgatgt atattacaag cacaatcaag    660
   acgaagagca gtcgactggc caagccggtg ctgtgcctcg gaactctctg cacagccttc    720
   ctgacaggcc tcaaccgggt ctctgagtat cggaaccact gctcggacgt gattgctggt    780
   ttcatcctgg gcactgcagt ggccctgttt ctgggaatgt gtgtggttca taactttaaa    840
   ggaacgcaag gatctccttc caaacccaag cctgaggatc cccgtggagt acccctaatg    900
   gctttcccaa ggatagaaag ccctctggaa accttaagtg cacagaatca ctctgcgtcc    960
   atgaccgaag ttacctga                                                  978
<210>  60
<211>  325
<212>  PRT
<213>  homo sapiens
<400>  60
Met Ala Val Gly Asn Asn Thr Gln Arg Ser Tyr Ser Ile Ile Pro Cys
1               5                   10                  15
Phe Ile Phe Val Glu Leu Val Ile Met Ala Gly Thr Val Leu Leu Ala
            20                  25                  30
Tyr Tyr Phe Glu Cys Thr Asp Thr Phe Gln Val His Ile Gln Gly Phe
        35                  40                  45
Phe Cys Gln Asp Gly Asp Leu Met Lys Pro Tyr Pro Gly Thr Glu Glu
    50                  55                  60
Glu Ser Phe Ile Thr Pro Leu Val Leu Tyr Cys Val Leu Ala Ala Thr
65                  70                  75                  80
Pro Thr Ala Ile Ile Phe Ile Gly Glu Ile Ser Met Tyr Phe Ile Lys
                85                  90                  95
Ser Thr Arg Glu Ser Leu Ile Ala Gln Glu Lys Thr Ile Leu Thr Gly
            100                 105                 110
Glu Cys Cys Tyr Leu Asn Pro Leu Leu Arg Arg Ile Ile Arg Phe Thr
        115                 120                 125
Gly Val Phe Ala Phe Gly Leu Phe Ala Thr Asp Ile Phe Val Asn Ala
    130                 135                 140

Gly Gln Val Val Thr Gly His Leu Thr Pro Tyr Phe Leu Thr Val Cys
145                 150                 155                 160
Lys Pro Asn Tyr Thr Ser Ala Asp Cys Gln Ala His His Gln Phe Ile
                165                 170                 175
Asn Asn Gly Asn Ile Cys Thr Gly Asp Leu Glu Val Ile Glu Lys Ala
            180                 185                 190
Arg Arg Ser Phe Pro Ser Lys His Ala Ala Leu Ser Ile Tyr Ser Ala
        195                 200                 205
Leu Tyr Ala Thr Met Tyr Ile Thr Ser Thr Ile Lys Thr Lys Ser Ser
    210                 215                 220
Arg Leu Ala Lys Pro Val Leu Cys Leu Gly Thr Leu Cys Thr Ala Phe
225                 230                 235                 240
Leu Thr Gly Leu Asn Arg Val Ser Glu Tyr Arg Asn His Cys Ser Asp
                245                 250                 255
Val Ile Ala Gly Phe Ile Leu Gly Thr Ala Val Ala Leu Phe Leu Gly
            260                 265                 270
Met Cys Val Val His Asn Phe Lys Gly Thr Gln Gly Ser Pro Ser Lys
        275                 280                 285
Pro Lys Pro Glu Asp Pro Arg Gly Val Pro Leu Met Ala Phe Pro Arg
    290                 295                 300
Ile Glu Ser Pro Leu Glu Thr Leu Ser Ala Gln Asn His Ser Ala Ser
305                 310                 315                 320
Met Thr Glu Val Thr
                325
<210>  61
<211>  24
<212>  DNA
<213>  Mus musculus
<400>  61
caggggccag tggatagacc gatg                                              24
<210>  62
<211>  24
<212>  DNA
<213>  Mus musculus
<400>  62
caggggccag tggatagact gatg                                              24
<210>  63
<211>  23
<212>  DNA
<213>  Mus musculus
<400>  63
gctcactgga tggtgggaag atg                                               23
<210>  64
<211>  4
<212>  PRT
<213>  artificial
<220>
<223>  artificial linker
<400>  64
Gly Gly Gly Ser
1
<210>  65
<211>  4
<212>  PRT
<213>  artificial
<220>
<223>  artificial linker
<400>  65
Ser Gly Gly Gly
1
<210>  66
<211>  5
<212>  PRT
<213>  artificial
<220>
<223>  artificial linker
<400>  66
Gly Gly Gly Gly Ser
1               5
<210>  67
<211>  5
<212>  PRT
<213>  artificial
<220>
<223>  artificial linker
<400>  67
Ser Gly Gly Gly Gly

1               5
<210>  68
<211>  6
<212>  PRT
<213>  artificial
<220>
<223>  artificial linker
<400>  68
Gly Gly Gly Gly Gly Ser
1               5
<210>  69
<211>  6
<212>  PRT
<213>  artificial
<220>
<223>  artificial linker
<400>  69
Ser Gly Gly Gly Gly Gly
1               5
<210>  70
<211>  7
<212>  PRT
<213>  artificial
<220>
<223>  artificial linker
<400>  70
Gly Gly Gly Gly Gly Gly Ser
1               5
<210>  71
<211>  7
<212>  PRT
<213>  artificial
<220>
<223>  artificial linker
<400>  71
Ser Gly Gly Gly Gly Gly Gly
1               5
<210>  72
<211>  325
<212>  PRT
<213>  Pan troglodytes
<400>  72
Met Ala Val Gly Asn Asn Thr Gln Arg Ser Tyr Ser Ile Ile Pro Cys
1               5                   10                  15
Phe Ile Phe Val Glu Leu Val Ile Met Ala Gly Thr Val Leu Leu Ala
            20                  25                  30
Tyr Tyr Phe Glu Cys Thr Asp Thr Phe Gln Val His Ile Gln Gly Phe
        35                  40                  45
Phe Cys Gln Asp Gly Asp Leu Met Lys Pro Tyr Pro Gly Thr Glu Glu
    50                  55                  60
Glu Ser Phe Ile Thr Pro Leu Val Leu Tyr Cys Val Leu Ala Ala Thr
65                  70                  75                  80
Pro Thr Ala Ile Ile Phe Ile Gly Glu Ile Ser Met Tyr Phe Ile Lys
                85                  90                  95
Ser Thr Arg Glu Ser Leu Ile Ala Gln Glu Lys Thr Ile Leu Thr Gly
            100                 105                 110
Glu Cys Cys Tyr Leu Asn Pro Leu Leu Arg Arg Ile Ile Arg Phe Thr
        115                 120                 125
Gly Val Phe Ala Phe Gly Leu Phe Ala Thr Asp Ile Phe Val Asn Ala
    130                 135                 140
Gly Gln Val Val Thr Gly His Leu Thr Pro Tyr Phe Leu Thr Val Cys
145                 150                 155                 160
Lys Pro Asn Tyr Thr Ser Ala Asp Cys Gln Ala His His Gln Phe Ile
                165                 170                 175
Asn Asn Gly Asn Ile Cys Thr Gly Asp Leu Glu Val Ile Glu Lys Ala
            180                 185                 190
Arg Arg Ser Phe Pro Ser Lys His Ala Ala Leu Ser Ile Tyr Ser Ala
        195                 200                 205
Leu Tyr Ala Thr Met Tyr Ile Thr Ser Thr Ile Lys Thr Lys Ser Ser
    210                 215                 220
Arg Leu Ala Lys Pro Val Leu Cys Leu Gly Thr Leu Cys Thr Ala Phe
225                 230                 235                 240
Leu Thr Gly Leu Asn Arg Val Ser Glu Tyr Arg Asn His Cys Ser Asp
                245                 250                 255
Val Ile Ala Gly Phe Ile Leu Gly Thr Ala Val Ala Leu Phe Leu Gly
            260                 265                 270
Met Cys Val Val His Asn Phe Lys Gly Thr Gln Gly Ser Pro Ser Lys

        275                 280                 285
Pro Lys Pro Glu Asp Pro Arg Gly Val Pro Leu Met Ala Phe Pro Arg
    290                 295                 300
Ile Glu Ser Pro Leu Glu Thr Leu Ser Ala Gln Asn His Ser Ala Ser
305                 310                 315                 320
Met Thr Glu Val Thr
                325
<210> 73
<211> 325
<212> PRT
<213> Mus musculus
<400> 73
Met Ala Val Glu Asn Asn Thr Gln Arg Ser Tyr Ser Ile Ile Pro Cys
1               5                   10                  15
Phe Ile Phe Val Glu Leu Val Ile Met Ala Gly Thr Val Leu Leu Ala
            20                  25                  30
Tyr Tyr Phe Glu Cys Thr Asp Thr Phe Gln Val His Ile Gln Gly Phe
        35                  40                  45
Phe Cys Gln Asp Gly Asp Leu Met Lys Pro Tyr Pro Gly Thr Glu Glu
    50                  55                  60
Glu Ser Phe Ile Ser Pro Leu Val Leu Tyr Cys Val Leu Ala Ala Thr
65                  70                  75                  80
Pro Thr Ala Ile Ile Phe Ile Gly Glu Ile Ser Met Tyr Phe Ile Lys
                85                  90                  95
Ser Thr Arg Glu Ser Leu Ile Ala Glu Glu Lys Met Ile Leu Thr Gly
            100                 105                 110
Asp Cys Cys Tyr Leu Ser Pro Leu Leu Arg Arg Ile Ile Arg Phe Ile
        115                 120                 125
Gly Val Phe Ala Phe Gly Leu Phe Ala Thr Asp Ile Phe Val Asn Ala
    130                 135                 140
Gly Gln Val Val Thr Gly His Leu Thr Pro Tyr Phe Leu Thr Val Cys
145                 150                 155                 160
Gln Pro Asn Tyr Thr Ser Thr Asp Cys Arg Ala His Gln Gln Phe Ile
                165                 170                 175
Asn Asn Gly Asn Ile Cys Thr Gly Asp Leu Glu Val Ile Glu Lys Ala
            180                 185                 190
Arg Arg Ser Phe Pro Ser Lys His Ala Ala Leu Ser Ile Tyr Ser Ala
        195                 200                 205
Leu Tyr Ala Thr Met Tyr Ile Thr Ser Thr Ile Lys Thr Lys Ser Ser
    210                 215                 220
Arg Leu Ala Lys Pro Val Leu Cys Leu Gly Thr Leu Cys Thr Ala Phe
225                 230                 235                 240
Leu Thr Gly Leu Asn Arg Val Ser Glu Tyr Arg Asn His Cys Ser Asp
                245                 250                 255
Val Ile Ala Gly Phe Ile Leu Gly Thr Ala Val Ala Leu Phe Leu Gly
            260                 265                 270
Met Cys Val Val His Asn Phe Arg Gly Thr Gln Gly Ser Pro Ser Lys
        275                 280                 285
Pro Lys Pro Glu Asp Pro Arg Gly Val Pro Leu Met Ala Phe Pro Arg
    290                 295                 300
Ile Glu Ser Pro Leu Glu Thr Leu Ser Ala Gln Asn His Ser Ala Ser
305                 310                 315                 320
Met Thr Glu Val Thr
                325
<210> 74
<211> 325
<212> PRT
<213> Rattus norvegicus
<400> 74
Met Ala Val Glu Asn Asn Thr Gln Arg Ser Tyr Ser Ile Ile Pro Cys
1               5                   10                  15
Phe Ile Phe Val Glu Leu Val Ile Met Ala Gly Thr Val Leu Leu Ala
            20                  25                  30
Tyr Tyr Phe Glu Cys Thr Asp Thr Phe Gln Val His Ile Gln Gly Phe
        35                  40                  45
Phe Cys Gln Asp Gly Asp Leu Met Lys Pro Tyr Pro Gly Thr Glu Glu
    50                  55                  60
Glu Ser Phe Ile Ser Pro Leu Val Leu Tyr Cys Val Leu Ala Ala Thr
65                  70                  75                  80
Pro Thr Ala Ile Ile Phe Ile Gly Glu Ile Ser Met Tyr Phe Ile Lys
                85                  90                  95
Ser Thr Arg Glu Ser Leu Ile Ala Glu Glu Lys Met Ile Leu Thr Gly
            100                 105                 110
Asp Cys Cys Tyr Leu Ser Pro Leu Leu Arg Arg Ile Val Arg Phe Ile
        115                 120                 125
Gly Val Phe Ala Phe Gly Leu Phe Ala Thr Asp Ile Phe Val Asn Ala
    130                 135                 140

Gly Gln Val Val Thr Gly His Leu Thr Pro Tyr Phe Leu Thr Val Cys
145                 150                 155                 160
Gln Pro Asn Tyr Thr Ser Thr Asp Cys Arg Ala His His Gln Phe Ile
                165                 170                 175
Asn Asn Gly Asn Ile Cys Thr Gly Asp Leu Glu Val Ile Glu Lys Ala
            180                 185                 190
Arg Arg Ser Phe Pro Ser Lys His Ala Ala Leu Ser Ile Tyr Ser Ala
        195                 200                 205
Leu Tyr Ala Thr Met Tyr Ile Thr Ser Thr Ile Lys Thr Lys Ser Ser
    210                 215                 220
Arg Leu Ala Lys Pro Val Leu Cys Leu Gly Asp Leu Cys Thr Ala Phe
225                 230                 235                 240
Leu Thr Gly Leu Asn Arg Val Ser Glu Tyr Arg Asn His Cys Ser Asp
                245                 250                 255
Val Ile Ala Gly Phe Ile Leu Gly Thr Ala Val Ala Leu Phe Leu Gly
            260                 265                 270
Met Cys Val Val His Asn Phe Lys Gly Thr Gln Gly Ser Ala Ser Lys
        275                 280                 285
Pro Lys Pro Glu Asp Pro Arg Gly Val Pro Leu Met Ala Phe Pro Arg
    290                 295                 300
Ile Glu Ser Pro Leu Glu Thr Leu Ser Ala Gln Asn His Ser Ala Ser
305                 310                 315                 320
Met Thr Glu Val Thr
                325
<210>  75
<211>  325
<212>  PRT
<213>  Canis familiaris
<400>  75
Met Ala Val Gly Asn Asn Thr Gln Arg Ser His Ser Ile Ile Pro Cys
1               5                   10                  15
Phe Ile Phe Val Glu Leu Val Ile Met Ala Gly Thr Val Leu Leu Ala
            20                  25                  30
Tyr Tyr Phe Glu Cys Thr Asp Thr Phe Gln Val His Ile Gln Gly Phe
        35                  40                  45
Phe Cys Gln Asp Gly Asp Leu Met Lys Pro Tyr Pro Gly Thr Glu Glu
    50                  55                  60
Glu Ser Phe Ile Thr Pro Leu Val Leu Tyr Cys Val Leu Ala Ala Thr
65                  70                  75                  80
Pro Thr Ala Ile Ile Phe Ile Gly Glu Ile Ser Met Tyr Phe Ile Lys
                85                  90                  95
Ser Thr Arg Glu Ser Leu Ile Ala Gln Glu Lys Thr Ile Leu Thr Gly
            100                 105                 110
Glu Cys Cys Tyr Leu Asn Pro Leu Leu Arg Arg Ile Ile Arg Phe Ile
        115                 120                 125
Gly Val Phe Ala Phe Gly Leu Phe Ala Thr Asp Ile Phe Val Asn Ala
    130                 135                 140
Gly Gln Val Val Thr Gly His Leu Thr Pro Tyr Phe Leu Thr Val Cys
145                 150                 155                 160
Lys Pro Asn Tyr Thr Ser Thr Asp Cys Arg Ala His His Gln Phe Ile
                165                 170                 175
Asn Asn Gly Asn Ile Cys Thr Gly Asp Met Glu Val Ile Glu Lys Ala
            180                 185                 190
Arg Arg Ser Phe Pro Ser Lys His Ala Ala Leu Ser Ile Tyr Ser Ala
        195                 200                 205
Leu Tyr Ala Thr Met Tyr Ile Thr Ser Thr Ile Lys Thr Lys Ser Ser
    210                 215                 220
Arg Leu Ala Lys Pro Val Leu Cys Leu Gly Thr Leu Cys Thr Ala Phe
225                 230                 235                 240
Leu Thr Gly Leu Asn Arg Val Ser Glu Tyr Arg Asn His Cys Ser Asp
                245                 250                 255
Val Ile Ala Gly Phe Ile Leu Gly Thr Ala Val Ala Leu Phe Leu Gly
            260                 265                 270
Met Cys Val Val His Asn Phe Lys Gly Thr Gln Gly Ser Pro Ser Lys
        275                 280                 285
Pro Lys Pro Glu Asp Pro Arg Gly Val Pro Leu Met Ala Phe Pro Arg
    290                 295                 300
Ile Glu Ser Pro Leu Glu Thr Leu Ser Ala Gln Asn His Ser Ala Ser
305                 310                 315                 320
Met Thr Glu Val Thr
                325
<210>  76
<211>  309
<212>  PRT
<213>  Gallus gallus
<400>  76
Met Leu Tyr Phe Gln Leu Val Ile Met Ala Gly Thr Val Leu Leu Ala

1               5                   10                  15
Tyr Tyr Phe Glu Cys Thr Asp Thr Phe Gln Val His Ile Gln Gly Phe
                20                  25                  30
Phe Cys Gln Asp Gly Asn Leu Met Lys Pro Tyr Pro Gly Thr Glu Asp
        35                  40                  45
Glu Ser Phe Ile Ser Pro Leu Val Leu Tyr Cys Val Leu Ala Ser Thr
    50                  55                  60
Pro Thr Ala Ile Ile Phe Ile Gly Glu Ile Ser Met Tyr Phe Ile Lys
65                  70                  75                  80
Ser Thr Arg Glu Thr Leu Ile Ile Gln Glu Lys Thr Ile Leu Thr Gly
                85                  90                  95
Glu Cys Cys Tyr Leu Asn Pro Leu Leu Arg Arg Ile Ile Arg Phe Ile
            100                 105                 110
Gly Val Phe Ala Phe Gly Leu Phe Ala Thr Asp Ile Phe Val Asn Ala
        115                 120                 125
Gly Gln Val Val Thr Gly Asn Leu Ala Pro Tyr Phe Leu Thr Val Cys
    130                 135                 140
Lys Pro Asn Tyr Thr Gly Asn Asp Cys Arg Thr His His Gln Phe Ile
145                 150                 155                 160
Asn Asn Gly Asn Ile Cys Thr Gly Asp Val Glu Val Ile Glu Lys Ala
                165                 170                 175
Arg Arg Ser Phe Pro Ser Lys His Ala Ala Leu Ser Ile Tyr Ser Ala
            180                 185                 190
Leu Tyr Ala Thr Met Tyr Ile Thr Ser Thr Ile Lys Thr Lys Ser Ser
        195                 200                 205
Arg Leu Ala Lys Pro Val Leu Cys Leu Gly Thr Leu Cys Ala Ala Phe
    210                 215                 220
Leu Thr Gly Leu Asn Arg Val Ser Glu Tyr Arg Asn His Cys Ser Asp
225                 230                 235                 240
Val Ile Ala Gly Phe Ile Leu Gly Ser Ala Val Ala Leu Phe Leu Gly
                245                 250                 255
Ile Cys Val Val His Asn Phe Lys Gly Thr His Gly Ala Pro Ser Lys
            260                 265                 270
Pro Lys Pro Glu Asp Pro Arg Gly Met Pro Leu Met Ala Phe Pro Arg
        275                 280                 285
Val Glu Ser Pro Leu Glu Thr Leu Ser Ala Gln Asn His Ser Ala Ser
    290                 295                 300
Met Thr Glu Val Thr
305
<210>  77
<211>  351
<212>  DNA
<213>  Mus musculus
<400>  77
caggtccaac tgcagcagcc tggggctgaa ctggtgaagc ctggggcttc agtgaagctg      60
tcctgcaagg cttctggcta cactttcacc agctactgga tgaactgggt gaagcagagg     120
cctggacgag gcctcgagtg gattggaaag attcatcctt ctgatagtga aactcactac     180
aatcaaaagt tcaagagcaa ggccacactg actgtagaca aatcctccag cacggcctac     240
atccaactca acagcctgac atctgaggac tctgcggtct attattgtac aagggggtat     300
aataactacc ttgactactg gggccaaggc accactctca cagtctcctc a              351
<210>  78
<211>  117
<212>  PRT
<213>  Mus musculus
<400>  78
Gln Val Gln Leu Gln Gln Pro Gly Ala Glu Leu Val Lys Pro Gly Ala
1               5                   10                  15
Ser Val Lys Leu Ser Cys Lys Ala Ser Gly Tyr Thr Phe Thr Ser Tyr
            20                  25                  30
Trp Met Asn Trp Val Lys Gln Arg Pro Gly Arg Gly Leu Glu Trp Ile
        35                  40                  45
Gly Lys Ile His Pro Ser Asp Ser Glu Thr His Tyr Asn Gln Lys Phe
    50                  55                  60
Lys Ser Lys Ala Thr Leu Thr Val Asp Lys Ser Ser Ser Thr Ala Tyr
65                  70                  75                  80
Ile Gln Leu Asn Ser Leu Thr Ser Glu Asp Ser Ala Val Tyr Tyr Cys
                85                  90                  95
Thr Arg Gly Tyr Asn Asn Tyr Leu Asp Tyr Trp Gly Gln Gly Thr Thr
            100                 105                 110
Leu Thr Val Ser Ser
        115
<210>  79
<211>  333
<212>  DNA
<213>  Mus musculus
<400>  79
gacattgtgc tcacccaatc tccagcttct ttggctgtgt ctctagggca gagtgtcacc      60

atctcctgca gagccagtga aagtgttgaa tattatggca ctagtttaat gcagtggtat 120
caacagaaac caggacagcc acccaaactc ctcatctatg gtgcatccaa cgtagaatct 180
ggggtccctg ccaggtttag tggcagtggg tcttggacag acttcagcct caacatccat 240
cctgtggagg aggatgatat tgcaatatat ttctgtcagc aaagtaggaa ggttccttcg 300
acgttcggtg gaggcaccaa gctggaaatc aaa 333
<210> 80
<211> 111
<212> PRT
<213> Mus musculus
<400> 80
Asp Ile Val Leu Thr Gln Ser Pro Ala Ser Leu Ala Val Ser Leu Gly
1               5                   10                  15
Gln Ser Val Thr Ile Ser Cys Arg Ala Ser Glu Ser Val Glu Tyr Tyr
            20                  25                  30
Gly Thr Ser Leu Met Gln Trp Tyr Gln Gln Lys Pro Gly Gln Pro Pro
        35                  40                  45
Lys Leu Leu Ile Tyr Gly Ala Ser Asn Val Glu Ser Gly Val Pro Ala
    50                  55                  60
Arg Phe Ser Gly Ser Gly Ser Trp Thr Asp Phe Ser Leu Asn Ile His
65                  70                  75                  80
Pro Val Glu Glu Asp Asp Ile Ala Ile Tyr Phe Cys Gln Gln Ser Arg
                85                  90                  95
Lys Val Pro Ser Thr Phe Gly Gly Gly Thr Lys Leu Glu Ile Lys
            100                 105                 110
<210> 81
<211> 360
<212> DNA
<213> Mus musculus
<400> 81
caggtccaac tgcagcagcc tggggctgaa ctggtgaagc ctggggcttc agtgaagctg 60
tcctgcaagg cttctggcta cactttcacc agctactgga tgaactgggt gaagcagagg 120
cctggacgag gcctcgagtg gattggaaag attcatcctt ctgatagtga aactcactac 180
aatcaaaagt tcaagagcaa ggccacactg actgtagaca aatcctccag cacagcctac 240
atccaactca gcagcctgac atctgaggac tctgcggtct attattgtgc aagatcgggt 300
ggtaactacg gggcctggtt tgtttactgg ggccaaggga ctctggtcac tgtctctgca 360
<210> 82
<211> 120
<212> PRT
<213> Mus musculus
<400> 82
Gln Val Gln Leu Gln Gln Pro Gly Ala Glu Leu Val Lys Pro Gly Ala
1               5                   10                  15
Ser Val Lys Leu Ser Cys Lys Ala Ser Gly Tyr Thr Phe Thr Ser Tyr
            20                  25                  30
Trp Met Asn Trp Val Lys Gln Arg Pro Gly Arg Gly Leu Glu Trp Ile
        35                  40                  45
Gly Lys Ile His Pro Ser Asp Ser Glu Thr His Tyr Asn Gln Lys Phe
    50                  55                  60
Lys Ser Lys Ala Thr Leu Thr Val Asp Lys Ser Ser Ser Thr Ala Tyr
65                  70                  75                  80
Ile Gln Leu Ser Ser Leu Thr Ser Glu Asp Ser Ala Val Tyr Tyr Cys
                85                  90                  95
Ala Arg Ser Gly Gly Asn Tyr Gly Ala Trp Phe Val Tyr Trp Gly Gln
            100                 105                 110
Gly Thr Leu Val Thr Val Ser Ala
        115                 120
<210> 83
<211> 333
<212> DNA
<213> Mus musculus
<400> 83
gacattgtgc tcacccaatc tccagcttct ttggctgtgt ctctagggca gagtgtcacc 60
atctcctgca gagccagtga aagtgttgaa tattatggca ctagttttat gcagtggtgc 120
caacagaaac caggacagcc acccaaactc ctcatctatg gtgcatccaa cgtaggatct 180
ggggtccctg ccaggtttag tggcagtggg tctggggcag acttcagcct caacatccat 240
cctgtggagg aggatgatat tgcaatgtat ttctgtcagc aaagtaggaa ggttccgtat 300
acgttcggat cggggaccag gctggaaatc aaa 333
<210> 84
<211> 111
<212> PRT
<213> Mus musculus
<400> 84
Asp Ile Val Leu Thr Gln Ser Pro Ala Ser Leu Ala Val Ser Leu Gly
1               5                   10                  15
Gln Ser Val Thr Ile Ser Cys Arg Ala Ser Glu Ser Val Glu Tyr Tyr
            20                  25                  30
Gly Thr Ser Phe Met Gln Trp Cys Gln Gln Lys Pro Gly Gln Pro Pro

35                  40                  45
Lys Leu Leu Ile Tyr Gly Ala Ser Asn Val Gly Ser Gly Val Pro Ala
    50                  55                  60
Arg Phe Ser Gly Ser Gly Ser Gly Ala Asp Phe Ser Leu Asn Ile His
65                  70                  75                  80
Pro Val Glu Glu Asp Asp Ile Ala Met Tyr Phe Cys Gln Gln Ser Arg
                85                  90                  95
Lys Val Pro Tyr Thr Phe Gly Ser Gly Thr Arg Leu Glu Ile Lys
            100                 105                 110
<210>  85
<211>  351
<212>  DNA
<213>  Mus musculus
<400>  85
caggtccaac tgcagcagcc tggggctgaa ctggtgaagc ctggggcttc agtgaagctg      60
tcctgcaagg cttctggcta cactttcacc agctactgga tgaactgggt gaagcagagg     120
cctggacgag gcctcgagtg gattggaaag attcatcctt ctgatagtga aactcactac     180
aatcaaaaat tcaagagcaa ggccacactg actgtagaca aatcctccag cacagcctac     240
atccaactca gcagcctgac atctgaggac tctgcggtct attattgtgc aagggggtat      300
agtaactacc ttgactactg gggccaaggc accactctca cagtctcctc a              351
<210>  86
<211>  117
<212>  PRT
<213>  Mus musculus
<400>  86
Gln Val Gln Leu Gln Gln Pro Gly Ala Glu Leu Val Lys Pro Gly Ala
1               5                   10                  15
Ser Val Lys Leu Ser Cys Lys Ala Ser Gly Tyr Thr Phe Thr Ser Tyr
            20                  25                  30
Trp Met Asn Trp Val Lys Gln Arg Pro Gly Arg Gly Leu Glu Trp Ile
        35                  40                  45
Gly Lys Ile His Pro Ser Asp Ser Glu Thr His Tyr Asn Gln Lys Phe
    50                  55                  60
Lys Ser Lys Ala Thr Leu Thr Val Asp Lys Ser Ser Ser Thr Ala Tyr
65                  70                  75                  80
Ile Gln Leu Ser Ser Leu Thr Ser Glu Asp Ser Ala Val Tyr Tyr Cys
                85                  90                  95
Ala Arg Gly Tyr Ser Asn Tyr Leu Asp Tyr Trp Gly Gln Gly Thr Thr
            100                 105                 110
Leu Thr Val Ser Ser
        115
<210>  87
<211>  333
<212>  DNA
<213>  Mus musculus
<400>  87
gacattgtgc tcacccaatc tccagcttct ttggctgtgt ctctagggca gagagtcacc      60
atctcctgca gagccagtga aagtgttgaa tattatggca ctagtttaat gcagtggtac     120
caacagaaac caggacagcc acccaaactc ctcatctatg gtgcatccaa cgtagaatct     180
ggggtccctg ccaggtttag tggcagtggg tctgggacag acttcagcct caacatccat     240
cctgtggagg aggatgatat tgcaatgtat ttctgtcagc aaagtaggaa ggttccttcg     300
acgttcggtg gaggcaccaa gctggaaatc aaa                                  333
<210>  88
<211>  111
<212>  PRT
<213>  Mus musculus
<400>  88
Asp Ile Val Leu Thr Gln Ser Pro Ala Ser Leu Ala Val Ser Leu Gly
1               5                   10                  15
Gln Arg Val Thr Ile Ser Cys Arg Ala Ser Glu Ser Val Glu Tyr Tyr
            20                  25                  30
Gly Thr Ser Leu Met Gln Trp Tyr Gln Gln Lys Pro Gly Gln Pro Pro
        35                  40                  45
Lys Leu Leu Ile Tyr Gly Ala Ser Asn Val Glu Ser Gly Val Pro Ala
    50                  55                  60
Arg Phe Ser Gly Ser Gly Ser Gly Thr Asp Phe Ser Leu Asn Ile His
65                  70                  75                  80
Pro Val Glu Glu Asp Asp Ile Ala Met Tyr Phe Cys Gln Gln Ser Arg
                85                  90                  95
Lys Val Pro Ser Thr Phe Gly Gly Gly Thr Lys Leu Glu Ile Lys
            100                 105                 110

SEQUENCE LISTING
<110> The University of Tokyo; Forerunner Pharma Research Co., Ltd.
<120> Diagnosis and treatment of cancers using anti-PRG-3 antibody
<130> PCG-9020WO
<150> JP 2007-000804
<151> 2007-01-05
<160> 88
<170> PatentIn version 3.1
<210> 1
<211> 15
<212> DNA
<213> Mus musculus
<400> 1
agctactgga tgaac 15
<210> 2
<211> 5
<212> PRT
<213> Mus musculus
<400> 2
Ser Tyr Trp Met Asn
1               5
<210> 3
<211> 51
<212> DNA
<213> Mus musculus
<400> 3
aagattcatc cttctgatag tgaaactcac tacaatcaaa agttcaagag c 51
<210> 4
<211> 17
<212> PRT
<213> Mus musculus
<400> 4
Lys Ile His Pro Ser Asp Ser Glu Thr His Tyr Asn Gln Lys Phe Lys
1               5                   10                  15
Ser

<210> 5
<211> 24
<212> DNA
<213> Mus musculus
<400> 5
gggtataata actaccttga ctac 24
<210> 6
<211> 8
<212> PRT
<213> Mus musculus
<400> 6
Gly Tyr Asn Asn Tyr Leu Asp Tyr
1               5
<210> 7
<211> 1011
<212> DNA
<213> Mus musculus
<400> 7
gccaaaacaa cacccccatc agtctatcca ctggcccctg ggtgtggaga tacaactggt 60
tcctctgtga ctctgggatg cctggtcaag ggctacttcc ctgagtcagt gactgtgact 120
tggaactctg gatccctgtc cagcagtgtg cacaccttcc cagctctcct gcagtctgga 180
ctctacacta tgagcagctc agtgactgtc ccctccagca cttggccaag tcagaccgtc 240
acctgcagcg ttgctcaccc agccagcagc accacggtgg acaaaaaact tgagcccagc 300
gggcccattt caacaatcaa cccctgtcct ccatgcaagg agtgtcacaa atgcccagct 360
cctaacctcg agggtggacc atccgtcttc atcttccctc caaatatcaa ggatgtactc 420
atgatctccc tgacacccaa ggtcacgtgt gtggtggtgg atgtgagcga ggatgaccca 480
gacgtccgga tcagctggtt tgtgaacaac gtggaagtac acacagctca gacacaaacc 540
catagagagg attacaacag tactatccgg gtggtcagtg ccctccccat ccagcaccag 600
gactggatga gtggcaagga gttcaaatgc aaggtcaaca acaaagacct cccatcaccc 660
atcgagagaa ccatctcaaa aattaaaggg ctagtcagag ctccacaagt atacatcttg 720
ccgccaccag cagagcagtt gtccaggaaa gatgtcagtc tcacttgcct ggtcgtgggc 780
ttcaaccctg gagacatcag tgtggagtgg accagcaatg ggcatacaga ggagaactac 840
aaggacaccg caccagtcct agactctgac ggttcttact tcatatacag caagctcgat 900

at aaaaacaa gcaagt ggga gaaaacagat t cct t ct cat gcaacgt gag acacgagggt 960
ct gaaaaat t act acct gaa gaagaccat c t cccggt ct c cgggt aaat g a 1011
<210> 8
<211> 336
<212> PRT
<213> Mus musculus
<400> 8
   Ala Lys Thr Thr Pro Pro Ser Val Tyr Pro Leu Ala Pro Gly Cys Gly
   1 5 10 15
   Asp Thr Thr Gly Ser Ser Val Thr Leu Gly Cys Leu Val Lys Gly Tyr
   20 25 30
   Phe Pro Glu Ser Val Thr Val Thr Trp Asn Ser Gly Ser Leu Ser Ser
   35 40 45
   Ser Val His Thr Phe Pro Ala Leu Leu Gln Ser Gly Leu Tyr Thr Met
   50 55 60
   Ser Ser Ser Val Thr Val Pro Ser Ser Thr Trp Pro Ser Gln Thr Val
   65 70 75 80
   Thr Cys Ser Val Ala His Pro Ala Ser Ser Thr Thr Val Asp Lys Lys
   85 90 95
   Leu Glu Pro Ser Gly Pro Ile Ser Thr Ile Asn Pro Cys Pro Pro Cys
   100 105 110
   Lys Glu Cys His Lys Cys Pro Ala Pro Asn Leu Glu Gly Gly Pro Ser
   115 120 125
   Val Phe Ile Phe Pro Pro Asn Ile Lys Asp Val Leu Met Ile Ser Leu
   130 135 140
   Thr Pro Lys Val Thr Cys Val Val Val Asp Val Ser Glu Asp Asp Pro
   145 150 155 160
   Asp Val Arg Ile Ser Trp Phe Val Asn Asn Val Glu Val His Thr Ala
   165 170 175
   Gln Thr Gln Thr His Arg Glu Asp Tyr Asn Ser Thr Ile Arg Val Val
   180 185 190
   Ser Ala Leu Pro Ile Gln His Gln Asp Trp Met Ser Gly Lys Glu Phe
   195 200 205
   Lys Cys Lys Val Asn Asn Lys Asp Leu Pro Ser Pro Ile Glu Arg Thr
   210 215 220
   Ile Ser Lys Ile Lys Gly Leu Val Arg Ala Pro Gln Val Tyr Ile Leu
   225 230 235 240
   Pro Pro Pro Ala Glu Gln Leu Ser Arg Lys Asp Val Ser Leu Thr Cys
   245 250 255
   Leu Val Val Gly Phe Asn Pro Gly Asp Ile Ser Val Glu Trp Thr Ser
   260 265 270
   Asn Gly His Thr Glu Glu Asn Tyr Lys Asp Thr Ala Pro Val Leu Asp
   275 280 285
   Ser Asp Gly Ser Tyr Phe Ile Tyr Ser Lys Leu Asp Ile Lys Thr Ser
   290 295 300
   Lys Trp Glu Lys Thr Asp Ser Phe Ser Cys Asn Val Arg His Glu Gly
   305 310 315 320
   Leu Lys Asn Tyr Tyr Leu Lys Lys Thr Ile Ser Arg Ser Pro Gly Lys
   325 330 335
<210> 9
<211> 993
<212> DNA
<213> homo sapi ens
<400> 9
gct agcacca agggcccat c ggt ct t cccc ct ggcaccct cct ccaagag cacct ct ggg 60
ggcacagcgg ccct gggct g cct ggt caag gact act t cc ccgaaccggt gacggt gt cg 120
t ggaact cag gcgccct gac cagcggcgt g cacacct t cc cggct gt cct acagt cct ca 180
ggact ct act ccct cagcag cgt ggt gacc gt gccct cca gcagct t ggg cacccagacc 240
t acat ct gca acgt gaat ca caagcccagc aacaccaagg t ggacaagaa agt t gagccc 300
aaat ct t gt g acaaaact ca cacat gccca ccgt gcccag cacct gaact cct ggggga 360
ccgt cagt ct t cct ct t ccc cccaaaaccc aaggacaccc t cat gat ct c ccggacccct 420
gaggt cacat gcgt ggt ggt ggacgt gagc cacgaagacc ct gaggt caa gt t caact gg 480
t acgt ggacg gcgt ggaggt gcat aat gcc aagacaaagc cgcgggagga gcagt acaac 540
agcacgt acc gt gt ggt cag cgt cct cacc gt cct gcacc aggact ggct gaat ggcaag 600
gagt acaagt gcaaggt ct c caacaaagcc ct cccagccc ccat cgagaa aaccat ct cc 660
aaagccaaag ggcagccccg agaaccacag gt gt acaccc t gcccccat c ccgggat gag 720
ct gaccaaga accaggt cag cct gacct gc ct ggt caaag gct t ct at cc cagcgacat c 780
gccgt ggagt gggagagcaa t gggcagccg gagaacaact acaagaccac gcct cccgt g 840
ct ggact ccg acggct cct t ct t cct ct ac agcaagct ca ccgt ggacaa gagcaggt gg 900

cagcagggga acgtcttctc atgctccgtg atgcatgagg ctctgcacaa ccactacacg 960
cagaagagcc tctccctgtc tccgggtaaa tga 993
<210> 10
<211> 330
<212> PRT
<213> homo sapiens
<400> 10
<210> 11
<211> 45
<212> DNA
<213> Mus musculus
<400> 11
agagccagtg aaagtgttga atattatggc actagtttaa tgcag 45
<210> 12
<211> 15
<212> PRT
<213> Mus musculus
<400> 12
Arg Ala Ser Glu Ser Val Glu Tyr Tyr Gly Thr Ser Leu Met Gln
1               5                   10                  15
<210> 13
<211> 21
<212> DNA
<213> Mus musculus
<400> 13
ggtgcatcca acgtagaatc t 21
<210> 14

<211> 7
<212> PRT
<213> Mus musculus
<400> 14
Gly Ala Ser Asn Val Glu Ser
1               5
<210> 15
<211> 27
<212> DNA
<213> Mus musculus
<400> 15
cagcaaagta ggaaggttcc ttcgacg 27
<210> 16
<211> 9
<212> PRT
<213> Mus musculus
<400> 16
Gln Gln Ser Arg Lys Val Pro Ser Thr
1               5
<210> 17
<211> 324
<212> DNA
<213> Mus musculus
<400> 17
cgggctgatg ctgcaccaac tgtatccatc ttcccaccat ccagtgagca gttaacatct 60
ggaggtgcct cagtcgtgtg cttcttgaac aacttctacc ccaaagacat caatgtcaag 120
tggaagattg atggcagtga acgacaaaat ggcgtcctga acagttggac tgatcaggac 180
agcaaagaca gcacctacag catgagcagc accctcacgt tgaccaagga cgagtatgaa 240
cgacataaca gctatacctg tgaggccact cacaagacat caacttcacc cattgtcaag 300
agcttcaaca ggaatgagtg ttag 324
<210> 18
<211> 107
<212> PRT
<213> Mus musculus
<400> 18
Arg Ala Asp Ala Ala Pro Thr Val Ser Ile Phe Pro Pro Ser Ser Glu
1               5                   10                  15
Gln Leu Thr Ser Gly Gly Ala Ser Val Val Cys Phe Leu Asn Asn Phe
            20                  25                  30
Tyr Pro Lys Asp Ile Asn Val Lys Trp Lys Ile Asp Gly Ser Glu Arg
        35                  40                  45
Gln Asn Gly Val Leu Asn Ser Trp Thr Asp Gln Asp Ser Lys Asp Ser
    50                  55                  60
Thr Tyr Ser Met Ser Ser Thr Leu Thr Leu Thr Lys Asp Glu Tyr Glu
65                  70                  75                  80
Arg His Asn Ser Tyr Thr Cys Glu Ala Thr His Lys Thr Ser Thr Ser
                85                  90                  95
Pro Ile Val Lys Ser Phe Asn Arg Asn Glu Cys
            100                 105
<210> 19
<211> 324
<212> DNA
<213> homo sapiens
<400> 19
cgtacggtgg ctgcaccatc tgtcttcatc ttcccgccat ctgatgagca gttgaaatct 60
ggaactgcct ctgttgtgtg cctgctgaat aacttctatc ccagagaggc caaagtacag 120
tggaaggtgg ataacgccct ccaatcgggt aactcccagg agagtgtcac agagcaggac 180
agcaaggaca gcacctacag cctcagcagc accctgacgc tgagcaaagc agactacgag 240
aaacacaaag tctacgcctg cgaagtcacc catcagggcc tgagctcgcc cgtcacaaag 300
agcttcaaca ggggagagtg ttga 324
<210> 20
<211> 107
<212> PRT
<213> homo sapiens
<400> 20
<210> 21
<211> 15
<212> DNA
<213> Mus musculus
<400> 21
agctactgga tgaac 15
<210> 22
<211> 5
<212> PRT
<213> Mus musculus
<400> 22
Ser Tyr Trp Met Asn
1               5
<210> 23
<211> 51
<212> DNA
<213> Mus musculus
<400> 23
aagattcatc cttctgatag tgaaactcac tacaatcaaa agttcaagag c 51
<210> 24
<211> 17
<212> PRT
<213> Mus musculus
<400> 24
Lys Ile His Pro Ser Asp Ser Glu Thr His Tyr Asn Gln Lys Phe Lys
1               5                   10                  15
Ser

<210> 25
<211> 33
<212> DNA
<213> Mus musculus
<400> 25
tcgggtggta actacggggc ctggtttgtt tac 33
<210> 26
<211> 11
<212> PRT
<213> Mus musculus
<400> 26
Ser Gly Gly Asn Tyr Gly Ala Trp Phe Val Tyr
1               5                   10
<210> 27
<211> 45
<212> DNA
<213> Mus musculus
<400> 27
agagccagtg aaagtgttga atattatggc actagtttta tgcag 45
<210> 28
<211> 15
<212> PRT
<213> Mus musculus
<400> 28
Arg Ala Ser Glu Ser Val Glu Tyr Tyr Gly Thr Ser Phe Met Gln
1               5                   10                  15
<210> 29
<211> 21
<212> DNA
<213> Mus musculus
<400> 29

ggt gcat cca acgt aggat c t 21
<210> 30
<211> 7
<212> PRT
<213> Mus muscul us
<400> 30
   Gly Ala Ser Asn Val Gly Ser
   1 5
<210> 31
<211> 27
<212> DNA
<213> Mus muscul us
<400> 31
cagcaaagt a ggaaggt t cc gt at acg 27
<210> 32
<211> 9
<212> PRT
<213> Mus muscul us
<400> 32
   Gln Gln Ser Arg Lys Val Pro Tyr Thr
   1 5
<210> 33
<211> 993
<212> DNA
<213> Mus muscul us
<400> 33
gccaaaacaa cagccccat c ggt ct at cca ct ggcccct g t gt gt ggaga t acaact ggc 60
t cct cggt ga ct ct aggat g cct ggt caag ggt t at t t cc ct gagccagt gacct t gacc 120
t ggaact ct g gat ccct gt c cagt ggt gt g cacacct t cc cagct gt cct gcagt ct gac 180
ct ct acaccc t cagcagct c agt gact gt a acct cgagca cct ggcccag ccagt ccat c 240
acct gcaat g t ggcccaccc ggcaagcagc accaaggt gg acaagaaaat t gagcccaga 300
gggcccacaa t caagccct g t cct ccat gc aaat gcccag cacct aacct ct t gggt gga 360
ccat ccgt ct t cat ct t ccc t ccaaagat c aaggat gt ac t cat gat ct c cct gagcccc 420
at agt cacat gt gt ggt ggt ggat gt gagc gaggat gacc cagat gt cca gat cagct gg 480
t t t gt gaaca acgt ggaagt acacacagct cagacacaaa cccat agaga ggat t acaac 540
agt act ct cc gggt ggt cag t gccct cccc at ccagcacc aggact ggat gagt ggcaag 600
gagt t caaat gcaaggt caa caacaaagac ct cccagcgc ccat cgagag aaccat ct ca 660
aaacccaaag ggt cagt aag agct ccacag gt at at gt ct t gcct ccacc agaagaagag 720
at gact aaga aacaggt cac t ct gacct gc at ggt cacag act t cat gcc t gaagacat t 780
t acgt ggagt ggaccaacaa cgggaaaaca gagct aaact acaagaacac t gaaccagt c 840
ct ggact ct g at ggt t ct t a ct t cat gt ac agcaagct ga gagt ggaaaa gaagaact gg 900
gt ggaaagaa at agct act c ct gt t cagt g gt ccacgagg gt ct gcacaa t caccacacg 960
act aagagct t ct cccggac t ccgggt aaa t ga 993
<210> 34
<211> 330
<212> PRT
<213> Mus muscul us
<400> 34
   Ala Lys Thr Thr Ala Pro Ser Val Tyr Pro Leu Ala Pro Val Cys Gly
   1 5 10 15
   Asp Thr Thr Gly Ser Ser Val Thr Leu Gly Cys Leu Val Lys Gly Tyr
   20 25 30
   Phe Pro Glu Pro Val Thr Leu Thr Trp Asn Ser Gly Ser Leu Ser Ser
   35 40 45
   Gly Val His Thr Phe Pro Ala Val Leu Gln Ser Asp Leu Tyr Thr Leu
   50 55 60
   Ser Ser Ser Val Thr Val Thr Ser Ser Thr Trp Pro Ser Gln Ser Ile
   65 70 75 80
   Thr Cys Asn Val Ala His Pro Ala Ser Ser Thr Lys Val Asp Lys Lys
   85 90 95
   Ile Glu Pro Arg Gly Pro Thr Ile Lys Pro Cys Pro Pro Cys Lys Cys
   100 105 110
   Pro Ala Pro Asn Leu Leu Gly Gly Pro Ser Val Phe Ile Phe Pro Pro
   115 120 125
   Lys Ile Lys Asp Val Leu Met Ile Ser Leu Ser Pro Ile Val Thr Cys
   130 135 140
   Val Val Val Asp Val Ser Glu Asp Asp Pro Asp Val Gln Ile Ser Trp
   145 150 155 160
   Phe Val Asn Asn Val Glu Val His Thr Ala Gln Thr Gln Thr His Arg
   165 170 175
   Glu Asp Tyr Asn Ser Thr Leu Arg Val Val Ser Ala Leu Pro Ile Gln
   180 185 190
   His Gln Asp Trp Met Ser Gly Lys Glu Phe Lys Cys Lys Val Asn Asn
   195 200 205
   Lys Asp Leu Pro Ala Pro Ile Glu Arg Thr Ile Ser Lys Pro Lys Gly
   210 215 220
   Ser Val Arg Ala Pro Gln Val Tyr Val Leu Pro Pro Pro Glu Glu Glu
   225 230 235 240
   Met Thr Lys Lys Gln Val Thr Leu Thr Cys Met Val Thr Asp Phe Met
   245 250 255
   Pro Glu Asp Ile Tyr Val Glu Trp Thr Asn Asn Gly Lys Thr Glu Leu
   260 265 270
   Asn Tyr Lys Asn Thr Glu Pro Val Leu Asp Ser Asp Gly Ser Tyr Phe
   275 280 285
   Met Tyr Ser Lys Leu Arg Val Glu Lys Lys Asn Trp Val Glu Arg Asn
   290 295 300
   Ser Tyr Ser Cys Ser Val Val His Glu Gly Leu His Asn His His Thr
   305 310 315 320
   Thr Lys Ser Phe Ser Arg Thr Pro Gly Lys
   325 330
<210> 35
<211> 15
<212> DNA
<213> Mus musculus
<400> 35
agctactgga tgaac 15
<210> 36
<211> 5
<212> PRT
<213> Mus musculus
<400> 36
Ser Tyr Trp Met Asn
1               5
<210> 37
<211> 51
<212> DNA
<213> Mus musculus
<400> 37
aagattcatc cttctgatag tgaaactcac tacaatcaaa aattcaagag c 51
<210> 38
<211> 17
<212> PRT
<213> Mus musculus
<400> 38
Lys Ile His Pro Ser Asp Ser Glu Thr His Tyr Asn Gln Lys Phe Lys
1               5                   10                  15
Ser

<210> 39
<211> 24
<212> DNA
<213> Mus musculus
<400> 39
gggtatagta actaccttga ctac 24
<210> 40
<211> 8
<212> PRT
<213> Mus musculus
<400> 40
Gly Tyr Ser Asn Tyr Leu Asp Tyr
1               5
<210> 41
<211> 45
<212> DNA
<213> Mus musculus
<400> 41
agagccagtg aaagtgttga atattatggc actagtttaa tgcag 45
<210> 42

<211>  15
<212>  PRT
<213>  Mus musculus
<400>  42
Arg Ala Ser Glu Ser Val Glu Tyr Tyr Gly Thr Ser Leu Met Gln
1               5                   10                  15
<210>  43
<211>  21
<212>  DNA
<213>  Mus musculus
<400>  43
ggtgcatcca acgtagaatc t                                                21
<210>  44
<211>  7
<212>  PRT
<213>  Mus musculus
<400>  44
Gly Ala Ser Asn Val Glu Ser
1               5
<210>  45
<211>  27
<212>  DNA
<213>  Mus musculus
<400>  45
cagcaaagta ggaaggttcc ttcgacg                                          27
<210>  46
<211>  9
<212>  PRT
<213>  Mus musculus
<400>  46
Gln Gln Ser Arg Lys Val Pro Ser Thr
1               5
<210>  47
<211>  405
<212>  DNA
<213>  Mus musculus
<400>  47
atgggatgga gctgtatcat cctcttcttg atagcagcta caggtgtcca ctcccaggtc      60
caactgcagc agcctggggc tgaactggtg aagcctgggg cttcagtgaa gctgtcctgc     120
aaggcttctg gctacacttt caccagctac tggatgaact gggtgaagca gaggcctgga     180
cgaggcctcg agtggattgg aaagattcat ccttctgata gtgaaactca ctacaatcaa     240
aagttcaaga gcaaggccac actgactgta gacaaatcct ccagcacggc ctacatccaa     300
ctcaacagcc tgacatctga ggactctgcg gtctattatt gtacaagggg gtataataac     360
taccttgact actggggcca aggcaccact ctcacagtct cctca                     405
<210>  48
<211>  135
<212>  PRT
<213>  Mus musculus
<400>  48
Met Gly Trp Ser Cys Ile Ile Leu Phe Leu Ile Ala Ala Thr Gly Val
1               5                   10                  15
His Ser Gln Val Gln Leu Gln Gln Pro Gly Ala Glu Leu Val Lys Pro
            20                  25                  30
Gly Ala Ser Val Lys Leu Ser Cys Lys Ala Ser Gly Tyr Thr Phe Thr
        35                  40                  45
Ser Tyr Trp Met Asn Trp Val Lys Gln Arg Pro Gly Arg Gly Leu Glu
    50                  55                  60
Trp Ile Gly Lys Ile His Pro Ser Asp Ser Glu Thr His Tyr Asn Gln
65                  70                  75                  80
Lys Phe Lys Ser Lys Ala Thr Leu Thr Val Asp Lys Ser Ser Ser Thr
                85                  90                  95
Ala Tyr Ile Gln Leu Asn Ser Leu Thr Ser Glu Asp Ser Ala Val Tyr
            100                 105                 110
Tyr Cys Thr Arg Gly Tyr Asn Asn Tyr Leu Asp Tyr Trp Gly Gln Gly
        115                 120                 125
Thr Thr Leu Thr Val Ser Ser
    130                 135
<210>  49
<211>  392

<212> DNA
<213> Mus musculus
<400> 49
tggagacaga cacactcctg ctatgggtgc tgctgctctg ggttccaggc tccactggtg 60
acattgtgct cacccaatct ccagcttctt tggctgtgtc tctagggcag agtgtcacca 120
tctcctgcag agccagtgaa agtgttgaat attatggcac tagtttaatg cagtggtatc 180
aacagaaacc aggacagcca cccaaactcc tcatctatgg tgcatccaac gtagaatctg 240
gggtccctgc caggtttagt ggcagtgggt cttggacaga cttcagcctc aacatccatc 300
ctgtggagga ggatgatatt gcaatatatt tctgtcagca aagtaggaag gttccttcga 360
cgttcggtgg aggcaccaag ctggaaatca aa 392
<210> 50
<211> 130
<212> PRT
<213> Mus musculus
<400> 50
Glu Thr Asp Thr Leu Leu Leu Trp Val Leu Leu Leu Trp Val Pro Gly
1               5                   10                  15
Ser Thr Gly Asp Ile Val Leu Thr Gln Ser Pro Ala Ser Leu Ala Val
            20                  25                  30
Ser Leu Gly Gln Ser Val Thr Ile Ser Cys Arg Ala Ser Glu Ser Val
        35                  40                  45
Glu Tyr Tyr Gly Thr Ser Leu Met Gln Trp Tyr Gln Gln Lys Pro Gly
    50                  55                  60
Gln Pro Pro Lys Leu Leu Ile Tyr Gly Ala Ser Asn Val Glu Ser Gly
65                  70                  75                  80
Val Pro Ala Arg Phe Ser Gly Ser Gly Ser Trp Thr Asp Phe Ser Leu
                85                  90                  95
Asn Ile His Pro Val Glu Glu Asp Asp Ile Ala Ile Tyr Phe Cys Gln
            100                 105                 110
Gln Ser Arg Lys Val Pro Ser Thr Phe Gly Gly Gly Thr Lys Leu Glu
        115                 120                 125
Ile Lys
    130
<210> 51
<211> 416
<212> DNA
<213> Mus musculus
<400> 51
tgggatggag ctgtatcatc ctcttcttgg tagcagcagc tacaggtgtc cactcccagg 60
tccaactgca gcagcctggg gctgaactgg tgaagcctgg ggcttcagtg aagctgtcct 120
gcaaggcttc tggctacact ttcaccagct actggatgaa ctgggtgaag cagaggcctg 180
gacgaggcct cgagtggatt ggaaagattc atccttctga tagtgaaact cactacaatc 240
aaaagttcaa gagcaaggcc acactgactg tagacaaatc ctccagcaca gcctacatcc 300
aactcagcag cctgacatct gaggactctg cggtctatta ttgtgcaaga tcgggtggta 360
actacggggc ctggtttgtt tactggggcc aagggactct ggtcactgtc tctgca 416
<210> 52
<211> 139
<212> PRT
<213> Mus musculus
<400> 52
Met Gly Trp Ser Cys Ile Ile Leu Phe Leu Val Ala Ala Ala Thr Gly
1               5                   10                  15
Val His Ser Gln Val Gln Leu Gln Gln Pro Gly Ala Glu Leu Val Lys
            20                  25                  30
Pro Gly Ala Ser Val Lys Leu Ser Cys Lys Ala Ser Gly Tyr Thr Phe
        35                  40                  45
Thr Ser Tyr Trp Met Asn Trp Val Lys Gln Arg Pro Gly Arg Gly Leu
    50                  55                  60
Glu Trp Ile Gly Lys Ile His Pro Ser Asp Ser Glu Thr His Tyr Asn
65                  70                  75                  80
Gln Lys Phe Lys Ser Lys Ala Thr Leu Thr Val Asp Lys Ser Ser Ser
                85                  90                  95
Thr Ala Tyr Ile Gln Leu Ser Ser Leu Thr Ser Glu Asp Ser Ala Val
            100                 105                 110
Tyr Tyr Cys Ala Arg Ser Gly Gly Asn Tyr Gly Ala Trp Phe Val Tyr
        115                 120                 125
Trp Gly Gln Gly Thr Leu Val Thr Val Ser Ala
    130                 135
<210> 53

<211> 393
<212> DNA
<213> Mus musculus
<400> 53
atggagacag acacactcct gctatgggtg ctgctgctct gggttccagg ctccactggt 60
gacattgtgc tcacccaatc tccagcttct ttggctgtgt ctctagggca gagtgtcacc 120
atctcctgca gagccagtga aagtgttgaa tattatggca ctagttttat gcagtggtgc 180
caacagaaac caggacagcc acccaaactc ctcatctatg gtgcatccaa cgtaggatct 240
ggggtccctg ccaggtttag tggcagtggg tctggggcag acttcagcct caacatccat 300
cctgtggagg aggatgatat tgcaatgtat ttctgtcagc aaagtaggaa ggttccgtat 360
acgttcggat cggggaccag gctggaaata aaa 393
<210> 54
<211> 131
<212> PRT
<213> Mus musculus
<400> 54
Met Glu Thr Asp Thr Leu Leu Leu Trp Val Leu Leu Leu Trp Val Pro
1               5                   10                  15
Gly Ser Thr Gly Asp Ile Val Leu Thr Gln Ser Pro Ala Ser Leu Ala
            20                  25                  30
Val Ser Leu Gly Gln Ser Val Thr Ile Ser Cys Arg Ala Ser Glu Ser
        35                  40                  45
Val Glu Tyr Tyr Gly Thr Ser Phe Met Gln Trp Cys Gln Gln Lys Pro
    50                  55                  60
Gly Gln Pro Pro Lys Leu Leu Ile Tyr Gly Ala Ser Asn Val Gly Ser
65                  70                  75                  80
Gly Val Pro Ala Arg Phe Ser Gly Ser Gly Ser Gly Ala Asp Phe Ser
                85                  90                  95
Leu Asn Ile His Pro Val Glu Glu Asp Asp Ile Ala Met Tyr Phe Cys
            100                 105                 110
Gln Gln Ser Arg Lys Val Pro Tyr Thr Phe Gly Ser Gly Thr Arg Leu
        115                 120                 125
Glu Ile Lys
    130
<210> 55
<211> 408
<212> DNA
<213> Mus musculus
<400> 55
atgggatgga gctgtatcat cctcttcttg gtagcagcag ctacaggtgt ccactcccag 60
gtccaactgc agcagcctgg ggctgaactg gtgaagcctg gggcttcagt gaagctgtcc 120
tgcaaggctt ctggctacac tttcaccagc tactggatga actgggtgaa gcagaggcct 180
ggacgaggcc tcgagtggat tggaaagatt catccttctg atagtgaaac tcactacaat 240
caaaaattca agagcaaggc cacactgact gtagacaaat cctccagcac agcctacatc 300
caactcagca gcctgacatc tgaggactct gcggtctatt attgtgcaag ggggtatagt 360
aactaccttg actactgggg ccaaggcacc actctcacag tctcctca 408
<210> 56
<211> 136
<212> PRT
<213> Mus musculus
<400> 56
Met Gly Trp Ser Cys Ile Ile Leu Phe Leu Val Ala Ala Ala Thr Gly
1               5                   10                  15
Val His Ser Gln Val Gln Leu Gln Gln Pro Gly Ala Glu Leu Val Lys
            20                  25                  30
Pro Gly Ala Ser Val Lys Leu Ser Cys Lys Ala Ser Gly Tyr Thr Phe
        35                  40                  45
Thr Ser Tyr Trp Met Asn Trp Val Lys Gln Arg Pro Gly Arg Gly Leu
    50                  55                  60
Glu Trp Ile Gly Lys Ile His Pro Ser Asp Ser Glu Thr His Tyr Asn
65                  70                  75                  80
Gln Lys Phe Lys Ser Lys Ala Thr Leu Thr Val Asp Lys Ser Ser Ser
                85                  90                  95
Thr Ala Tyr Ile Gln Leu Ser Ser Leu Thr Ser Glu Asp Ser Ala Val
            100                 105                 110
Tyr Tyr Cys Ala Arg Gly Tyr Ser Asn Tyr Leu Asp Tyr Trp Gly Gln
        115                 120                 125
Gly Thr Thr Leu Thr Val Ser Ser
    130                 135

<210> 57
<211> 393
<212> DNA
<213> Mus musculus
<400> 57
atggagacag acacactcct gctatgggtg ctgctgctct gggttccagg ttccactggt 60
gacattgtgc tcacccaatc tccagcttct ttggctgtgt ctctagggca gagagtcacc 120
atctcctgca gagccagtga aagtgttgaa tattatggca ctagtttaat gcagtggtac 180
caacagaaac caggacagcc acccaaactc ctcatctatg gtgcatccaa cgtagaatct 240
ggggtccctg ccaggtttag tggcagtggg tctgggacag acttcagcct caacatccat 300
cctgtggagg aggatgatat tgcaatgtat ttctgtcagc aaagtaggaa ggttccttcg 360
acgttcggtg gaggcaccaa gctggaaatc aaa 393
<210> 58
<211> 131
<212> PRT
<213> Mus musculus
<400> 58
Met Glu Thr Asp Thr Leu Leu Leu Trp Val Leu Leu Leu Trp Val Pro
1               5                   10                  15
Gly Ser Thr Gly Asp Ile Val Leu Thr Gln Ser Pro Ala Ser Leu Ala
            20                  25                  30
Val Ser Leu Gly Gln Arg Val Thr Ile Ser Cys Arg Ala Ser Glu Ser
        35                  40                  45
Val Glu Tyr Tyr Gly Thr Ser Leu Met Gln Trp Tyr Gln Gln Lys Pro
    50                  55                  60
Gly Gln Pro Pro Lys Leu Leu Ile Tyr Gly Ala Ser Asn Val Glu Ser
65                  70                  75                  80
Gly Val Pro Ala Arg Phe Ser Gly Ser Gly Ser Gly Thr Asp Phe Ser
                85                  90                  95
Leu Asn Ile His Pro Val Glu Glu Asp Asp Ile Ala Met Tyr Phe Cys
            100                 105                 110
Gln Gln Ser Arg Lys Val Pro Ser Thr Phe Gly Gly Gly Thr Lys Leu
        115                 120                 125
Glu Ile Lys
    130
<210> 59
<211> 978
<212> DNA
<213> homo sapiens
<400> 59
atggctgtag gaaacaacac tcaacgaagt tattccatca tcccgtgttt tatatttgtt 60
gagcttgtca tcatggctgg gacagtgctg cttgcctact acttcgaatg cactgacact 120
tttcaggtgc atatccaagg attcttctgt caggacggag acttaatgaa gccttaccca 180
gggacagagg aagaaagctt catcacccct ctggtgctct attgtgtgct ggctgccacc 240
ccaactgcta ttatttttat tggtgagata tccatgtatt tcataaaatc aacaagagaa 300
tccctgattg ctcaggagaa aacaattctg accggagaat gctgttacct gaacccctta 360
cttcgaagga tcataagatt cacaggggtg tttgcatttg gactttttgc tactgacatt 420
tttgtaaacg ccggacaagt ggtcactggg cacttaacgc catacttcct gactgtgtgc 480
aagccaaact acaccagtgc agactgccaa gcgcaccacc agtttataaa caatgggaac 540
atttgtactg gggacctgga agtgatagaa aaggctcgga gatcctttcc ctccaaacac 600
gctgctctga gcatttactc cgccttatat gccacgatgt atattacaag cacaatcaag 660
acgaagagca gtcgactggc caagccggtg ctgtgcctcg gaactctctg cacagccttc 720
ctgacaggcc tcaaccgggt ctctgagtat cggaaccact gctcggacgt gattgctggt 780
ttcatcctgg gcactgcagt ggccctgttt ctgggaatgt gtgtggttca taactttaaa 840
ggaacgcaag gatctccttc caaacccaag cctgaggatc cccgtggagt acccctaatg 900
gctttcccaa ggatagaaag ccctctggaa accttaagtg cacagaatca ctctgcgtcc 960
atgaccgaag ttacctga 978
<210> 60
<211> 325
<212> PRT
<213> homo sapiens
<400> 60
Met Ala Val Gly Asn Asn Thr Gln Arg Ser Tyr Ser Ile Ile Pro Cys
1               5                   10                  15
Phe Ile Phe Val Glu Leu Val Ile Met Ala Gly Thr Val Leu Leu Ala
            20                  25                  30
Tyr Tyr Phe Glu Cys Thr Asp Thr Phe Gln Val His Ile Gln Gly Phe
        35                  40                  45
Phe Cys Gln Asp Gly Asp Leu Met Lys Pro Tyr Pro Gly Thr Glu Glu

    50                  55                  60
Glu Ser Phe Ile Thr Pro Leu Val Leu Tyr Cys Val Leu Ala Ala Thr
65                  70                  75                  80
Pro Thr Ala Ile Ile Phe Ile Gly Glu Ile Ser Met Tyr Phe Ile Lys
                85                  90                  95
Ser Thr Arg Glu Ser Leu Ile Ala Gln Glu Lys Thr Ile Leu Thr Gly
            100                 105                 110
Glu Cys Cys Tyr Leu Asn Pro Leu Leu Arg Arg Ile Ile Arg Phe Thr
        115                 120                 125
Gly Val Phe Ala Phe Gly Leu Phe Ala Thr Asp Ile Phe Val Asn Ala
    130                 135                 140
Gly Gln Val Val Thr Gly His Leu Thr Pro Tyr Phe Leu Thr Val Cys
145                 150                 155                 160
Lys Pro Asn Tyr Thr Ser Ala Asp Cys Gln Ala His His Gln Phe Ile
                165                 170                 175
Asn Asn Gly Asn Ile Cys Thr Gly Asp Leu Glu Val Ile Glu Lys Ala
            180                 185                 190
Arg Arg Ser Phe Pro Ser Lys His Ala Ala Leu Ser Ile Tyr Ser Ala
        195                 200                 205
Leu Tyr Ala Thr Met Tyr Ile Thr Ser Thr Ile Lys Thr Lys Ser Ser
    210                 215                 220
Arg Leu Ala Lys Pro Val Leu Cys Leu Gly Thr Leu Cys Thr Ala Phe
225                 230                 235                 240
Leu Thr Gly Leu Asn Arg Val Ser Glu Tyr Arg Asn His Cys Ser Asp
                245                 250                 255
Val Ile Ala Gly Phe Ile Leu Gly Thr Ala Val Ala Leu Phe Leu Gly
            260                 265                 270
Met Cys Val Val His Asn Phe Lys Gly Thr Gln Gly Ser Pro Ser Lys
        275                 280                 285
Pro Lys Pro Glu Asp Pro Arg Gly Val Pro Leu Met Ala Phe Pro Arg
    290                 295                 300
Ile Glu Ser Pro Leu Glu Thr Leu Ser Ala Gln Asn His Ser Ala Ser
305                 310                 315                 320
Met Thr Glu Val Thr
                325
<210> 61
<211> 24
<212> DNA
<213> Mus musculus
<400> 61
caggggccag tggatagacc gatg 24
<210> 62
<211> 24
<212> DNA
<213> Mus musculus
<400> 62
caggggccag tggatagact gatg 24
<210> 63
<211> 23
<212> DNA
<213> Mus musculus
<400> 63
gctcactgga tggtgggaag atg 23
<210> 64
<211> 4
<212> PRT
<213> artificial
<220>
<223> artificial linker
<400> 64
Gly Gly Gly Ser
1
<210> 65
<211> 4
<212> PRT
<213> artificial
<220>
<223> artificial linker
<400> 65

Ser Gly Gly Gly
1
<210> 66
<211> 5
<212> PRT
<213> artificial
<220>
<223> artificial linker
<400> 66
Gly Gly Gly Gly Ser
1               5
<210> 67
<211> 5
<212> PRT
<213> artificial
<220>
<223> artificial linker
<400> 67
Ser Gly Gly Gly Gly
1               5
<210> 68
<211> 6
<212> PRT
<213> artificial
<220>
<223> artificial linker
<400> 68
Gly Gly Gly Gly Gly Ser
1               5
<210> 69
<211> 6
<212> PRT
<213> artificial
<220>
<223> artificial linker
<400> 69
Ser Gly Gly Gly Gly Gly
1               5
<210> 70
<211> 7
<212> PRT
<213> artificial
<220>
<223> artificial linker
<400> 70
Gly Gly Gly Gly Gly Gly Ser
1               5
<210> 71
<211> 7
<212> PRT
<213> artificial
<220>
<223> artificial linker
<400> 71
Ser Gly Gly Gly Gly Gly Gly
1               5
<210> 72
<211> 325
<212> PRT
<213> Pan troglodytes
<400> 72
Met Ala Val Gly Asn Asn Thr Gln Arg Ser Tyr Ser Ile Ile Pro Cys
1               5                   10                  15
Phe Ile Phe Val Glu Leu Val Ile Met Ala Gly Thr Val Leu Leu Ala
            20                  25                  30
Tyr Tyr Phe Glu Cys Thr Asp Thr Phe Gln Val His Ile Gln Gly Phe
        35                  40                  45
Phe Cys Gln Asp Gly Asp Leu Met Lys Pro Tyr Pro Gly Thr Glu Glu
    50                  55                  60

Glu Ser Phe Ile Thr Pro Leu Val Leu Tyr Cys Val Leu Ala Ala Thr
65                  70                  75                  80
Pro Thr Ala Ile Ile Phe Ile Gly Glu Ile Ser Met Tyr Phe Ile Lys
                85                  90                  95
Ser Thr Arg Glu Ser Leu Ile Ala Gln Glu Lys Thr Ile Leu Thr Gly
            100                 105                 110
Glu Cys Cys Tyr Leu Asn Pro Leu Leu Arg Arg Ile Ile Arg Phe Thr
        115                 120                 125
Gly Val Phe Ala Phe Gly Leu Phe Ala Thr Asp Ile Phe Val Asn Ala
    130                 135                 140
Gly Gln Val Val Thr Gly His Leu Thr Pro Tyr Phe Leu Thr Val Cys
145                 150                 155                 160
Lys Pro Asn Tyr Thr Ser Ala Asp Cys Gln Ala His His Gln Phe Ile
                165                 170                 175
Asn Asn Gly Asn Ile Cys Thr Gly Asp Leu Glu Val Ile Glu Lys Ala
            180                 185                 190
Arg Arg Ser Phe Pro Ser Lys His Ala Ala Leu Ser Ile Tyr Ser Ala
        195                 200                 205
Leu Tyr Ala Thr Met Tyr Ile Thr Ser Thr Ile Lys Thr Lys Ser Ser
    210                 215                 220
Arg Leu Ala Lys Pro Val Leu Cys Leu Gly Thr Leu Cys Thr Ala Phe
225                 230                 235                 240
Leu Thr Gly Leu Asn Arg Val Ser Glu Tyr Arg Asn His Cys Ser Asp
                245                 250                 255
Val Ile Ala Gly Phe Ile Leu Gly Thr Ala Val Ala Leu Phe Leu Gly
            260                 265                 270
Met Cys Val Val His Asn Phe Lys Gly Thr Gln Gly Ser Pro Ser Lys
        275                 280                 285
Pro Lys Pro Glu Asp Pro Arg Gly Val Pro Leu Met Ala Phe Pro Arg
    290                 295                 300
Ile Glu Ser Pro Leu Glu Thr Leu Ser Ala Gln Asn His Ser Ala Ser
305                 310                 315                 320
Met Thr Glu Val Thr
                325
<210> 73
<211> 325
<212> PRT
<213> Mus musculus
<400> 73
Met Ala Val Glu Asn Asn Thr Gln Arg Ser Tyr Ser Ile Ile Pro Cys
1               5                   10                  15
Phe Ile Phe Val Glu Leu Val Ile Met Ala Gly Thr Val Leu Leu Ala
            20                  25                  30
Tyr Tyr Phe Glu Cys Thr Asp Thr Phe Gln Val His Ile Gln Gly Phe
        35                  40                  45
Phe Cys Gln Asp Gly Asp Leu Met Lys Pro Tyr Pro Gly Thr Glu Glu
    50                  55                  60
Glu Ser Phe Ile Ser Pro Leu Val Leu Tyr Cys Val Leu Ala Ala Thr
65                  70                  75                  80
Pro Thr Ala Ile Ile Phe Ile Gly Glu Ile Ser Met Tyr Phe Ile Lys
                85                  90                  95
Ser Thr Arg Glu Ser Leu Ile Ala Glu Glu Lys Met Ile Leu Thr Gly
            100                 105                 110
Asp Cys Cys Tyr Leu Ser Pro Leu Leu Arg Arg Ile Ile Arg Phe Ile
        115                 120                 125
Gly Val Phe Ala Phe Gly Leu Phe Ala Thr Asp Ile Phe Val Asn Ala
    130                 135                 140
Gly Gln Val Val Thr Gly His Leu Thr Pro Tyr Phe Leu Thr Val Cys
145                 150                 155                 160
Gln Pro Asn Tyr Thr Ser Thr Asp Cys Arg Ala His Gln Gln Phe Ile
                165                 170                 175
Asn Asn Gly Asn Ile Cys Thr Gly Asp Leu Glu Val Ile Glu Lys Ala
            180                 185                 190
Arg Arg Ser Phe Pro Ser Lys His Ala Ala Leu Ser Ile Tyr Ser Ala
        195                 200                 205
Leu Tyr Ala Thr Met Tyr Ile Thr Ser Thr Ile Lys Thr Lys Ser Ser
    210                 215                 220
Arg Leu Ala Lys Pro Val Leu Cys Leu Gly Thr Leu Cys Thr Ala Phe
225                 230                 235                 240

Leu Thr Gly Leu Asn Arg Val Ser Glu Tyr Arg Asn His Cys Ser Asp
                245                 250                 255
Val Ile Ala Gly Phe Ile Leu Gly Thr Ala Val Ala Leu Phe Leu Gly
            260                 265                 270
Met Cys Val Val His Asn Phe Arg Gly Thr Gln Gly Ser Pro Ser Lys
        275                 280                 285
Pro Lys Pro Glu Asp Pro Arg Gly Val Pro Leu Met Ala Phe Pro Arg
    290                 295                 300
Ile Glu Ser Pro Leu Glu Thr Leu Ser Ala Gln Asn His Ser Ala Ser
305                 310                 315                 320
Met Thr Glu Val Thr
                325
<210> 74
<211> 325
<212> PRT
<213> Rattus norvegicus
<400> 74
Met Ala Val Glu Asn Asn Thr Gln Arg Ser Tyr Ser Ile Ile Pro Cys
1               5                   10                  15
Phe Ile Phe Val Glu Leu Val Ile Met Ala Gly Thr Val Leu Leu Ala
            20                  25                  30
Tyr Tyr Phe Glu Cys Thr Asp Thr Phe Gln Val His Ile Gln Gly Phe
        35                  40                  45
Phe Cys Gln Asp Gly Asp Leu Met Lys Pro Tyr Pro Gly Thr Glu Glu
    50                  55                  60
Glu Ser Phe Ile Ser Pro Leu Val Leu Tyr Cys Val Leu Ala Ala Thr
65                  70                  75                  80
Pro Thr Ala Ile Ile Phe Ile Gly Glu Ile Ser Met Tyr Phe Ile Lys
                85                  90                  95
Ser Thr Arg Glu Ser Leu Ile Ala Glu Glu Lys Met Ile Leu Thr Gly
            100                 105                 110
Asp Cys Cys Tyr Leu Ser Pro Leu Leu Arg Arg Ile Val Arg Phe Ile
        115                 120                 125
Gly Val Phe Ala Phe Gly Leu Phe Ala Thr Asp Ile Phe Val Asn Ala
    130                 135                 140
Gly Gln Val Val Thr Gly His Leu Thr Pro Tyr Phe Leu Thr Val Cys
145                 150                 155                 160
Gln Pro Asn Tyr Thr Ser Thr Asp Cys Arg Ala His His Gln Phe Ile
                165                 170                 175
Asn Asn Gly Asn Ile Cys Thr Gly Asp Leu Glu Val Ile Glu Lys Ala
            180                 185                 190
Arg Arg Ser Phe Pro Ser Lys His Ala Ala Leu Ser Ile Tyr Ser Ala
        195                 200                 205
Leu Tyr Ala Thr Met Tyr Ile Thr Ser Thr Ile Lys Thr Lys Ser Ser
    210                 215                 220
Arg Leu Ala Lys Pro Val Leu Cys Leu Gly Asp Leu Cys Thr Ala Phe
225                 230                 235                 240
Leu Thr Gly Leu Asn Arg Val Ser Glu Tyr Arg Asn His Cys Ser Asp
                245                 250                 255
Val Ile Ala Gly Phe Ile Leu Gly Thr Ala Val Ala Leu Phe Leu Gly
            260                 265                 270
Met Cys Val Val His Asn Phe Lys Gly Thr Gln Gly Ser Ala Ser Lys
        275                 280                 285
Pro Lys Pro Glu Asp Pro Arg Gly Val Pro Leu Met Ala Phe Pro Arg
    290                 295                 300
Ile Glu Ser Pro Leu Glu Thr Leu Ser Ala Gln Asn His Ser Ala Ser
305                 310                 315                 320
Met Thr Glu Val Thr
                325
<210> 75
<211> 325
<212> PRT
<213> Canis familiaris
<400> 75
Met Ala Val Gly Asn Asn Thr Gln Arg Ser His Ser Ile Ile Pro Cys
1               5                   10                  15
Phe Ile Phe Val Glu Leu Val Ile Met Ala Gly Thr Val Leu Leu Ala
            20                  25                  30
Tyr Tyr Phe Glu Cys Thr Asp Thr Phe Gln Val His Ile Gln Gly Phe

        35                  40                  45
Phe Cys Gln Asp Gly Asp Leu Met Lys Pro Tyr Pro Gly Thr Glu Glu
    50                  55                  60
Glu Ser Phe Ile Thr Pro Leu Val Leu Tyr Cys Val Leu Ala Ala Thr
65                  70                  75                  80
Pro Thr Ala Ile Ile Phe Ile Gly Glu Ile Ser Met Tyr Phe Ile Lys
                85                  90                  95
Ser Thr Arg Glu Ser Leu Ile Ala Gln Glu Lys Thr Ile Leu Thr Gly
            100                 105                 110
Glu Cys Cys Tyr Leu Asn Pro Leu Leu Arg Arg Ile Ile Arg Phe Ile
        115                 120                 125
Gly Val Phe Ala Phe Gly Leu Phe Ala Thr Asp Ile Phe Val Asn Ala
    130                 135                 140
Gly Gln Val Val Thr Gly His Leu Thr Pro Tyr Phe Leu Thr Val Cys
145                 150                 155                 160
Lys Pro Asn Tyr Thr Ser Thr Asp Cys Arg Ala His His Gln Phe Ile
                165                 170                 175
Asn Asn Gly Asn Ile Cys Thr Gly Asp Met Glu Val Ile Glu Lys Ala
            180                 185                 190
Arg Arg Ser Phe Pro Ser Lys His Ala Ala Leu Ser Ile Tyr Ser Ala
        195                 200                 205
Leu Tyr Ala Thr Met Tyr Ile Thr Ser Thr Ile Lys Thr Lys Ser Ser
    210                 215                 220
Arg Leu Ala Lys Pro Val Leu Cys Leu Gly Thr Leu Cys Thr Ala Phe
225                 230                 235                 240
Leu Thr Gly Leu Asn Arg Val Ser Glu Tyr Arg Asn His Cys Ser Asp
                245                 250                 255
Val Ile Ala Gly Phe Ile Leu Gly Thr Ala Val Ala Leu Phe Leu Gly
            260                 265                 270
Met Cys Val Val His Asn Phe Lys Gly Thr Gln Gly Ser Pro Ser Lys
        275                 280                 285
Pro Lys Pro Glu Asp Pro Arg Gly Val Pro Leu Met Ala Phe Pro Arg
    290                 295                 300
Ile Glu Ser Pro Leu Glu Thr Leu Ser Ala Gln Asn His Ser Ala Ser
305                 310                 315                 320
Met Thr Glu Val Thr
                325
<210> 76
<211> 309
<212> PRT
<213> Gallus gallus
<400> 76
Met Leu Tyr Phe Gln Leu Val Ile Met Ala Gly Thr Val Leu Leu Ala
1               5                   10                  15
Tyr Tyr Phe Glu Cys Thr Asp Thr Phe Gln Val His Ile Gln Gly Phe
            20                  25                  30
Phe Cys Gln Asp Gly Asn Leu Met Lys Pro Tyr Pro Gly Thr Glu Asp
        35                  40                  45
Glu Ser Phe Ile Ser Pro Leu Val Leu Tyr Cys Val Leu Ala Ser Thr
    50                  55                  60
Pro Thr Ala Ile Ile Phe Ile Gly Glu Ile Ser Met Tyr Phe Ile Lys
65                  70                  75                  80
Ser Thr Arg Glu Thr Leu Ile Ile Gln Glu Lys Thr Ile Leu Thr Gly
                85                  90                  95
Glu Cys Cys Tyr Leu Asn Pro Leu Leu Arg Arg Ile Ile Arg Phe Ile
            100                 105                 110
Gly Val Phe Ala Phe Gly Leu Phe Ala Thr Asp Ile Phe Val Asn Ala
        115                 120                 125
Gly Gln Val Val Thr Gly Asn Leu Ala Pro Tyr Phe Leu Thr Val Cys
    130                 135                 140
Lys Pro Asn Tyr Thr Gly Asn Asp Cys Arg Thr His His Gln Phe Ile
145                 150                 155                 160
Asn Asn Gly Asn Ile Cys Thr Gly Asp Val Glu Val Ile Glu Lys Ala
                165                 170                 175
Arg Arg Ser Phe Pro Ser Lys His Ala Ala Leu Ser Ile Tyr Ser Ala
            180                 185                 190
Leu Tyr Ala Thr Met Tyr Ile Thr Ser Thr Ile Lys Thr Lys Ser Ser
        195                 200                 205
Arg Leu Ala Lys Pro Val Leu Cys Leu Gly Thr Leu Cys Ala Ala Phe

    210                 215                 220
Leu Thr Gly Leu Asn Arg Val Ser Glu Tyr Arg Asn His Cys Ser Asp
225                 230                 235                 240
Val Ile Ala Gly Phe Ile Leu Gly Ser Ala Val Ala Leu Phe Leu Gly
                245                 250                 255
Ile Cys Val Val His Asn Phe Lys Gly Thr His Gly Ala Pro Ser Lys
            260                 265                 270
Pro Lys Pro Glu Asp Pro Arg Gly Met Pro Leu Met Ala Phe Pro Arg
        275                 280                 285
Val Glu Ser Pro Leu Glu Thr Leu Ser Ala Gln Asn His Ser Ala Ser
    290                 295                 300
Met Thr Glu Val Thr
305
<210> 77
<211> 351
<212> DNA
<213> Mus musculus
<400> 77
caggtccaac tgcagcagcc tggggctgaa ctggtgaagc ctggggcttc agtgaagctg 60
tcctgcaagg cttctggcta cactttcacc agctactgga tgaactgggt gaagcagagg 120
cctggacgag gcctcgagtg gattggaaag attcatcctt ctgatagtga aactcactac 180
aatcaaaagt tcaagagcaa ggccacactg actgtagaca aatcctccag cacggcctac 240
atccaactca acagcctgac atctgaggac tctgcggtct attattgtac aagggggtat 300
aataactacc ttgactactg gggccaaggc accactctca cagtctcctc a 351
<210> 78
<211> 117
<212> PRT
<213> Mus musculus
<400> 78
Gln Val Gln Leu Gln Gln Pro Gly Ala Glu Leu Val Lys Pro Gly Ala
1               5                   10                  15
Ser Val Lys Leu Ser Cys Lys Ala Ser Gly Tyr Thr Phe Thr Ser Tyr
            20                  25                  30
Trp Met Asn Trp Val Lys Gln Arg Pro Gly Arg Gly Leu Glu Trp Ile
        35                  40                  45
Gly Lys Ile His Pro Ser Asp Ser Glu Thr His Tyr Asn Gln Lys Phe
    50                  55                  60
Lys Ser Lys Ala Thr Leu Thr Val Asp Lys Ser Ser Ser Thr Ala Tyr
65                  70                  75                  80
Ile Gln Leu Asn Ser Leu Thr Ser Glu Asp Ser Ala Val Tyr Tyr Cys
                85                  90                  95
Thr Arg Gly Tyr Asn Asn Tyr Leu Asp Tyr Trp Gly Gln Gly Thr Thr
            100                 105                 110
Leu Thr Val Ser Ser
        115
<210> 79
<211> 333
<212> DNA
<213> Mus musculus
<400> 79
gacattgtgc tcacccaatc tccagcttct ttggctgtgt ctctagggca gagtgtcacc 60
atctcctgca gagccagtga aagtgttgaa tattatggca ctagtttaat gcagtggtat 120
caacagaaac caggacagcc acccaaactc ctcatctatg gtgcatccaa cgtagaatct 180
ggggtccctg ccaggtttag tggcagtggg tcttggacag acttcagcct caacatccat 240
cctgtggagg aggatgatat tgcaatatat ttctgtcagc aaagtaggaa ggttccttcg 300
acgttcggtg gaggcaccaa gctggaaatc aaa 333
<210> 80
<211> 111
<212> PRT
<213> Mus musculus
<400> 80
Asp Ile Val Leu Thr Gln Ser Pro Ala Ser Leu Ala Val Ser Leu Gly
1               5                   10                  15
Gln Ser Val Thr Ile Ser Cys Arg Ala Ser Glu Ser Val Glu Tyr Tyr
            20                  25                  30
Gly Thr Ser Leu Met Gln Trp Tyr Gln Gln Lys Pro Gly Gln Pro Pro
        35                  40                  45
Lys Leu Leu Ile Tyr Gly Ala Ser Asn Val Glu Ser Gly Val Pro Ala
    50                  55                  60

Arg Phe Ser Gly Ser Gly Ser Trp Thr Asp Phe Ser Leu Asn Ile His
65                  70                  75                  80
Pro Val Glu Glu Asp Asp Ile Ala Ile Tyr Phe Cys Gln Gln Ser Arg
                85                  90                  95
Lys Val Pro Ser Thr Phe Gly Gly Gly Thr Lys Leu Glu Ile Lys
            100                 105                 110
<210> 81
<211> 360
<212> DNA
<213> Mus musculus
<400> 81
caggtccaac tgcagcagcc tggggctgaa ctggtgaagc ctggggcttc agtgaagctg 60
tcctgcaagg cttctggcta cactttcacc agctactgga tgaactgggt gaagcagagg 120
cctggacgag gcctcgagtg gattggaaag attcatcctt ctgatagtga aactcactac 180
aatcaaaagt tcaagagcaa ggccacactg actgtagaca aatcctccag cacagcctac 240
atccaactca gcagcctgac atctgaggac tctgcggtct attattgtgc aagatcgggt 300
ggtaactacg gggcctggtt tgtttactgg ggccaaggga ctctggtcac tgtctctgca 360
<210> 82
<211> 120
<212> PRT
<213> Mus musculus
<400> 82
Gln Val Gln Leu Gln Gln Pro Gly Ala Glu Leu Val Lys Pro Gly Ala
1               5                   10                  15
Ser Val Lys Leu Ser Cys Lys Ala Ser Gly Tyr Thr Phe Thr Ser Tyr
            20                  25                  30
Trp Met Asn Trp Val Lys Gln Arg Pro Gly Arg Gly Leu Glu Trp Ile
        35                  40                  45
Gly Lys Ile His Pro Ser Asp Ser Glu Thr His Tyr Asn Gln Lys Phe
    50                  55                  60
Lys Ser Lys Ala Thr Leu Thr Val Asp Lys Ser Ser Ser Thr Ala Tyr
65                  70                  75                  80
Ile Gln Leu Ser Ser Leu Thr Ser Glu Asp Ser Ala Val Tyr Tyr Cys
                85                  90                  95
Ala Arg Ser Gly Gly Asn Tyr Gly Ala Trp Phe Val Tyr Trp Gly Gln
            100                 105                 110
Gly Thr Leu Val Thr Val Ser Ala
        115                 120
<210> 83
<211> 333
<212> DNA
<213> Mus musculus
<400> 83
gacattgtgc tcacccaatc tccagcttct ttggctgtgt ctctagggca gagtgtcacc 60
atctcctgca gagccagtga aagtgttgaa tattatggca ctagttttat gcagtggtgc 120
caacagaaac caggacagcc acccaaactc ctcatctatg gtgcatccaa cgtaggatct 180
ggggtccctg ccaggtttag tggcagtggg tctggggcag acttcagcct caacatccat 240
cctgtggagg aggatgatat tgcaatgtat ttctgtcagc aaagtaggaa ggttccgtat 300
acgttcggat cggggaccag gctggaaatc aaa 333
<210> 84
<211> 111
<212> PRT
<213> Mus musculus
<400> 84
Asp Ile Val Leu Thr Gln Ser Pro Ala Ser Leu Ala Val Ser Leu Gly
1               5                   10                  15
Gln Ser Val Thr Ile Ser Cys Arg Ala Ser Glu Ser Val Glu Tyr Tyr
            20                  25                  30
Gly Thr Ser Phe Met Gln Trp Cys Gln Gln Lys Pro Gly Gln Pro Pro
        35                  40                  45
Lys Leu Leu Ile Tyr Gly Ala Ser Asn Val Gly Ser Gly Val Pro Ala
    50                  55                  60
Arg Phe Ser Gly Ser Gly Ser Gly Ala Asp Phe Ser Leu Asn Ile His
65                  70                  75                  80
Pro Val Glu Glu Asp Asp Ile Ala Met Tyr Phe Cys Gln Gln Ser Arg
                85                  90                  95
Lys Val Pro Tyr Thr Phe Gly Ser Gly Thr Arg Leu Glu Ile Lys
            100                 105                 110
<210> 85

<211> 351
<212> DNA
<213> Mus musculus
<400> 85
caggtccaac tgcagcagcc tggggctgaa ctggtgaagc ctggggcttc agtgaagctg 60
tcctgcaagg cttctggcta cactttcacc agctactgga tgaactgggt gaagcagagg 120
cctggacgag gcctcgagtg gattggaaag attcatcctt ctgatagtga aactcactac 180
aatcaaaaat tcaagagcaa ggccacactg actgtagaca aatcctccag cacagcctac 240
atccaactca gcagcctgac atctgaggac tctgcggtct attattgtgc aagggggtat 300
agtaactacc ttgactactg gggccaaggc accactctca cagtctcctc a 351
<210> 86
<211> 117
<212> PRT
<213> Mus musculus
<400> 86
Gln Val Gln Leu Gln Gln Pro Gly Ala Glu Leu Val Lys Pro Gly Ala
1               5                   10                  15
Ser Val Lys Leu Ser Cys Lys Ala Ser Gly Tyr Thr Phe Thr Ser Tyr
            20                  25                  30
Trp Met Asn Trp Val Lys Gln Arg Pro Gly Arg Gly Leu Glu Trp Ile
        35                  40                  45
Gly Lys Ile His Pro Ser Asp Ser Glu Thr His Tyr Asn Gln Lys Phe
    50                  55                  60
Lys Ser Lys Ala Thr Leu Thr Val Asp Lys Ser Ser Ser Thr Ala Tyr
65                  70                  75                  80
Ile Gln Leu Ser Ser Leu Thr Ser Glu Asp Ser Ala Val Tyr Tyr Cys
                85                  90                  95
Ala Arg Gly Tyr Ser Asn Tyr Leu Asp Tyr Trp Gly Gln Gly Thr Thr
            100                 105                 110
Leu Thr Val Ser Ser
        115
<210> 87
<211> 333
<212> DNA
<213> Mus musculus
<400> 87
gacattgtgc tcacccaatc tccagcttct ttggctgtgt ctctagggca gagagtcacc 60
atctcctgca gagccagtga aagtgttgaa tattatggca ctagtttaat gcagtggtac 120
caacagaaac caggacagcc acccaaactc ctcatctatg gtgcatccaa cgtagaatct 180
ggggtccctg ccaggtttag tggcagtggg tctgggacag acttcagcct caacatccat 240
cctgtggagg aggatgatat tgcaatgtat ttctgtcagc aaagtaggaa ggttccttcg 300
acgttcggtg gaggcaccaa gctggaaatc aaa 333
<210> 88
<211> 111
<212> PRT
<213> Mus musculus
<400> 88
Asp Ile Val Leu Thr Gln Ser Pro Ala Ser Leu Ala Val Ser Leu Gly
1               5                   10                  15
Gln Arg Val Thr Ile Ser Cys Arg Ala Ser Glu Ser Val Glu Tyr Tyr
            20                  25                  30
Gly Thr Ser Leu Met Gln Trp Tyr Gln Gln Lys Pro Gly Gln Pro Pro
        35                  40                  45
Lys Leu Leu Ile Tyr Gly Ala Ser Asn Val Glu Ser Gly Val Pro Ala
    50                  55                  60
Arg Phe Ser Gly Ser Gly Ser Gly Thr Asp Phe Ser Leu Asn Ile His
65                  70                  75                  80
Pro Val Glu Glu Asp Asp Ile Ala Met Tyr Phe Cys Gln Gln Ser Arg
                85                  90                  95
Lys Val Pro Ser Thr Phe Gly Gly Gly Thr Lys Leu Glu Ile Lys
            100                 105                 110